# EUROPÄISCHE PATENTSCHRIFT

(11) **EP 0 186 098 B1**
(45) Veröffentlichungstag und Bekanntmachung des Hinweises auf die Patenterteilung: **17.06.1992**
(21) Anmeldenummer: 85116083.8
(22) Anmeldetag: 17.12.1985
(51) Int. Cl.: C12N 15/20, C12N 1/20, C12P 21/02, C07H 21/04, C07K 15/26

(54) **Pferde-Interferone**
Equine interferons
Interférons de cheval

(30) Priorität: 18.12.1984 DE 3446122; 18.12.1984 DE 3446124; 16.08.1985 DE 3529262; 02.10.1985 DE 3535115
(43) Veröffentlichungstag der Anmeldung: 02.07.1986
(73) Patentinhaber: BOEHRINGER INGELHEIM INTERNATIONAL GmbH, 55218 Ingelheim am Rhein (DE)
(72) Erfinder: Himmler, Adolf, Dipl.-Ing., A-1120 Wien (AT); Hauptmann, Rudolf, Dr., A-1040 Wien (AT); Hauel, Norbert, Dr., A-7950 Biberach 1 (AT); Adolf, Günther, Dr., A-1050 Wien (AT); Swetly, Peter, Dr., A-1130 Wien (AT)

(56) Entgegenhaltungen:
- EP-A- 0 042 246
- EP-A- 0 080 848
- EP-A- 0 088 622
- WO-A-80/02375
- US-A- 4 262 090
- JOURNAL OF MOLECULAR BIOLOGY, Band 166, 1983, Seiten 457-475, Academic Press Inc., Ltd., London, GB; V. WILSON et al.: "A comparison of vertebrate interferon gene families detected by hybridization with human interferon DNA"
- CHEMICAL ABSTRACTS, Band 93, Nr. 11, 15. September 1980, Seite 554, Nr. 112220y, Columbus, Ohio, US; S.-C. TSAI: "Canine interferon", & DISS. ABSTR. INT. B 1980, 41(1) 154
- CHEMICAL ABSTRACTS, Band 98, Nr. 17, 25. April 1983, Seite 439, Nr. 141702r, Columbus, Ohio, US; T. YILMA et al.: "Preliminary characterization of equine interferons and their antiviral activities on bovine, ovine, and human cells", & J. INTERFERON RES. 1982, 2(3), 363-70

## Beschreibung

Gegenstand der vorliegenden Erfindung ist ein Verfahren zur Herstellung von rekombinanten Pferde Interferonen sowie die Interferone selbst.

Interferone sind Proteine, die von eukaryotischen Zellen nach Virus Infektion oder anderen Stimulationen sekretiert werden und ihrerseits die Zellen vor Virusinfektionen schützen können. Mittlerweile sind vier Klassen an Interferonen bekannt; bezeichnet werden sie mit Interferon-alpha, Interferon-beta, Interferon-omega und Interferon-gamma (abgekürzt: IEN-a, IFN-ß, IFN-y und IFN-w). Sie unterscheiden sich in ihrem Aufbau und in ihren Wirkungen. So können Interferone regulierend die Zellen des Immunsgstems, oder auch die Differenzierung von Zellen und das Wachstum von Tumoren beeinflussen.

Lange Zeit war angenommen worden, daß die Interferone speziesspezifisch wirken. In-vitro Versuche zeigten jedoch, daß IFN-Präparate von Rindern eine antivirale Wirkung beim Affen und beim Menschen auslösen können (32). Diese Speziesinteraktivität hängt möglicherweise mit der mehr oder weniger großen Homologie der Gene bzw. der Proteine zusammen; aufgrund der geringen Mengen an tierischen Interferonen konnte diese Annahme nicht überprüft werden.

Trotz der festgestellten Speziesinteraktivitäten sind Nebenwirkungen wie Antigenitäten bei der Anwendung speziesfremder Interferone zu erwarten, die für eine Therapie nicht zu tolerieren sind.

Da andererseits jedoch die Nutz- und Haustierhaltung einen bedeutenden wirtschaftlichen Wert darstellt, besteht ein Bedarf an Interferonen für die verschiedenen Spezies, die der Veterinärmediziner anwenden kann.

Hochgereinigtes Tierinterferon der verschiedenen Spezies würde darüberhinaus die willkommene Gelegenheit bieten, die Wirkmechanismen für Interferone zu untersuchen, um zu Modell-vorstellungen zu kommen, die auf den Menschen zu übertragen sind.

Die ersten Untersuchungen mit tierischen Interferonen wurden mit Präparationen aus natürlichem Zellmaterial durchgeführt; Ausbeute und Reinheit der nach diesem Verfahren hergestellten Interferone lassen sie als ungeeignet für die Herstellung von Arzneimitteln erscheinen.

Durch die Entwicklung der rekombinanten DNA-Technik ist es möglich, von Mikroorganismen heterologe Proteine produzieren zu lassen. So hergestellt wurden beispielsweise auch Human-Interferone (Hu-IFN); seit neuestem auch ein Rinder-alpha- und ein Rinder-beta-Interferon.

Gegenstand der vorliegenden Erfindung sind neue Pferde Interferone (EqIFN) und ihre gegebenenfalls N-glykosilierten Derivate.

Gegenstand der Erfindung sind außerdem die für diese Interferone kodierenden Gensequenzen ebenso wie rekombinante Moleküle, die diese Sequenzen enthalten, Expressions-Vektoren wie Plasmide, mit den Sequenzen als Inserts und verschiedene Wirtsorganismen oder Kulturen, die die Herstellung der Pferde Interferone ermöglichen.

Ein bevorzugter Gegenstand der Erfindung sind die Pferde Interferone und die sie kodierenden Sequenzen der nachfolgenden Formeln: 
1                   5                   10                  15
Cys Asp Leu Pro His Thr His Ser Leu Gly Asn Thr Arg Val Leu
TGT GAC CTG CCT CAC ACC CAT AGC CTG GGC AAC ACA AGG GTC TTG
                    20                  25                  30
Met Leu Leu Gly Gln Met Arg Arg Ile Ser Pro Phe Ser Cys Leu
ATG CTC CTG GGG CAA ATG AGG AGA ATC TCC CCC TTC TCC TGC CTG
                    35                  40                  45
Lys Asp Arg Asn Asp Phe Gly Phe Pro Gln Glu Val Phe Asp Gly
AAG GAC AGA AAT GAC TTT GGA TTC CCC CAG GAG GTG TTT GAC GGC
                    50                  55                  60
Asn Gln Phe Arg Lys Pro Gln Ala Ile Ser Ala Val His Glu Thr
AAC CAG TTC CGG AAG CCT CAA GCC ATC TCT GCG GTC CAT GAG ACG
                    65                  70                  75
Ile Gln Gln Ile Phe His Leu Phe Ser Thr Asp Gly Ser Ser Ala
ATC CAA CAG ATC TTC CAC CTC TTC AGC ACA GAC GGC TCG TCT GCC
                    80                  85                  90
Ala Trp Asp Glu Ser Leu Leu Asp Lys Leu Tyr Thr Gly Leu Tyr
GCC TGG GAC GAG AGC CTC CTA GAC AAA CTC TAC ACT GGA CTC TAT
                    95                  100                 105
Gln Gln Leu Thr Glu Leu Glu Ala Cys Leu Ser Gln Glu Val Gly
CAG CAG CTG ACT GAG CTG GAA GCC TGT CTG AGC CAG GAG GTG GGG
                    110                 115                 120
Val Glu Glu Thr Pro Leu Met Asn Glu Asp Ser Leu Leu Ala Val
GTG GAA GAG ACG CCC CTG ATG AAC GAG GAC TCC CTG CTG GCT GTG
                    125                 130                 135
Arg Arg Tyr Phe Gln Arg Ile Ala Leu Tyr Leu Gln Glu Lys Lys
AGG AGA TAC TTC CAA AGA ATC GCT CTC TAT CTG CAA GAG AAG AAA
                    140                 145                 150
Tyr Ser Pro Cys Ala Trp Glu Ile Val Arg Ala Glu Ile Met Arg
TAC AGC CCT TGT GCC TGG GAG ATC GTC AGA GCA GAA ATC ATG AGA
                    155                 160                 165
Ser Phe Ser Ser Ser Thr Asn Leu Pro Gln Ser +
TCC TTC TCT TCA TCC ACA AAC TTG CCG CAG AGT TAA,
Formel I
 
1                   5                   10                  15
Cys Asp Leu Pro His Thr His Ser Leu Gly Asn `hr Arg Val Leu
TGT GAC CTG CCT CAC ACC CAT AGC CTG GGC AAC ACA AGG GTC TTG
                    20                  25                  30
Met Leu Leu Gly Gln Met Arg Arg Ile Ser Pro Phe Ser Cys Leu
ATG CTC CTG GGA CAA ATG AGG AGA ATC TCC CCC TTC TCC TGC CTG
                    35                  40                  45
Lys Asp Arg Asn Asp Phe Gly Phe Pro Gln Glu Val Phe Asp Gly
AAG GAC AGA AAT GAC TTT GGA TTC CCC CAG GAG GTG TTT GAC GGC
                    50                  55                  60
Asn Gln Phe Arg Lys Pro Gln Ala Ile Ser Ala Val His Glu Thr
AAC CAG TTC CGG AAG CCT CAA GCC ATC TCC GCG GTC CAT GAG ACG
                    65                  70                  75
Ile Gln Gln Ile Phe His Leu Phe Ser Thr Asp Gly Ser Ser Ala
ATC CAA CAG ATC TTC CAC CTC TTC AGC ACA GAC GGC TCG TCT GCT
                    80                  85                  90
Ala Trp Asp Glu Ser Leu Leu Asp Lys Leu Tyr Thr Gly Leu Tyr
GCC TGG GAC GAG AGC CTC CTA GAC AAG CTC TAC ACT GGA CTC TAT
                    95                  100                 105
Gln Gln Leu Thr Glu Leu Glu Ala Cys Leu Ser Gln Glu Val Gly
CAG CAG CTG ACT GAG CTG GAA GCC TGT CTG AGC CAG GAG GTG GGG
                    110                 115                 120
Val Glu Glu Thr Pro Leu Met Asn Glu Asp Ser Leu Leu Ala Val
GTG GAA GAG ACG CCC CTG ATG AAC GAG GAC TCC CTG CTG GCT GTG
                    125                 130                 135
Arg Arg Tyr Phe Gln Arg Ile Ala Leu Tyr Leu Gln Glu Lys Lys
AGG AGA TAC TTC CAA AGA ATC GCT CTC TAT CTG CAA GAG AAG AAA
                    140                 145                 150
Tyr Ser Pro Cys Ala Trp Glu Ile Val Arg Ala Glu Ile Met Arg
TAC AGC CCT TGT GCC TGG GAG ATC GTC AGA GCA GAA ATC ATG AGA
                    155                 160                 165
Cys Phe Ser Ser Ser Thr Asn Leu Gln Gln Ser +
TGC TTC TCT TCA TCC ACA AAC TTG CAG CAG AGT TAA,
Formel II
 
5 10 15
Val Asn Tyr Asp Leu Leu Arg Ser Gln Leu Arg Ser Ser Asn Ser
GTG AAC TAT GAC TTG CTT CGG TCC CAA CTA AGA AGC AGC AAT TCA
20 25 30
Ala Cys Leu Met Leu Leu Arg Gln Leu Asn Gly Ala Pro Gln Arg
GCA TGT CTG ATG CTC CTG CGG CAG TTG AAT GGA GCC CCT CAA CGT
35 40 45
Cys Pro Glu Asp Thr Met Asn Phe Gln Val Pro Glu Glu Ile Glu
TGC CCC GAG GAC ACA ATG AAC TTC CAG GTC CCT GAG GAG ATT GAG
50 55 60
Gln Ala Gln Gln Phe Gln Lys Glu Asp Ala Ala Leu Val Ile Tyr
CAA GCA CAG CAG TTC CAG AAG GAG GAT GCT GCA TTG GTC ATC TAT
65 70 75
Glu Met Leu Gln His Thr Trp Arg Ile Phe Arg Arg Asn Phe Ala
GAG ATG CTC CAG CAC ACC TGG CGT ATT TTC AGA AGA AAT TTC GCT
80 85 90
Ser Thr Gly Trp Asn Glu Thr Ile Val Lys Asn Leu Leu Val Glu
AGC ACT GGC TGG AAT GAG ACC ATC GTT AAG AAC CTC CTT GTG GAA
95 100 105
Val His Leu Gln Met Asp Arg Leu Glu Thr Asn Leu Glu Glu Ile
GTC CAT CTG CAG ATG GAC CGT CTG GAG ACA AAC CTG GAG GAA ATA
110 115 120
Met Glu Glu Glu Ser Ser Thr Trp Gly Asn Thr Thr Ile Leu Arg
ATG GAG GAG GAA AGC TCC ACC TGG GGA AAC ACA ACC ATT CTG CGC
125 130 135
Leu Lys Lys Tyr Tyr Gly Arg Ile Ser Gln Tyr Leu Lys Ala Lys
CTG AAG AAA TAC TAC GGA AGG ATC TCG CAG TAC CTG AAG GCC AAG
140 145 150
Lys Tyr Ser His Cys Ala Trp Thr Val Val Gln Ala Glu Met Leu
AAG TAC AGC CAC TGT GCC TGG ACA GTG GTC CAA GCG GAA ATG CTC
155 160 165
Arg Asn Leu Ala Phe Leu Asn Gly Leu Thr Asp Tyr Leu Gln Asn +
AGG AAC TTG GCC TTC CTT AAC GGA CTC ACA GAT TAC CTC CAA AAC TGA
Formel III
 
1                   5                   10                  15
Cys Asp Leu Pro Ala Ser Leu Asp Leu Arg Lys Gln Glu Thr Leu
TGC GAC CTG CCT GCG AGC CTT GAC TTG AGA AAG CAG GAG ACC CTC
                    20                  25                  30
Arg Val Leu His Gln Met Glu Thr Ile Ser Pro Pro Ser Cys Leu
AGA GTT CTG CAC CAG ATG GAG ACA ATC TCT CCT CCT TCC TGT CTG
                    35                  40                  45
Lys His Arg Thr Asp Phe Arg Phe Pro Gln Glu Gln Leu Asp Gly
AAG CAC AGG ACA GAC TTC AGG TTC CCC CAG GAG CAG CTG GAT GGC
                    50                  55                  60
Arg Gln Phe Pro Glu Ala Gln Ala Thr Ser Val Leu Gln Glu Met
AGG CAG TTC CCA GAG GCC CAG GCC ACG TCT GTC CTC CAG GAG ATG
                    65                  70                  75
Leu Gln Gln Ile Val Ser Leu Phe His Thr Glu Arg Ser Ser Ala
CTC CAG CAG ATC GTC AGC CTC TTC CAC ACA GAG CGC TCG TCT GCT
                    80                  85                  90
Ala Trp Asn Thr Thr Leu Leu Asp Arg Leu Leu Ala Gly Leu His
GCC TGG AAC ACG ACT CTG CTG GAC CGA CTC CTC GCG GGA CTC CAT
                    95                  100                 105
Gln Gln Leu Glu Asp Leu Asn Thr Cys Leu Asp Glu Gln Thr Gly
CAG CAG CTG GAA GAC CTC AAC ACC TGC TTG GAT GAG CAG ACA GGA
                    110                 115                 120
Glu Glu Glu Ser Ala Leu Gly Thr Val Gly Pro Thr Leu Ala Val
GAG GAA GAA TCC GCC CTG GGA ACT GTG GGC CCT ACA CTG GCC GTG
                    125                 130                 135
Lys Arg Tyr Phe Arg Arg Ile Arg Leu Tyr Leu Thr Glu Lys Lys
AAG AGG TAC TTC AGG AGA ATC CGT CTG TAC CTG ACA GAG AAG AAA
                    140                 145                 150
Tyr Ser Asp Cys Ala Trp Glu Ile Val Arg Val Asp Ile Met Arg
TAC AGT GAC TGT GCC TGG GAG ATT GTC AGA GTG GAC ATC ATG AGA
                    155                 160                 165
Ser Phe Ser Ser Ser Ala Asn Leu Gln Gly Arg Leu Gly Met Lys
TCC TTC TCT TCA TCA GCA AAC CTG CAA GGA AGG TTA GGA ATG AAG
                    170                 175                 180
Asp Gly Asp Leu Gly Ser Pro +
GAT GGA GAC CTG GGG TCA CCT TGA                     Formel IV
 
5 10 15
Cys Asp Leu Pro His Thr His Ser Leu Gly Asn Thr Arg Val Leu
TGT GAC CTG CCT CAC ACC CAT AGC CTG GGC AAC ACA AGG GTC TTG
20 25 30
Met Leu Leu Gly Gln Met Arg Arg Ile Ser Pro Phe Ser Cys Leu
ATG CTC CTG GGA CAA ATG AGG AGA ATC TCC CCC TTC TCC TGC CTG
35 40 45
Lys Asp Arg Asn Asp Phe Gly Phe Pro Gln Glu Val Phe Asp Gly
AAG GAC AGA AAT GAC TTT GGA TTC CCC CAG GAG GTG TTT GAC GGC
50 55 60
Asn Gln Phe Arg Lys Pro Gln Ala Ile Ser Ala Val His Glu Thr
AAC CAG TTC CGG AAG CCT CAA GCC ATC TCT GCG GTC CAT GAG ACG
65 70 75
Ile Gln Gln Ile Phe His Leu Phe Ser Thr Asp Gly Ser Ser Ala
ATC CAA CAG ATC TTC CAC CTC TTC AGC ACA GAC GGC TCG TCT GCT
80 85 90
Ala Trp Asp Glu Ser Leu Leu Asp Lys Leu Tyr Thr Gly Leu Tyr
GCC TGG GAC GAG AGC CTC CTA GAC AAG CTC TAC ACT GGA CTC TAT
95 100 105
Gln Gln Leu Thr Glu Leu Glu Ala Cys Leu Ser Gln Glu Val Gly
CAG CAG CTG ACT GAG CTG GAA GCC TGT CTG AGC CAG GAG GTG GGG
110 115 120
Val Glu Glu Thr Pro Leu Met Asn Glu Asp Ser Leu Leu Ala Val
GTG GAA GAG ACG CCC CTG ATG AAC GAG GAC TCC CTG CTG GCT GTG
125 130 135
Arg Arg Tyr Phe Gln Arg Ile Ala Leu Tyr Leu Gln Glu Lys Lys
AGG AGA TAC TTC CAA AGA ATC GCT CTC TAT CTG CAA GAG AAG AAA
140 145 150
Tyr Ser Pro Cys Ala Trp Glu Ile Val Arg Ala Glu Ile Met Arg
TAC AGC CCT TGT GCC TGG GAG ATC GTC AGA GCA GAA ATC ATG AGA
155 160 165
Ser Phe Ser Ser Ser Thr Asn Leu Pro Gln Ser TER
TCC TTC TCT TCA TCC ACA AAC TTG CCG CAG AGT TAA

Formel V
 
5 10 15
Cys Asp Leu Pro His Thr His Ser Leu Gly Asn Thr Arg Val Leu
TGT GAC CTG CCT CAC ACC CAT AGC CTG GGC AAC ACA AGG GTC TTG
20 25 30
Met Leu Leu Gly Gln Met Arg Arg Ile Ser Pro Phe Ser Cys Leu
ATG CTC CTG GGA CAA ATG AGG AGA ATC TCC CCC TTC TCC TGC CTG
35 40 45
Lys Asp Arg Asn Asp Phe Gly Phe Pro Gln Glu Val Phe Asp Gly
AAG GAC AGA AAT GAC TTT GGA TTC CCC CAG GAG GTG TTT GAC GGC
50 55 60
Asn Gln Phe Arg Lys Pro Gln Ala Ile Ser Ala Val His Glu Thr
AAC CAG TTC CGG AAG CCT CAA GCC ATC TCC GCG GTC CAT GAG ACG
65 70 75
Ile Gln Gln Ile Phe His Leu Phe Ser Thr Asp Gly Ser Ser Ala
ATC CAA CAG ATC TTC CAC CTC TTC AGC ACA GAC GGC TCG TCT GCC
80 85 90
Ala Trp Asp Glu Ser Leu Leu Asp Lys Leu Tyr Thr Gly Leu Tyr
GCC TGG GAC GAG AGC CTC CTA GAC AAG CTC TAC ACT GGA CTC TAT
95 100 105
Gln Gln Leu Thr Glu Leu Glu Ala Cys Leu Ser Gln Glu Val Gly
CAG CAG CTG ACT GAG CTG GAA GCC TGT CTG AGC CAG GAG GTG GGG
110 115 120
Val Glu Glu Thr Pro Leu Met Asn Glu Asp Ser Leu Leu Ala Val
GTG GAA GAG ACG CCC CTG ATG AAC GAG GAC TCC CTG CTG GCT GTG
125 130 135
Arg Arg Tyr Phe Gln Arg Ile Thr Leu Tyr Leu Gln Glu Lys Lys
AGG AGA TAC TTC CAA AGA ATC ACT CTC TAT CTG CAA GAG AAG AAA
140 145 150
Tyr Ser Pro Cys Ala Trp Glu Ile Val Arg Ala Glu Ile Met Arg
TAC AGC CCT TGT GCC TGG GAG ATC GTC AGA GCA GAA ATC ATG AGA
155 160
Ser Phe Ser Ser Ser Thr Asn Leu Pro Gln Ser TER
TCC TTC TCT TCA TCC ACA AAC TTG CCG CAA AGT TAA

Formel VI
 und

5 10 15
Cys Asp Leu Pro Gln Asn His Ile Leu Val Ser Arg Lys Asn Phe
TGT GAC CTG CCT CAG AAC CAC ATC CTG GTT AGC AGG AAG AAC TTC
20 25 30
Val Leu Leu Gly Gln Met Ser Arg Ile Ser Ser Ala Ile Cys Leu
GTG CTT CTG GGC CAA ATG AGC AGA ATC TCC TCC GCA ATC TGT CTG
35 40 45
Lys Asp Arg Lys Asp Phe Arg Phe Pro Gln Asp Met Ala Asp Gly
AAG GAC AGA AAA GAC TTC AGG TTC CCC CAG GAC ATG GCG GAT GGC
50 55 60
Arg Gln Phe Pro Glu Ala Gln Ala Ala Ser Val Leu His Glu Met
AGG CAG TTC CCA GAG GCC CAG GCC GCG TCT GTC CTC CAC GAG ATG
65 70 75
Leu Gln Gln Ile Phe Ser Leu Phe His Thr Glu Arg Ser Ser Ala
CTC CAG CAG ATC TTC AGC CTC TTC CAC ACA GAG CGC TCG TCT GCT
80 85 90
Ala Trp Asn Thr Thr Leu Leu Asp Glu Leu Cys Thr Gly Leu Leu
GCC TGG AAC ACG ACC CTC CTG GAC GAA CTC TGC ACG GGA CTC CTT
95 100 105
Arg Gln Leu Glu Asp Leu Asp Thr Cys Leu Glu Gln Glu Met Gly
CGG CAG CTG GAA GAC CTG GAC ACC TGT TTG GAG CAG GAG ATG GGA
110 115 120
Glu Glu Glu Ser Ala Leu Gly Thr Val Arg Pro Thr Leu Ala Val
GAG GAA GAA TCT GCC CTG GGA ACT GTG CGC CCT ACA CTG GCC GTG
125 130 135
Lys Arg Tyr Phe Arg Gly Ile His Leu Tyr Leu Lys Glu Lys Lys
AAG AGG TAC TTC CGG GGG ATC CAT CTC TAC CTG AAA GAG AAG AAA
140 145 150
Tyr Ser Asp Cys Ala Trp Glu Ile Val Arg Met Glu Ile Met Arg
TAC AGT GAC TGT GCC TGG GAG ATT GTC CGA ATG GAA ATC ATG AGA
155 160 165
Ser Phe Ser Ser Ser Ala Asn Leu Gln Gly Arg Leu Arg Met Lys
TCC TTC TCT TCA TCA GCA AAC CTG CAA GGA AGG TTA AGA ATG AAG
170
Asp Gly Asp Leu Gly Ser Pro TER
GAT GGA GAC CTG GGC TCA CCT TGA

Formel VII

Die Aufgabe der Erfindung wurde dadurch gelöst, daß man hochmolekulare DNA aus dem Gewebe der genannten Tiere, vorzugsweise aus der Leber nach einem modifizierten Verfahren gemäß Blin und Stafford (18) isolierte und mit Hilfe spezieller Endonucleasen statistisch fragmentierte. Die so erhaltenen unterschiedlich großen Fragmente wurden nach der Größe fraktioniert, vorzugsweise zur Bildung von 10-23 kb Fragmenten, um in einem Vektor, beispielsweise in einem Lambda- Vektor geklont zu werden. Anschließend wurden diese Vektoren in einem Bakterium, vorzugsweise E.coli, vermehrt.

Die Pferde-DNA wurde mit Hilfe der für reifes Humaninterferon-alpha2ARG kodierenden DNA und dem für humanes ß-Interferon kodierenden cDNA unter nicht stringenten Bedingungen gescreent.

Durch die niedrige Stringenz wurden auch Klone erfaßt, die in ihren Sequenzen erhebliche Unterschiede zu dem HuIFN-alpha-2Arg bzw.HuIFN-ß aufweisen.

Bei der Untersuchung der Pferde-DNA mit dem humanem-alpha-Gen wurden wie bei Rindern, Schweinen und Menschen durch Southern-Analyse mehrere Banden festgestellt, so daß man davon ausgehen kann, daß es auch beim Pferd eine Klasse von alpha-Interferongenen geben muß.

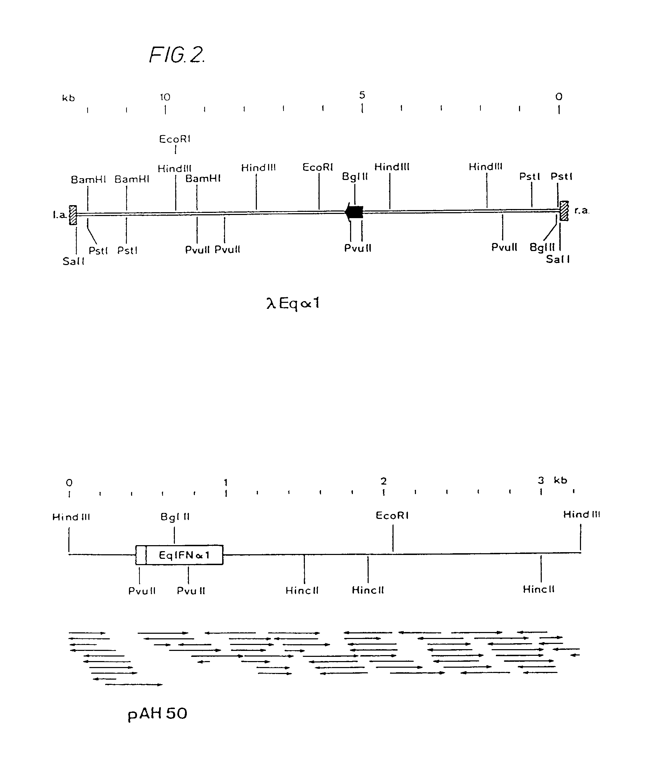

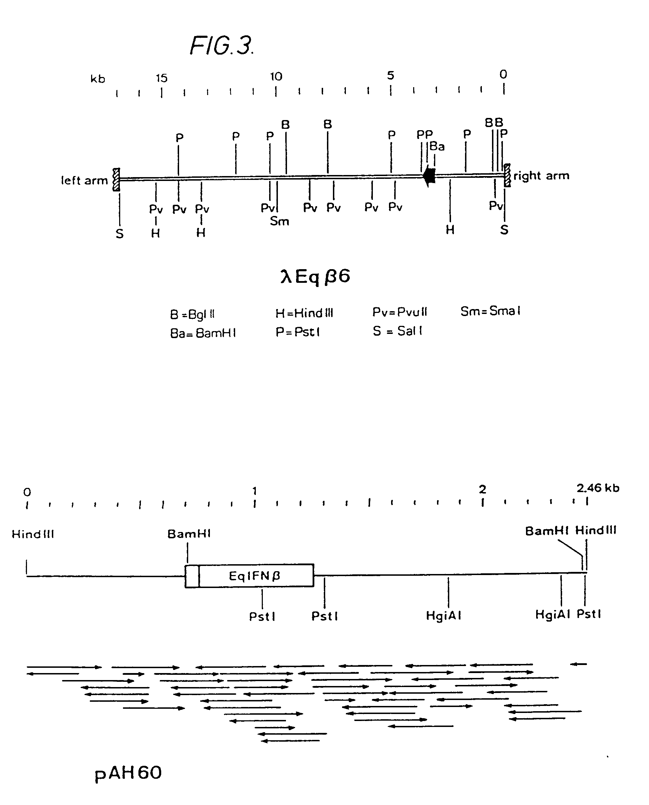

Von den hybridisierenden Rekombinanten wurde Phagen DNA hergestellt und von den resultierenden Klonen Eq-alphal, Eq-beta6 wurden Restriktionskarten erstellt (Fig. 2 und 3). Weitere mit der humanen IFN Probe hybridisierdende Lambda-Klone wurden Eq-alpha9, Eq-alpha16, Eq-alpha20 und Eq-alpha24 genannt. Ein 3,2 kb Hindlll-Fragment aus dem Klon Eq-alphal, ein 4,5 kb Pvull-Fragment des Klons Eq-beta6, ein 2,8 kb langes Hindlll-Fragment des Kloms Eq-alpha9, ein 3,3 kb EcoRl- und ein 5,5 kb langes EcoRl-Fragment des Klons Eq-alpha16, ein 2,2 kb EcoRI-Fragment des Klons Eq-alpha20 bzw. ein 3,2 kb langes Hindlll-Fragment aus dem Eq-alpha24 wurden in einem Vektor, beispielsweise pUC8, subkloniert und anschließend in einen Wirtsorganismus, beispielsweise E.coli JM101 transformiert. Die Isolierung der korrekten Phenotypen ergab die Plasmide pAH50, pAH60, pRH61, pRH62, pRh63, pRH82 bzw. pRH83 die als Inserts die für die Pferde-Interferone kodierenden Sequenzen enthalten.

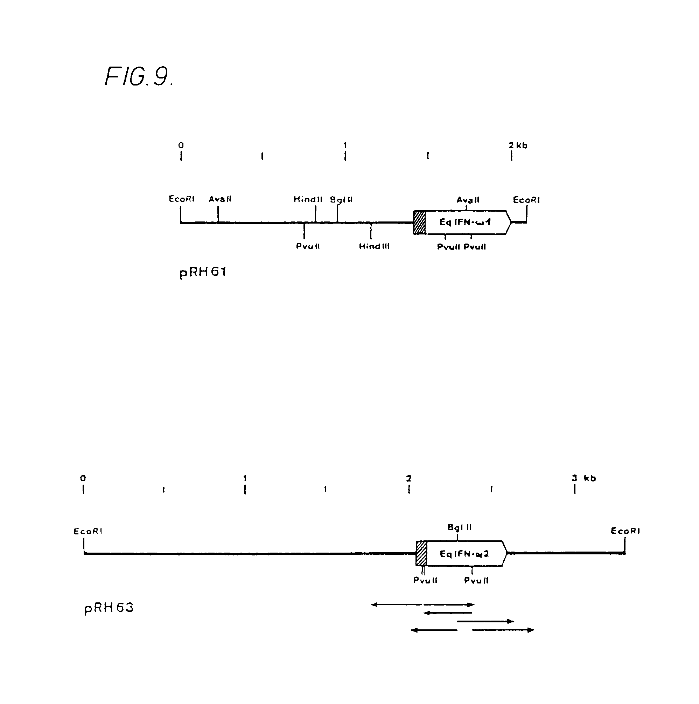

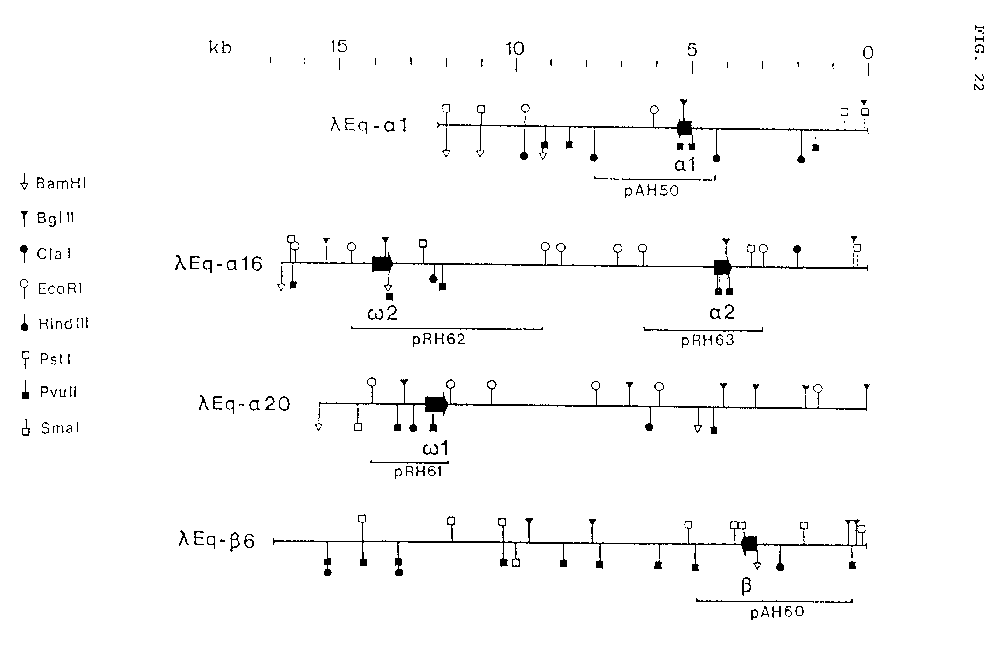

Die Restriktionskarten für pRH61, pRH63 sind in Figur 9 und für pRH62 in Figur 22 abgebildet.

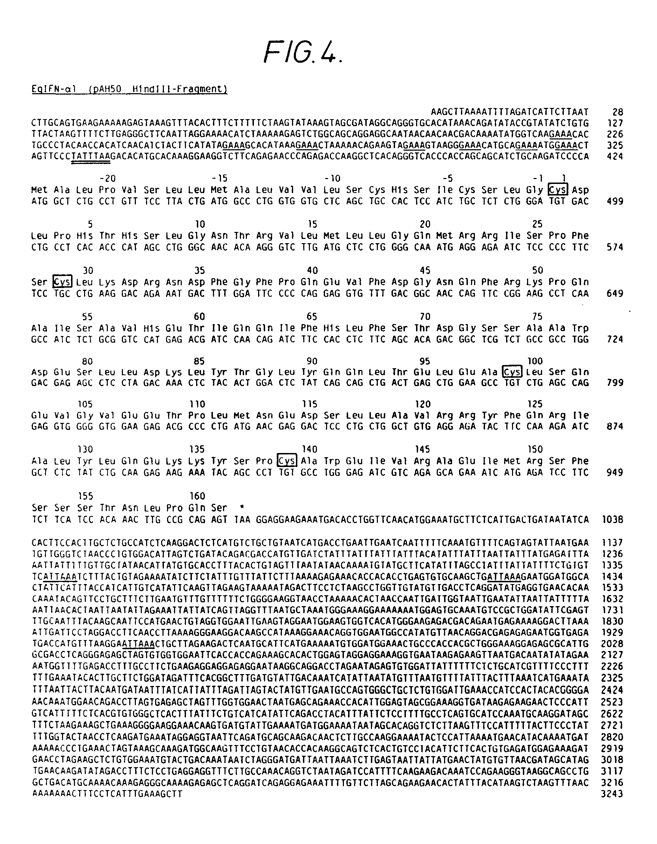

Die Inserts der Plasmide wurden nach der Dideoxy-Methode von Sanger (23) nach dem "Shotgun-Verfahren" sequenziert. Die Teilsequenzen dieser Inserts wurden mit Hilfe eines modifizierten Computerprogrammes zu einer Totalsequenz vereint (Fig. 4,8,10,12,23,26 und 27).

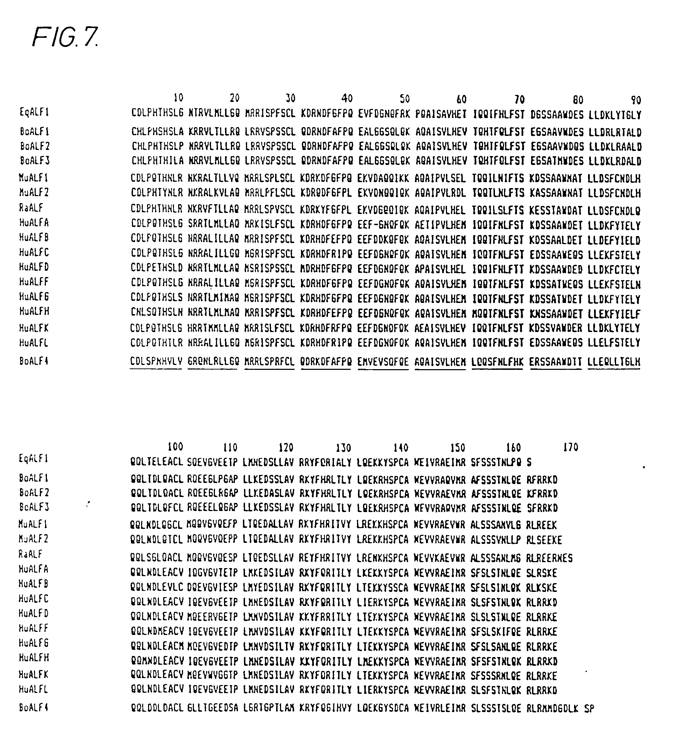

Der längste offene Leseraster für das Eq-IFN-alpha-Gen aus dem Klon Eq-alphal kodiert ein Polypeptid mit 184 Aminosäuren. Beachtenswert ist die signifikante Homologie zu bekannten alpha-Interferonen anderer Spezies. Wie bei den humanen, bovinen und murinen alpha-Interferonen besteht dieses Pferde-alpha-Interferon aus einem hydrophoben Signalpeptid mit 23 Aminosäuren, das einem reifen Protein mit überraschenderweise nur 161 Aminosäuren vorausgeht (Eq-IFN-alpha1). Vier Cysteinreste an den Stellen 1, 29, 99 und 139 sind zwischen den Spezies Pferd, Rind, Maus, Ratte und Mensch exakt erhalten (Fig 7). Die Verkürzung dieses Pferde-alpha-Interferons auf 161 Aminosäuren dürfte durch die Deletion einer Base nach der 159. Aminosäure bewirkt worden sein, ohne die die Transkription bis zur 166. Aminosäure, bis zum Stopcodon TGA weitergeschrieben worden wäre.

Dieser Befund weist darauf hin, daß die Polypeptidkette für reifes Pferde-Interferon-alphal eine Länge von 161 Aminosäuren haben kann, daß jedoch auch andere Formen mit bis zu 166 Aminosäuren existieren können. Auch diese Peptide sind selbstverständlich Gegenstand der vorliegenden Erfindung.

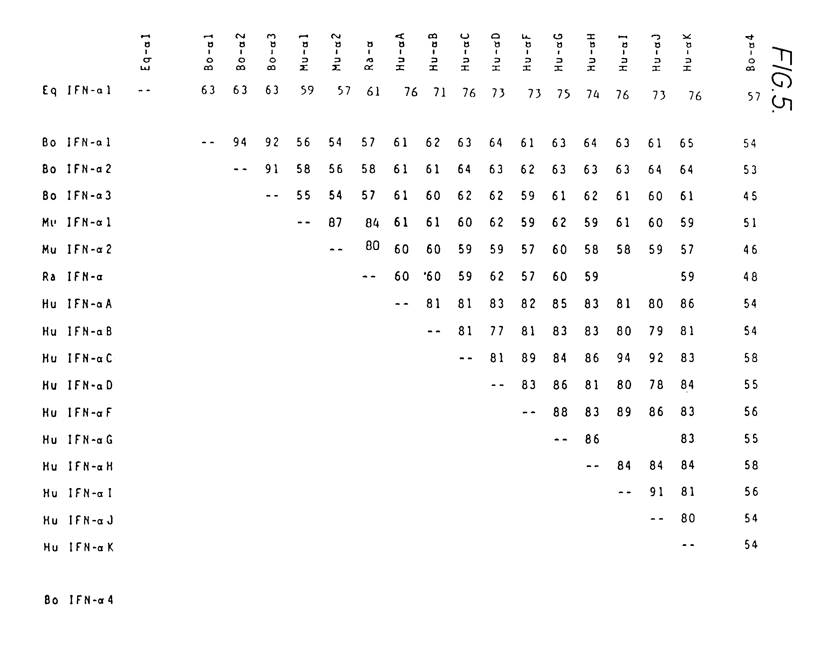

Uberraschenderweise zeigte sich beim paarweisen Vergleich der Aminosäuresequenzen, daß das Pferde-Interferon-alphal zu den menschlichen alpha-Interferonen eine größere Homologie aufweist (71-77%) als zu den Rinder- (57-67%), Ratten- (61%) oder Maus-alpha-Interferonen (54-59%) (Fig. 5). Die Homologie zwischen den verschiedenen alpha-Interferonen einer Gattung ist deutlich größer, als zwischen denen verschiedener Spezies (z.B. Mensch 77-100%, Rind 91-99%).

Der längste offene Leseraster für das Eq-IFN-alpha-Gen aus dem Klon Eq-a!pha16 kodiert ebenfalls ein Polypeptid mit 184 Aminosäuren (Signalpeptid 23 Aminosäuren, reifes Protein 161 Aminosäuren).

Die DNA-Sequenz des Klons pRH63 ist in der proteinkodierenden Region sehr ähnlich der des Klons pAH50, was für die Expression des Genes ausgenutzt werden kann (siehe Beispiel M; Fig. 11 und 15). Das Interferon aus klon pRH63 wurde aufgrund der hohen Homologie mit Eq-IFN-alphal (aus Klon pAH50) Eq- IFN-alpha2 benannt. Reifes Eq-IFN-alpha2 weist gegenüber Eq-IFN-alphal nur zwei unterschiedliche Aminosäurereste am C-terminalen Ende auf, wobei sich durch den Austausch an Position 151 Ser -- Cys in Eq-IFN-alpha2 ein fünfter Cysteinrest an einer bisher bei keinem anderen Interferon beobachteten Position befindet (siehe Fig. 19).

Ansonsten trifft das für das Eq-IFN-alphal Gesagte auch auf das Eq-IFN-alpha2 zu!

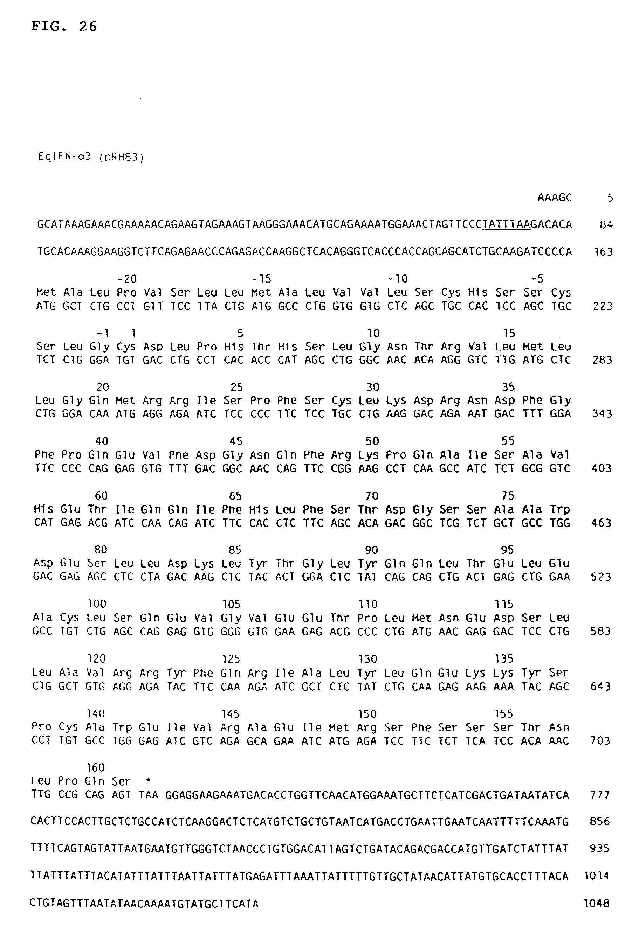

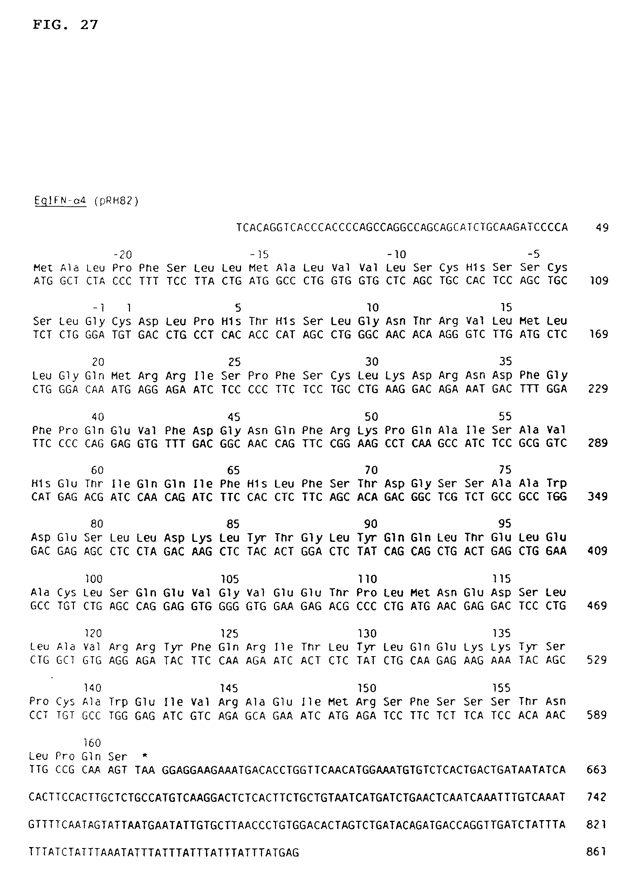

Die aus den Plasmiden pRH82 und pRH83 ermittelten DNA-Sequenzen (Fig. 26 und 27) ergaben, daß es sich um zwei funktionelle alpha-Interferongene des Pferdes handelt, die Eq-IFN-alpha3 (aus pRH83) bzw. Eq-IFN-alpha4 (aus pRH82) benannt wurden. Sie kodieren für Polypeptide bestehend aus einem 23 Aminosäuren langen Signalpeptid gefolgt von einem reifen Protein von 161 Aminosäuren. Der Homologiegrad der DNA-Sequenzen zwischen Eq-IFN-alphal (pAh50) und Eq-IFN-alpha3 (pRH83) bzw. zwischen Eq- IFN-alpha2 (pRH62) und Eq-IFN-alpha4 (pRH82) ist außerordentlich hoch. Die Aminosäuresequenzen der reifen Proteine sind vollkommen gleich. Durch die Degenerierung des genetischen Codes führen die Unterschiede in der Nukleotidsequenz in diesem Fall zu keinem Austausch in der Aminosäuresequenz. Möglicherweise handelt es sich bei Eq-IFN-alpha3 um eine allele Form des Eq-IFN-alphal.

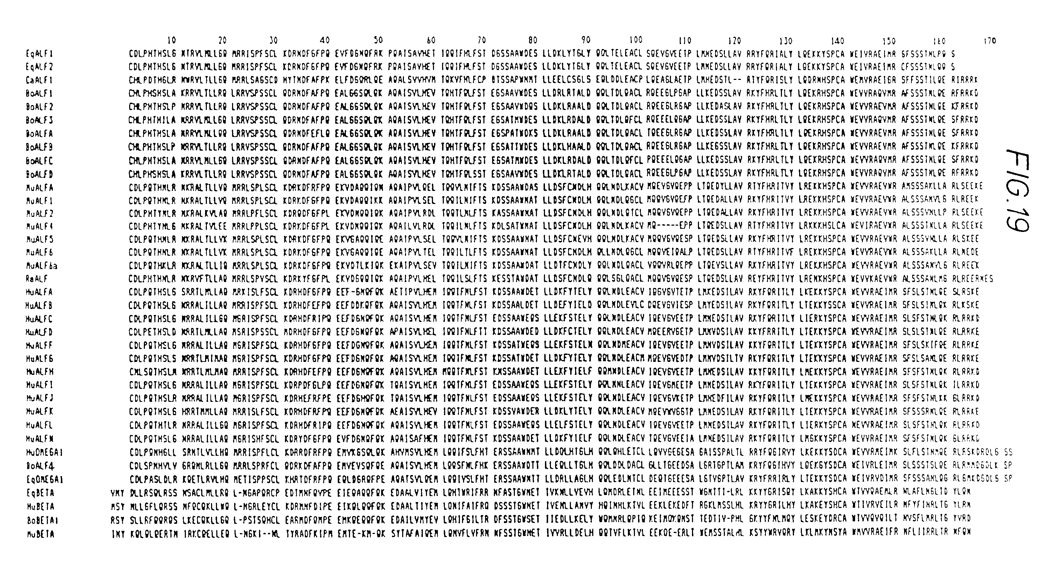

Das DNA-Fragment des Eq-alpha20 enthält die kodierende Sequenz für ein Protein mit 172 Aminosäuren und ein hydrophobes Signalpeptid mit 23 Aminosäuren. An Position 78-80 des reifen Proteins befindet sich eine potentielle N-Glycosilierungs-stelle Asn-Thr-Thr, die genau der von Eq-IFN-ß, Hu-IFN-ß, Mu-IFN-β, Mu-IFN-alpha1,2,4,5,6 entspricht (Fig. 19).

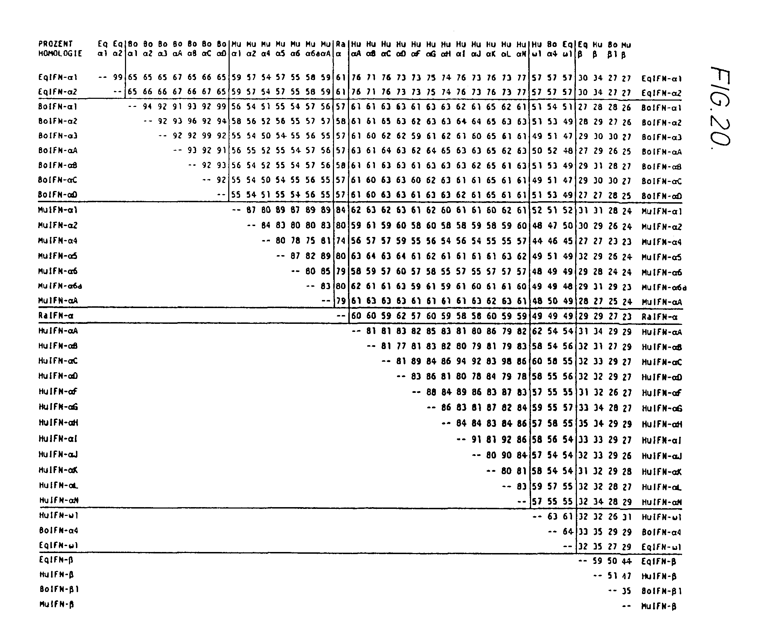

Die Proteinsequenzen in dieser Abbildung wurden so angeordnet, daß die größtmögliche Homologie zwischen den einzelnen Interferonen erreicht wurde. Zum Vergleich von IFN-alpha und IFN-ß Sequenzen wurden letztere um 3 Aminosäuren verschoben und eine Lücke eingeführt. Der paarweise Vergleich der Aminosäuresequenzen in Fig. 20 erfolgte ausgehend von dieser Anordnung über die längste gemeinsame Länge der Proteine.

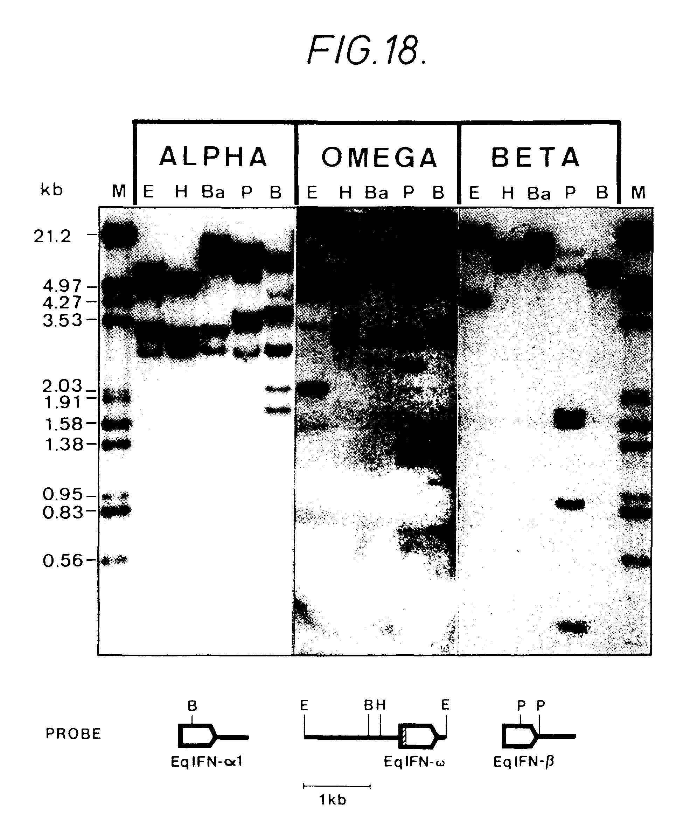

Aus den Abbildungen 19 und 20 geht hervor, daß das von der DNA-Sequenz des Klones pRH63 kodierte Protein mit den Typ I Interferonen verwandt ist (alpha- und ß-IFN). Die Charakteristika von 172 Aminosäuren, Glykosilierungsstelle an Position 78 und der etwa gleich großen Homologie der Interferone dieser Klasse zwischen verschiedenen Spezies (Mensch, Rind, Pferd) wie zwischen diesen längeren Interferonen und den alpha-Interferonen innerhalb einer Gattung, sowie die unterschiedlichen Sätze von hybridisierenden DNA-Fragmenten mit alpha-Interferon und Proben aus dem Klon pRH63 (Fig.18) lassen die Annahme zu, daß das Insert des Klons pRH63 einer neuen Klasse von Typ I Interforonon angehört, welche als Interferon-omega bezeichnet wird (33). Diese Bezeichnung ist weniger verwirrend als die von Capon et al. (34) verwendete: Typ I, Klasse 11 Interferon, welche zur Verwechslung mit Typ 11 Interferon (IFN-gamma) führen könnte.

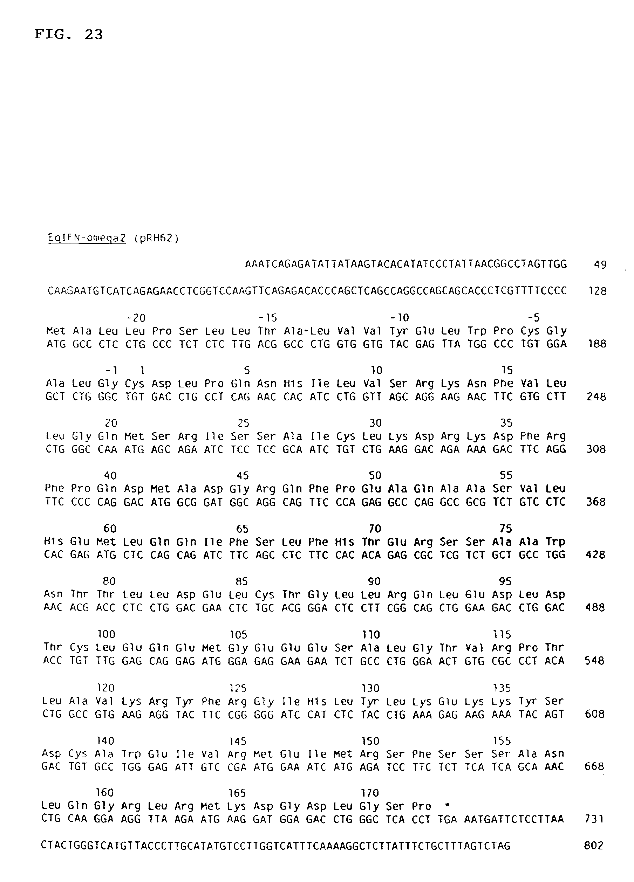

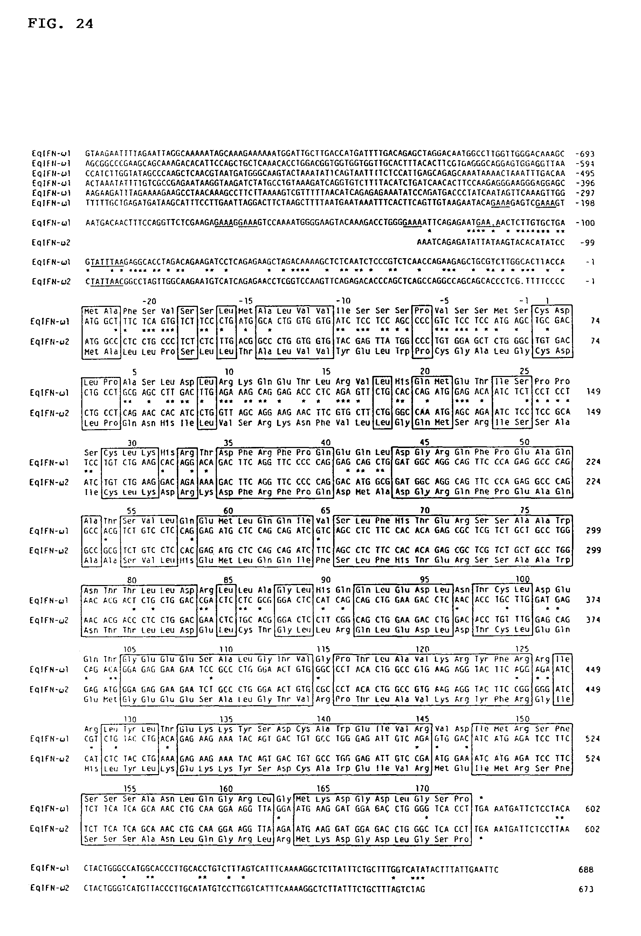

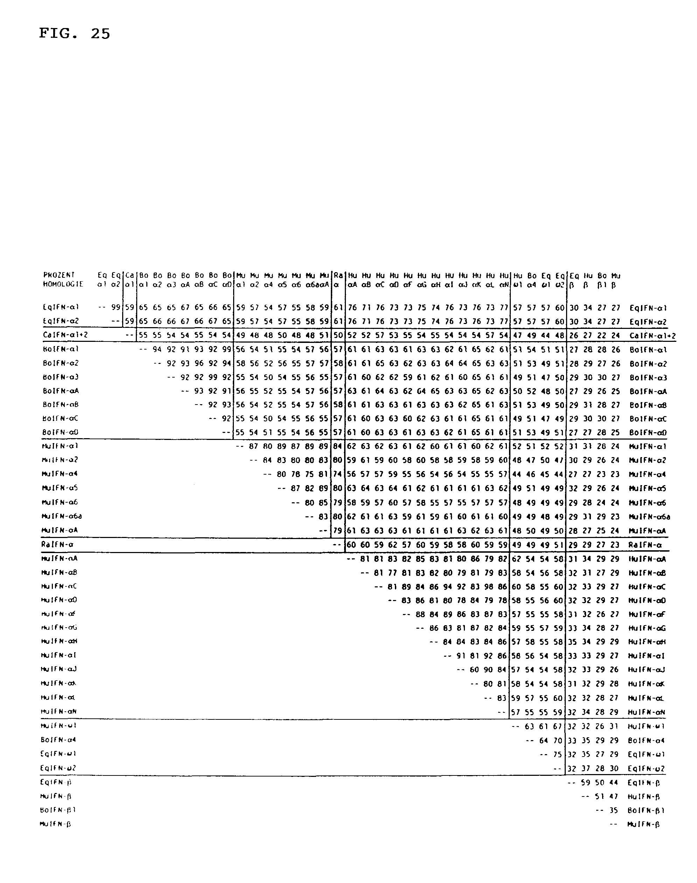

Die aus dem Plasmid pRH62 ermittelte DNA-Sequenz (Fig.23) enthält die gesamte kodierende Region für ein funktionelles Pferde-Interferon-Gen, das aufgrund der Homologie zu dem Interferon aus Plasmid pRH61 (Fig.24) und Hu-IFN-omegal Eq-IFN-omega2 benannt wurde. Eq-IFN-omega2 enthält überraschenderweise an Position 86 des reifen Proteins einen fünften Cysteinrest. Die Homologie zwischen den beiden equinen omega-Interferonen ist ab der 29. Aminosäure des reifen Proteins sehr hoch, die vier Cysteinreste sowie die potentielle N-Glykosylierunsstelle an Position 78-80 (Asn-Thr-Thr) sind vollkommen konserviert (Fig.23,24). Die Aminosäure-Homologe zu den Interferonen der omega-Klasse von Rind und Mensch ist höher (61-70%) als zu den equinen alpha-Interferonen (57-60%, Fig.25).

Die Sequenz des Pferde-beta-Interferons wurde analog der des alpha bestimmt. Der längste offene Leseraster für das beta-IFN-Gen kodiert für ein Polypeptid mit 186 Aminosäuren, wobei auch in diesem Fall die Homologie zu bekannten beta-Interferonen anderer Spezies zu beobachten ist. Wie bei dem humanen, den 3 bovinen und den murinen beta-Interferonen weist das Pferde-beta-Interferon ein hydrophobes Signalpeptid mit 21 Aminosäuren auf.

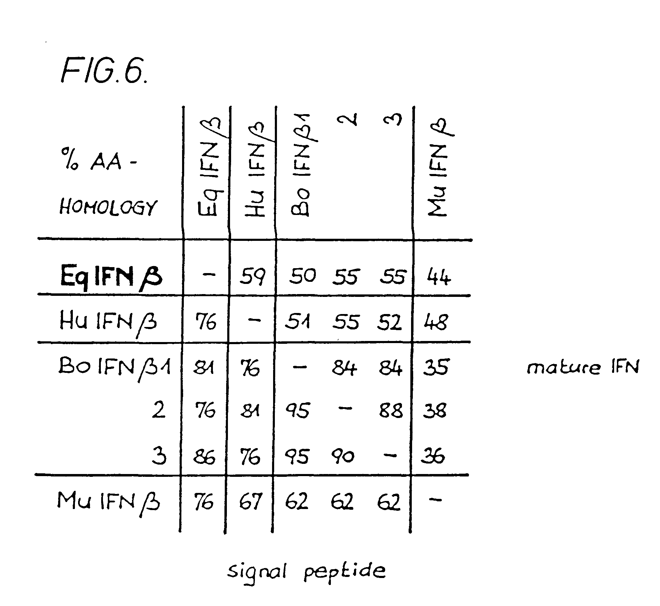

Uberraschenderweise zeigte sich auch beim beta-Interferon beim paarweisen Vergleich der Aminosäuresequenzen, daß das Pferde-beta-Interferon zu dem menschlichen beta-Interferon eine größere Homologie aufweist (59%) als zu den Rinder- (50-55%) oder Maus-beta-Interferonen (44%) (Fig. 6).

Andererseits, trotz der überraschend hohen Homologie Pferd/Mensch-beta-Interferon, fehlt wie bei den drei bovinen beta-Interferonen die im humanen beta-Interferon an Position 119 stehende Aminosäure auch im Pferde-beta-Interferon!

Das Pferde-beta-Interferon trägt zwei potentielle N-Glykosylierungsstellen: an Position 80 des reifen Proteins (ASN-GLU-THR, genau wie beim humanen und Maus-beta-Interferon) und an Position 115 (ASN-THR-THR). Bei den bovinen beta-Interferonen sind zwei mögliche N-Glykosylierungsstellen an Position 110 (ASN-PHE-THR bzw. ASN-SER-PHE) und 152 (ASN-VAL-SER bzw. ASN-PHE-SER) lokalisiert.

Wie beim Rind und beim Menschen sind die 3 Cysteinreste exakt erhalten geblieben (Position 17, 31 und 140 bzw. 141 beim Menschen).

An dieser Stelle sei erwähnt, daß es sich bei den erfindungsgemäßen Interferonen nicht nur um die reifen Interferone, die im einzelnen beschrieben sind, handelt, sondern ebenfalls um jedwede Modifikation dieser Polypeptide, die die Pferde -IFN-Aktivität nicht wesentlich verändert. Diese Modifikationen beinhalten z.B. Verkürzung des Moleküls z.B am N- oder C-terminalen Ende, Austausch von Aminosäuren durch andere Reste, chemische oder biochemische Bindungen des Moleküls an andere Moleküle, die inert oder aktiv sind. Bei den letztgenannten Modifikationen kann es sich beispielsweise um Hybridmoleküle aus einem oder mehreren erfindungsgemäßen Interferonen und/oder bekannten a- oder ß-Interferonen handeln.

Gegenstand der Erfindung sind daher nicht nur Gen-Sequenzen, die spezifisch für die erfindungsgemäßen Interferone codieren, sondern ebenfalls Modifikationen, die leicht und routinemäßig durch Mutation, Abbau, Transposition oder Addition erhalten werden können. Jede Sequenz, die für die erfindungsgemäßen Interferone codiert (d.h. die das biologische Aktivitätsspektrum aufweist, das hier beschrieben ist) und im Vergleich mit den gezeigten degeneriert ist, ist ebenfalls eingeschlossen; Fachleute auf diesem Gebiet sind in der Lage, DNA-Sequenzen der codierenden Regionen zu degenerieren. Ebenso ist jede Sequenz, die für ein Polypeptid mit dem Aktivitätsspektrum der erfindungsgemäßen Interferone codiert, und die mit den gezeigten Sequenzen (oder Teilen davon) unter stringenten Bedingungen hybridisiert (beispielsweise Bedingungen, die für mehr als 85 %, bevorzugt mehr als 90 % Homologie selektieren) beinhaltet.

Die Hybridisierungen werden in 6 x SSC/5 x Denhardt's Lösung/0,1 % SDS bei 65 ° C durchgeführt. Der Grad der Stringenz wird im Waschschritt festgelegt. So sind für eine Selektionierung auf DNA-Sequenzen mit ca. 85 % oder mehr Homologie die Bedingungen 0,2 x SSC/0,01 %, SDS/65°C und für eine Selektionierung auf DNA-Sequenzen mit ca. 90 % oder mehr Homologie die Bedingungen 0,1 x SSC/0,01 % SDS/65 ° C geeignet.

Erfindungsgemäße Interferon-Gene können unter Bedingungen in jeden Organismus eingebracht werden, die zu hohen Ausbeuten führen. Geeignete Wirte und Vektoren sind dem Fachmann bestens bekannt; als Beispiel sei auf die EP-A-0.093.619 hingewiesen.

Insbesondere sind Prokaryoten für die Expression bevorzugt, beispielsweise E. coli K 12, Stamm 294 (ATCC Nr. 31 446) oder E. coli X1776 (ATCC Nr. 31.537). Ebenso wie die vorerwähnten Stämme können auch E. coli W 3110 (F-, Lambda-, Prototroph, ATCC Nr. 27325), Bazillen wie Bacillus subtilis, und andere Enterobacteriaceae, wie Salmonella typhimurium oder Serratia marcescens und verschiedene Pseudomonaden verwendet werden.

Im allgemeinen können Plasmid-Vektoren, die Replikon und Kontrollsequenzen, die aus Spezies stammen, die kompatibel mit den Wirtszellen sind, enthalten, in Verbindung mit diesen Wirten verwendet werden. Der Vektor trägt üblicherweise neben einer Replikationsstelle Erkennungssequenzen, die es ermöglichen, die transformierten Zellen phenotypisch zu selektieren. Zum Beispiel wird E. coli üblicherweise mit pBR322 transformiert, ein Plasmid, das aus E. coli Spezies stammt (Bolivar, et al., Gene 2, 95 (1977)). pBR322 enthält Gene für Ampicillin- und Tetracyclin-Resistenz und liefert damit einfache Mittel, transformierte Zellen zu identifizieren. Das pBR322-Plasmid oder auch andere Plasmide müssen außerdem von sich aus Promotoren enthalten oder müssen dahingehend so modifiziert sein, daß sie Promotoren enthalten, die vom mikrobiellen Organismus zur Expression ihrer eigenen Proteine verwendet werden können. Die Promotoren, die am häufigsten bei der Herstellung rekombinanter DNA verwendet werden, beinhalten die Beta-Lactamase (Penicillinase) und Lactose-Promotor-Systeme (Chang et al., Nature 275, 615 (1978): Itakura et al., Science 198, 1056 (1977): Goeddel et al., Nature 281, 544 (1979)) und Tryptophan(trp) Promotor-Systeme (Goeddel et al., Nucleic Acids Res. 8, 4057 (1980); EP-A-0.036.776). Während die erwähnten die gebräuchlichsten Promotoren sind, sind darüber hinaus auch andere mikrobielle Promotoren entwickelt und benutzt worden. Die Gen-Sequenz für die erfindungsgemäßen Interferone kann beispielsweise unter der Kontrolle des Leftward-Promotors des Bakteriophagen Lambda (P_{L}) eingesetzt werden. Dieser Promotor ist einer der als besonders stark bekannten Promotoren, der steuerbar ist. Die Steuerung wird möglich durch den Lambda-Repressor, von dem benachbarte Restriktionsschnittstellen bekannt sind.

Ein temperaturempfindliches Allel dieses Repressor-Gens kann in einem Vektor, der eine vollständige IFN-omega-Sequenz enthält, eingefügt werden. Wird die Temperatur auf 42 ° C erhöht, wird der Repressor inaktiviert und der Promotor bis zu seiner maximalen Konzentration exprimiert. Die Summe der mRNA, die unter diesen Bedingungen produziert wird, sollte ausreichend sein, um eine Zelle zu erhalten, die unter ihren neuen synthetischen Ribonukleinsäuren ungefähr 10 % enthält, die von dem P_{L}-Promotor stammt. Auf diese Weise ist es möglich, eine Clon-Bank zu etablieren, in der eine funktionelle IFN-Sequenz in Nachbarschaft zu einer Ribosom-Bindungsstelle in variierenden Abständen zu dem Lambda-P_{L}-Promotor plaziert wird. Diese Klone können dann überprüft und der mit der höchsten Ausbeute selektiert werden.

Die Expression und Translation einer Sequenz codierend für die erfindungsgemäßen Proteine kann auch unter Kontrolle anderer Regulationssysteme, die als "homolog" zu dem Organismus in seiner untransformierten Form gelten können, ablaufen. So enthält z.B. chromosomale DNA von einem Lactoseabhängigen E. coli ein Lactose oder Lac-Operon, der durch Ausschüttung des Enzyms Beta-Galactosidase den Lactose-Abbau ermöglicht.

Die Lac-Kontrollelemente können aus dem Bacteriophagen Lambda-plac5, der infektös für E. coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicine Ei -Operator, Galactose-Operator, alkalischer Phosphatase-Operator, trp-Operator, Xylose-A-Operator, tac-Promotor u.ä..

Zusätzlich zu Prokaryoten können auch eukaryotische Mikroorganismen, wie Hefekulturen verwendet werden Saccharomyces cerevisiae ist die am meisten verwendete unter den eukaryotischen Mikroorganismen, obwohl eine Anzahl anderer Spezies allgemein erhältlich ist. Zur Expression in Saccharomyces wird beispielsweise das Plasmid YRp7 (Stinchcomb et al. Natur 282, 39 (1979); Kingsman et al., Gene 7, 141 (1979): Tschumper et al., Gene 10, 157 (1980)) und das Plasmid YEp 13 (Bwach et al., Gene 8, 121-133 (1979)) üblicherweise verwendet. Das Plasmid YRp7 enthält das TRP1-Gen, das eine Selektionierungsmarkierung für eine Hefemutante, die unfähig ist, in trypthophanfreiem Medium zu wachsen, bereitstellt; beispielsweise ATCC Nr. 44076.

Das Vorhandensein des TRP1 Schadens als Charakteristikum des Hefe-Wirts Genoms stellt dann ein wirksames Hilfsmittel dar, um Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2- minus-Mutante verwendet werden kann, enthält. Geeignete Promotor-Sequenzen für Hefe Vektoren beinhalten die 5'-flankierende Region der Gene des ADN I (Ammerer G., Methods of Enzymology 101, 192-201 (1983)), 3-Phosphoglycerate-Kinase (Hitzeman et al., J. Biol. Chem. 255, 2073 (1980)) oder andere glykolytische Enzyme (Kawaski und Fraenkel, BBRC 108, 1107-1112 (1982)) wie Enolase, Glycerinaldehyd-3-phosphat-Dehydrogenase, Hexokinase, Pyruvat-Decarboxylase, Phosphofructokinase, Glucose-6-Phosphat-Isomerase, Phosphoglucose-Isomerase und -Glucokinase. Bei der Konzentration geeigneter Expressionsplasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA vorzusehen.

Andere Promotoren, die zudem noch den Vorteil der durch Wachstumsbedingungen kontrollierten Transkription besitzen, sind die Promotor-Regionen der Gene für Alkohol-Dehydrogenase-2, Isocytochrom C, Saure-Phosphatase abbauende Enzyme, die mit dem Stickstoff-Metabolismus gekoppelt sind, die oben erwähnte Glycerinaldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MFa1, STE2, STE3, STE5 können bei temperaturregulierten Systemen durch die Verwendung von temperaturabhängigen sir Mutationen eingesetzt werden. (Rhine PH.D. in Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Plasmid Vektor, der einen Hefekompatiblen Promotor, originäre Replikations- und Terminationssequenzen enthält, geeignet.

Zusätzlich zu Mikroorganismen sind Kulturen multizellulärer Organismen ebenfalls geeignete Wirtsorganismen. Im Prinzip ist jede dieser Kulturen einsetzbar, ob von Wirbeltier- oder wirbellosen Tierkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde (Tissue, Culture, Academic Press, Kruse and Patterson, Editors (1973)). Beispiele solch nützlicher Wirtszellinien sind VERO- und HeLa-Zellen, Hamster-Eierstock (CHO)-Zellen und W138, BHK, COS-7 und MDCK-Zellinien. Expressionsvektoren für diese Zellen enthalten üblicherweise (wenn nötig) eine Replikationsstelle, einen Promotor, der vor dem zu exprimierenden Gen lokalisiert ist, gemeinsam mit jeder notwendigen Ribosomenbindungsstelle, RNA-Splicing-Stelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material vorgesehen. Beispielsweise stammen die üblicherweise verwendeten Promotoren aus Polyoma Adenovirus 2, und besonders häufig aus Simian Virus 40 (SV 40). Die frühen und späten Endpromotoren des SV 40 sind besonders nützlich, da beide leicht aus dem Virus als Fragment zu erhalten sind, das auch noch die virale Replikationsstelle des SV 40 enthält. (Fiers et al., Nature 273, 113 (1978)). Auch können kleinere oder größere Fragmente des SV 40 verwendet werden, vorausgesetzt, sie enthalten die annähernd 250 bp lange Sequenz, die von der Hindlll Schnittstelle bis zur Bgl 1 Schnittstelle in der viralen Replikationsstelle reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontroll-Sequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Die Gene können jedoch vorzugsweise in einem Expressions-Plasmid pER103 (E. Rastl-Dworkin et al., Gene 21, 237-248 (1983) und EP-A-0.115-613 - hinterlegt bei der DSM unter der Nummer DSM 2773 am 20. Dezember 1983) oder auch in dem Plasmid parpER33 (EP-A-0.115-613) exprimiert werden, da diese Vektoren alle Regulationselemente enthalten, die zu einer hohen Expressionsrate der klonierten Gene führen.

Ausgehend von dem Expressionsplasmid parpER33 wurde die für die erhöhte Plasmidstabilität in E.coli verantwortliche "par"-Sequenz und die Tryptophan Promoter-Operator-Sequenz samt der künstlichen ribosomalen Bindungsstelle in den Plasmidvektor pAT153 eingesetzt pAT153 ist ein verkürztes Derivat des Plasmides pBR322, dem ein Abschnitt fehlt, der für die Mobilisierung von DNA notwendig ist (36).

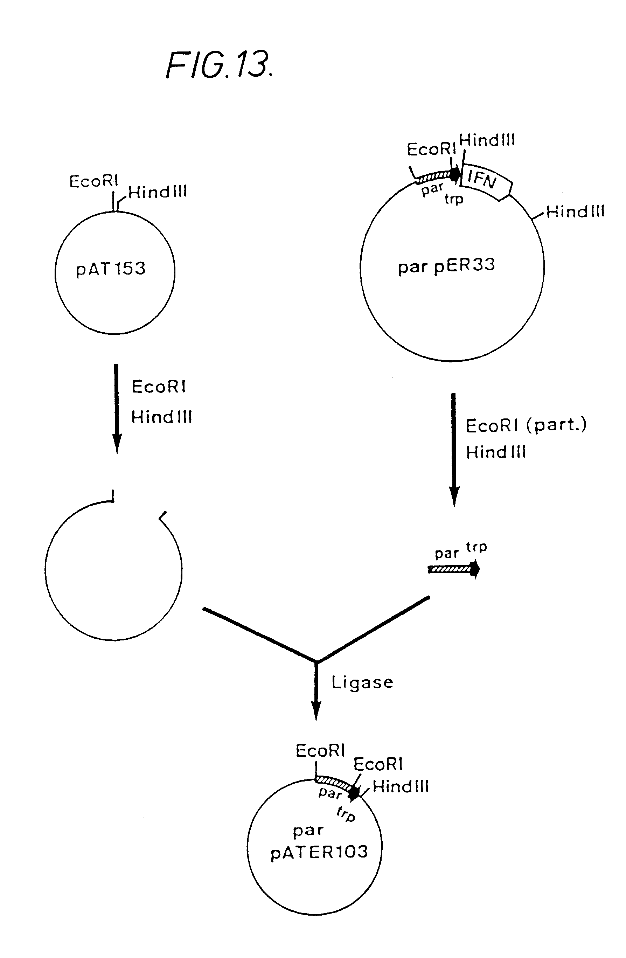

Die Vorgangsweise der Herstellung von Plasmid parpATER103 ist in Fig. 13 dargestellt. Das Plasmid parpER33 wurde mit Hindlll vollständig und mit EcoRI partiell geschnitten, das entstandene 0,47kb lange DNA-Fragment von einem Agarosegel isoliert und gereinigt und mit EcoRI und Hindlll zweifach geschnittenem pAT153 ligiert. Ein nach Transformation von E.coli HB101 erhaltenes Plasmid der gewünschten Struktur, die man durch Verdauung mit verschiedenen Restriktionsenzymen bestimmte, wurde parpATER103 benannt. Dieses Plasmid enthält den Replikationsursprung und das Ampicillin-Resistenzgen von Plasmid pAT153, sowie die für die Stabilisierung in E.coli wirksame par-Sequenz und für die effiziente Expression von Genen verwendbare Tryptophan-Promoter-Operator-Region und ribosomale Bindungsstelle.

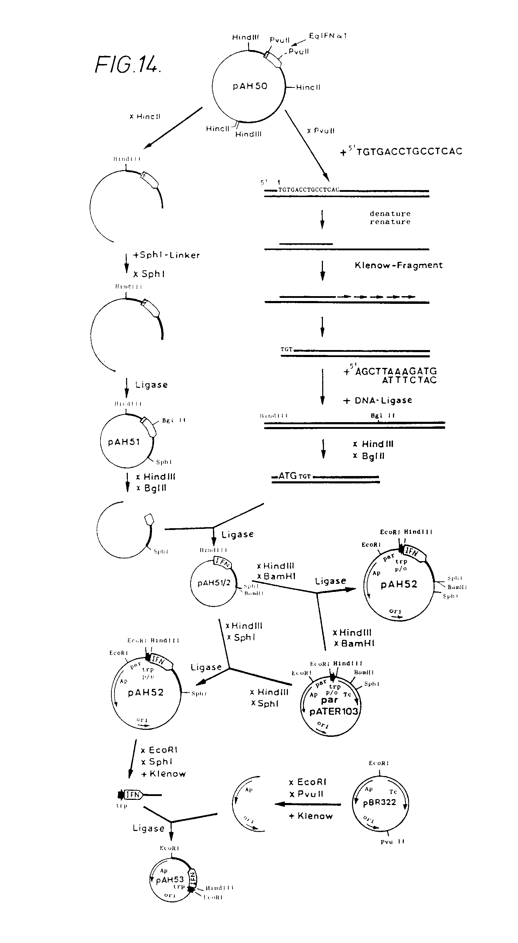

Die Herstellung der Expressionsplasmide pAH52, pAH52/2 und pAH53 sowie deren Vorstufen ist in Fig. 14 dargestellt. Zur Herstellung der Expressionsplasmide wurde das Plasmid pAH50 mit Hindll verdaut und das 4,2 kb lange DNA-Fragment, das das gesamte EqIFN-a1 Gen enthält, isoliert und gereinigt. Die Enden des Hindll-Fragmentes wurden mit Sphl-Linkern versehen. Anschließend wurde die DNA mit Sphl verdaut, mit Phenol und Chloroform extrahiert und mit Äthanol ausgefällt. Die DNA wurde mit Ligase zirkularisiert und E.coli HB101 transformiert. Ein Plasmid der gewünschten Struktur erhielt die Bezeichnung pAH51. Es enthält das EqIFN-a1-Gen mit einer verkürzten 3'-nichttranslatierten Region und einer zusätzlichen Sphl-Schnittstelle.

Um in der Endkonstruktion die DNA-Sequenz für das reife Pferde-a-Interferon in der richtigen Distanz mit der Promotersequenz zu verbinden, wurde von einem 0,4 kb langen DNA-Fragment ausgegangen, das aus mit Pvull geschnittenem Plasmid pAH50 isoliert wurde. Synthetisches 15mer Oligonukleotid mit der Sequenz 5'-TGTGACCTGCCTCAC wurde mit Polynukleotid-Kinase kinasiert. Es enthält die Sequenz, die für die ersten 5 Aminosäuren des reifen EqIFN-a1 aus Klon pAH50 codiert. Das 15mer wurde mit dem 0,4 kb Pvull-Fragment gemischt, der DNA-Doppelstrang denaturiert. Der an den Einzelstrang gebundene Oligonukleotid-Primer wurde mit dem Klenow-Fragment verlängert. Um einen eventuell verbliebenen 3'- Überhang sicher zu entfernen wurde die DNA anschließend mit T4 DNA-Polymerase inkubiert. Die entstandene DNA mit geraden Enden wurde mit Phenol und Chloroform extrahiert und ausgefällt. An dieses DNA-Stück wurde ein Gemisch zweier kinasierter, zueinander komplementärer Oligonukleotide: 12mer 5'-AGCTTAAAGATG, 8mer 5'-CATCTTTA (europäische Patentanmeldung No. 83 112 812.9) ligiert, das eine Hindlll-Schnittstelle und das Translationsstartcodon ATG erzeugt. Beide Oligonukleotide wurden mit Ligase an das DNA-Fragment ligiert. Nach Inaktivierung des Enzyms wurde die erhaltene DNA mit Hindlll und Bglll geschnitten und DNA-Fragmente von etwa 190 bp Länge isoliert und gereinigt. Das erhaltene DNA-Fragment wurde mit Hindlll und Bglll doppelt geschnittenem pAH51-Vektor ligiert und E.coli HB101 transformiert. Von 65 erhaltenen Kolonien wurde aus 4 Plasmiden, die das gewünschte Restriktionsmuster aufwiesen, ein Hindlll/BamHl DNA-Fragment isoliert und mit der Sanger-Methode sequenziert, wobei zwei Klone genau die gewünschte Sequenz enthielten. Ein solches Plasmid wurde pAH51/2 benannt. Dieses enthält die Sequenz für reifes EqIFN-a1 mit vorangestelltem Translationsstartcodon ATG und Hindlll-Schnittstelle.

Zur Herstellung der Expressionsplasmide pAH52 und pAH52/2 wurde das Plasmid pAH51/2 mit Sphl und Hindlll zweifach geschnitten, das entstehende 1,0 kb lange DNA-Fragment von einem Agarosegel isoliert und mit Hindlll und Sphl doppelt geschnittenem Plasmid parpATER103 ligiert. Ein nach Transformation von E.coli HB101 erhaltenes Plasmid der gewünschten Struktur wurde pAH52 benannt. Es enthält alle für eine induzierbare Expression von reifem EqIFN-a1 notwendigen Informationen. In analoger Weise wurde aus mit Hindlll und BamHl doppelt geschnittenem pAH51/2 und mit Hindlll/BamHl geschnittenem parpATER103 das Plasmid pAH52/2 hergestellt. Dieses Expressionsplasmid ist etwa 0,2 kb größer als pAH52 und weist zusätzlich eine singuläre BamHl-Schnittstelle auf.

Ein wesentlich verkleinertes Expressionsplasmid zur Herstellung von reifem EqIFN-a1 in E.coli, in dem Tryptophan-Promoter, Interferongen, Ampicillin-Resistenzgen und Replikationsursprung in einer Richtung orientiert sind, wurde aus den Plasmiden pAH52 und pBR322 hergestellt: pAH53. pAH52 wurde mit Sphl und EcoRI geschnitten, die Enzyme bei 70 ° C inaktiviert und die DNA-Enden nach Zusatz von dATP, dGTP, dCTP, dTTP mit Klenow-Fragment stumpf gemacht. Die DNA-Fragmente wurden auf einem Agarosegel nach der Größe fraktioniert und ein 1,1 kb langes Stück isoliert, das Promoter und Interferongen enthält. pBR322 wurde mit EcoRI und Pvull doppelt verdaut, die Enden wie oben beschrieben mit Klenow-Fragment begradigt und anschließend mit Kalbsdarmphosphatase dephosphoryliert. Ein DNA-Fragment von 2,4 kb Länge wurde von einem Agarosegel isoliert. Die beiden so erhaltenen DNA-Stücke wurden mit T4 DNA-Ligase verknüpft und E.coli HB101 transformiert. Ein so erhaltenes Plasmid, in dem Zwei EcoRI-Erkennungsstellen geschaffen wurden, erhielt die Bezeichnung pAH53.

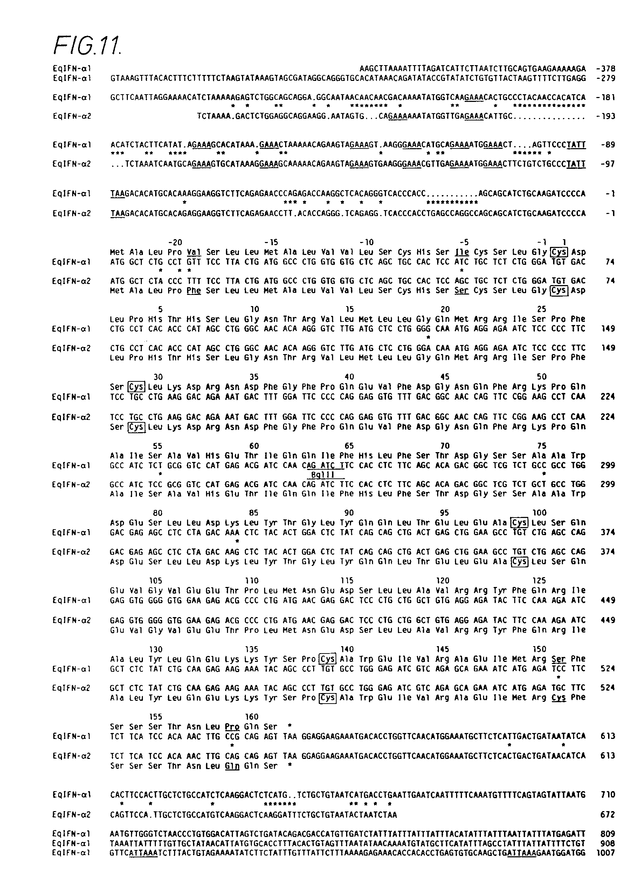

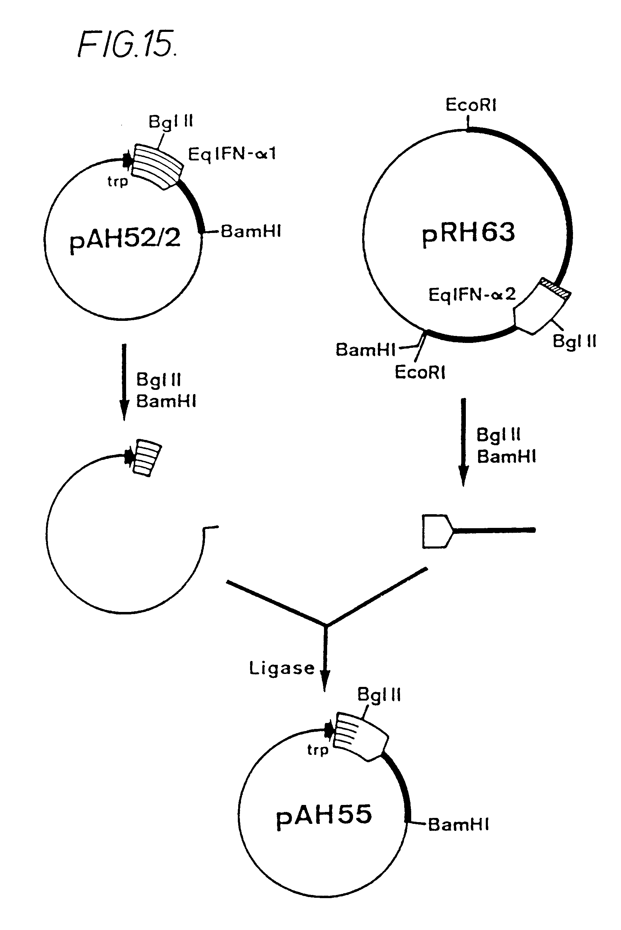

Aufgrund der hohen Homologie der Gene für EqIFN-a1 (pAH50) und Eq IFN-a2 (pRH63, Fig. 11) ist es möglich, aus dem Expressionsplasmid pAH52/2 und dem Lambda-Subklon pRH63 ein Expressionsplasmid für EqIFN-a2 herzustellen (Fig. 15). Da bis zur gemeinsamen Bglll-Stelle im für reifes Interferon codierenden Bereich nur zwei Basenunterschiede bestehen, die jedoch aufgrund des degenerierten Aminosäurecodes keinen Aminosäureunterschied bewirken, kann der erste Teil des Gens für EqIFN-a1 im Expressions- plasmid pAH52/2 auch für die Expression von EqIFN-a2 verwendet werden. pRH63 wurde mit Bglll und BamHl zweifach geschnitten und das entstehende DNA-Fragment von 1,0 kb Länge, das die codierende Sequenz für EqIFN-a2 ab der 64.Aminosäure enthält, von einem Agarosegel isoliert. pAH52/2 wurde ebenfalls mit Bglll und BamHl geschnitten, die Enden mit Kalbsdarmphosphatase dephosphoryliert und das größere der zwei entstehenden DNA-Fragmente von einem Agarosegel gewonnen. Dieses DNA-Stück enthält den Plasmidvektoranteil, den Promoter und die codierende Sequenz für die ersten 63 Aminosäuren des reifen Interferons. Die beiden beschriebenen DNA-Fragmente wurden mit Ligase verknüpft und E.coli HB101 transformiert. Ein so erhaltenes Plasmid, welches das Insert in der korrekten Orientierung enthält (mit BamHl und Bglll schneidbar!) wurde pAH55 benannt. Dieses Plasmid ermöglicht die Expression von reifem EqIFN-a2 in E.coli.

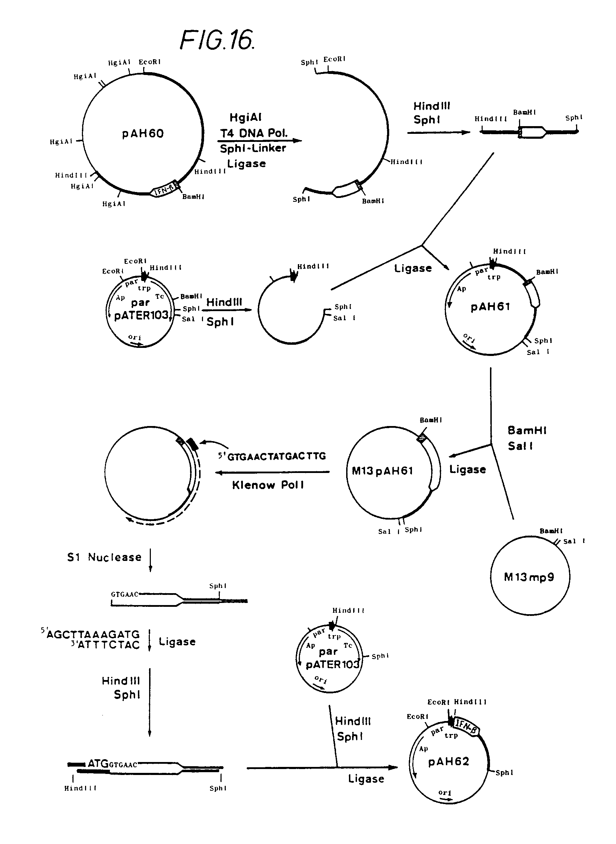

Für die Herstellung eines Expressionsplasmides für EqIFN-ß wurde zunächst das Pferde-DNA-Insert aus Plasmid pAH60 am 3'-Ende verkürzt und dieses in weiterer Folge so manipuliert, daß ein reifes EqIFN-β Protein von einem bakteriellen Promoter exprimiert wird. Die Vorgangsweise ist in Fig. 16 schematisch dargestellt. pAH60 wurde mit HgiAI geschnitten. Nach Inaktivierung des Enzyms wurden die 3'überhängenden DNA-Enden mit T4 DNA-Polymerase (Zugabe von dATP, dGTP, dCTP, dTTP) begradigt. An die stumpfen Enden wurden Sphl-Linker ligiert und die erhaltene DNA mit Sphl und Hindlll geschnitten. Ein entstandenes DNA-Fragment von 1,85 kb Länge wurde von einem Agarosegel isoliert und mit Hindlll und Sphl doppelt geschnittenem Plasmid parpATER103 ligiert. Ein nach Transformation von E.coli HB 101 erhaltener Klon mit dem gewünschten Plasmid wurde pAH61 benannt. Dieses Plasmid stellt eine Zwischenstufe für die weitere Konstruktion des Expressionsplasmides dar. pAH61 wurde mit BamHl und Sall zweifach geschnitten und ein entstandenes 1,3 kb langes DNA-Fragment von einem Agarosegel isoliert, gereinigt und mit BamHl/Sall doppelt verdauter M13mp9 Phagen-DNA ligiert. Nach Transformation von E.coli JM101 konnte von einem rekombinanten M13-Phagen (M23pAH61) einzelsträngige Phagen-DNA gewonnen werden. Diese Einzelstrang-DNA wurde mit kinasiertem 15mer Oligonukleotid 5'GTGAACTATGACTTG gemischt, auf 95 ° C erhitzt und langsam auf Raumtemperatur abgekühlt. Das Oligonukleotid bindet genau ab der ersten Base der Sequenz des reifen ß-Interferons. Die Zweitstrangsynthese an der Einzelstrangvorlage, ausgehend vom 15mer-Primer wurde nach Zugabe von dATP, dGTP, dCTP, dTTP und Klenow-Fragment durchgeführt. Die DNA wurde extrahiert und ausgefällt. Verbliebene, einzelsträngige DNA-Abschnitte wurden mit S1-Nuklease verdaut. An die durch diese Behandlung stumpfendig gemachte DNA wurde das Gemisch der 12mer und 8mer Oligonukleotide 5'-AGCTTAAAGATG und 5'-CATCTTTA anligiert und die entstandene DNA mit Hindlll und Sphl geschnitten. Ein DNA-Fragment der gewünschten Länge von 1,1 kb wurde von einem Agarosegel isoliert und mit Hindlll/Sphl doppelt geschnittenem Plasmid parpATER103 ligiert. Nach Transformation von E.coli HB101 wurden 54 Kolonien erhalten. Von 9 daraus isolierten Plasmid-DNAs wurde ein 1,3 kb langes EcoRl/Sall Fragment isoliert und mit der Sanger-Methode sequenziert. Ein daraus ermitteltes Plasmid mit der gewünschten Sequenz wurde pAH62 benannt. Dieses Plasmid ermöglicht die effiziente Expression von reifem EqIFN-ß Protein in E.coli. Ein Plasmid das eine Deletion der ersten Base (G) des reifen β-IFN Genes trägt, wurde pAH62deltaGl benannt. Dieses Plasmid erlaubt die Expression eines am Amino-Terminus verkürzten β-IFN durch Translationsstart am nächstfolgenden ATG (entspricht Aminosäure 19 des reifen ß-IFN), das überraschenderweise antivirale Aktivität, wenn auch deutlich weniger, verglichen mit dem unverkürzten Protein, aufweist.

Zum Nachweis der Expression der Interferonaktivität durch E.coli HB101, die das Plasmid pAH52, pAH52/2, pAH53, pAH55 oder pAH62 enthalten, wurden die Bakterien nach der Inkubation in einem geeigneten Kulturmedium aufgebrochen und der Überstand nach Sterilfiltration auf Interferonaktivität in einem Assay, der den cytopathischen Effekt (CPE) von VSV oder EMCV mißt, getestet. Verwendet wurden dazu NBL-6 Zellen (ATCC CCL 57, Pferdehaut-Epidermis-Zellen), die mit Vesicular-Stomatitis-Virus (VSV) infiziert worden waren und/oder A549 (ATCC CCL185, humane Lungencarcinom-Zellinie) die mit Encephalomyocarditis Virus (EMCV) infiziert worden waren. Die Ergebnisse sind im Beispiel 0 aufgelistet.

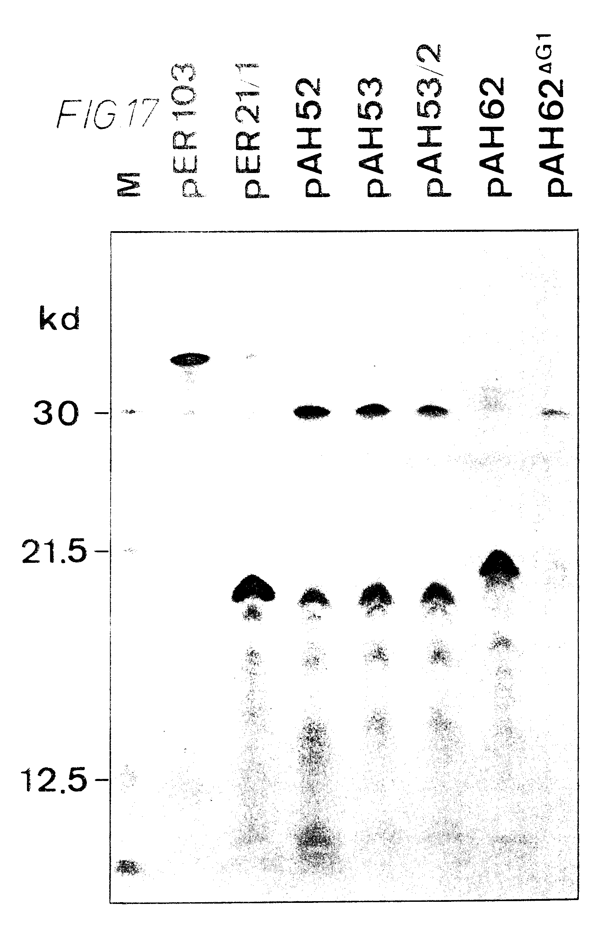

Der Nachweis der exprimierten Pferde-Interferone wurde durch Markierung der Proteine in Maxizellen erbracht. Plasmidcodierte Proteine können durch Verwendung der Maxizelltechnik (37) selektiv in vivo markiert werden. Der E.coli Stamm CSR603 (CGSC 5830) (F-, thr-1, leuB6, proA2, phr-1, recA1, argE3, thi-1, uvrA6, ara-14, lacY1, galK2, xyl-5, mtl-1, gyrA98 (nalA98), rpsL31, tsx-33, λ-, supE44,) besitzt keine Mechanismen für die Reparatur von durch UV-Bestrahlung entstandene Schäden der DNA. Durch Bestrahlung mit einer geeigneten UV-Strahlendosis wird das bakterielle Chromosom zerstört, einige der wesentlich kleineren und in mehreren Kopien pro Zelle vorhandenen Plasmid-DNAs bleiben dagegen funktionell. Nach Abtöten aller nicht geschädigten, sich vermehrenden Zellen durch das Antibiotikum D-Cycloserin und Aufbrauchen der endogenen mRNA werden in den verbleibenden Zellen nur noch am Plasmid codierte Gene transkribiert und translatiert und die gebildeten Proteine können durch Einbau von ³⁵S-Methionin radioaktiv markiert und nachgewiesen werden. E.coli CSR603 wurde nach üblichen Verfahren mit den Expressionsplasmiden transformiert und auf Ampicillinhaltigen Agarplatten auf transformierte Bakterien selektioniert. Die Präparation der Maxizellen und das Markieren der Proteine erfolgte nach einer Vorschrift von A. Sancar (37). In Fig. 17 ist das Autoradiogramm des getrockneten Geles dargestellt. Als Molekulargewichtsstandard wurde ein 14C-methyliertes Proteingemisch (Amersham) verwendet. Als Kontrollen wurden das Plasmid pER103, das nur den Promoter ohne Interferongen enthält und das Plasmid pER21/1 eingesetzt, welches zwei Kopien des humanen IFN-a2arg Genes enthält. Die Proteinbanden bei etwa 18 kd sind die von den Plasmiden exprimierten Interferone.

Zum Nachweis der Gesamtanzahl von Sequenzen im Pferdegenom, welche hohe Homologie mit Interferongenen der Klasse IFN-a, IFN-ß bzw. IFN-omega aufweisen, wurde hochmolekulare Pferde-DNA mit dem entsprechenden Restriktionsenzym vollständig verdaut und diese geschnittene DNA nach der Größe aufgetrennt. Nach Southern-Transfer auf Nitrozellulosefilter, denaturieren und fixieren der DNA wurde jedes Filter mit nicktranslatierter Probe hybridisiert. Als Probe für EqIFN-a wurde ein 1,0 kb langes Hindlll/Sphl-Fragment aus Plasmid pAH52, für EqIFN-ß ein 1,1 kb langes Hindlll/Sphl-Fragment aus Plasmid pAH62 verwendet, welches jeweils die codierende Sequenz für das gesamte reife Interferon enthält. Als Probe für EqlFN-omega wurde das 2,1 kb EcoRl-Insert aus Plasmid pRH61 eingesetzt. Die Filter wurden anschließend unter stringenten Bedingungen gewaschen, daß keine Kreuzhybridisierung zwischen diesen 3 Interferonsequenzen eintritt: Die Autoradiographie erfolgte auf DuPont Cronex X-ray Film unter Verwendung von Kodak Lanex-Regular Verstärkerfolie 7 Tage bei -80 C. Das Ergebnis ist in Figur 18 gezeigt. Es zeigte überraschenderweise, daß im Pferdegenom mindestens 7 Gene der IFN-a Klasse, mindestens 2 Gene der IFN-ß Klasse und mindestens 8 Gene der IFN-omega Klasse vorhanden sind.

Zur Herstellung des Expressionsplasmides pRH 100 wurde das Plasmid pER 103 (Eva Dworkin-Rastl et al., Gene 21 (1983) 237-248, EP-A-0.115-613) mit der Restriktionsendonuklease Hindlll linearisiert und die 5'terminalen Phosphatreste wurden entfernt.

Diese Plasmid-DNA wurde mit den phosphorylierten Oligonukleotiden d(AGCTTAAAGATGAGCT) und d(CATC TTTA gemischt und ligiert. Die Ligasereaktion wurde mit der Restriktionsendonuklease Sacl verdaut und durch Zugabe von T4-PNK ligiert. Die Oligonukleotide wurden analog der in der EP-A-0.115-613 beschriebenen Methode hergestellt.

Kompetenter E.coli Hb101 wurde mit dieser Ligasereaktion versetzt und inkubiert.

Von den entstandenen Bakterienkolonien wurden 12 willkürlich ausgesucht und daraus im Mikromaßstab die Plasmide isoliert (Birnboim und Doly, Nucl. Acids Res. 7 (1979) 1513-1523). Die resultierende DNA wurde mit der Restriktionsendonuklease Sacl geschnitten und die DNA auf einem Agarosegel (1 %, 1x TBE-Puffer) aufgetrennt. Die Wanderung der DNA als lineares Molekül der Größe von etwa 4.400 bp bestätigte die Einführung einer Sacl-Erkennungsstelle in das Plasmid. Eines dieser Plasmide wurde willkürlich ausgesucht. Mit der DNA aus der dazugehörigen Minipräparation wurde nochmals E.coli HB101 transformiert. Von den resultierenden transformierten Bakterien wurde eine Kolonie ausgewählt und in größerem Maßstab angezüchtet. Das daraus isolierte Plasmid wurde mit den Restriktionsendonukleasen EcoRI und BamHl geschnitten, die DNA auf einem 1%-igen Agarosegel aufgetrennt, und das kleinere Fragment aus dem Gel durch Elektroelution isoliert. Dieses etwa 460 bp lange EcoRl-BamHl DNA-Fragment wurde nach Sanger sequenziert. (F. Sanger et al., Proc.Natl.Acad.Sci. (1977) 5463-5467). Das dermaßen analysierte Plasmid wurde pRH 100 genannt.

Transformation der Zellen mit den Vehikeln kann durch eine Vielzahl an Verfahren erreicht werden. Beispielsweise kann sie durch Kalzium erfolgen, wobei entweder die Zellen in Magnesium gewaschen und die DNA den in Kalzium suspendierten Zellen zugegeben oder die Zellen einem Kopräzipitat von DNA und Kalziumphosphat ausgesetzt werden. Bei nachfolgender Genexpression werden die Zellen auf Medien übertragen, die für transformierte Zellen selektieren.

Nach erfolgter Transformation des Wirtes, Expression des Gens und Fermentation oder Zellkultivierung unter Bedingungen, bei denen die erfindungsgemäßen Proteine exprimiert wird, kann das Produkt üblicherweise durch bekannte chromatographische Trennmethoden extrahiert werden, um so ein Material zu erhalten, das die Proteine mit oder ohne Leader und Tailing-Sequenzen enthält. Die erfindungsgemäßen Interferone können mit einer Leader-Sequenz am N-Terminus exprimiert werden (Pre-IFN), die von einigen Wirtszellen entfernt werden kann. Wenn nicht, so ist eine Abspaltung des Leader-Polypeptids (wenn vorhanden) erforderlich, um reifes IFN zu erhalten. Alternativ kann der IFN Klon so modifiziert werden, daß das reife Protein im Mikroorganismus direkt statt des Pre-IFN produziert wird. Für diesen Fall kann die Precursor-Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die DNA-Sequenz für funktionelles oder reifes IFN kann mit MF-alpha-1 an der vermuteten Kathepsinähnlichen Schnittstelle (nach Lys-Arg) an Position 256 vom Initiationscodon ATG aus (Kurjan, Herskowitz, Cell 30, 933-943 (1982)) verbunden sein.

Basierend auf ihrem biologischen Wirkungsspektrum sind die neuen, erfindungsgemäßen Interferone verwendbar für jede Art der Behandlung für die auch die bekannten Interferone eingesetzt werden. Diese beinhalten beispielsweise Herpes, Rhinovirus, Equine- Abortion-Virus, verschiedene Krebsarten und ähnliches. Die neuen Interferone können alleine oder in Kombination mit anderen bekannten Interferonen oder biologisch aktiven Produkten eingesetzt werden, beispiels- weise mit IFN-alpha, IL-2, anderen Immun-Modulatoren und ähnlichen.

Die erfindungsgemäßen Interferone können parenteral verabreicht werden in Fällen, in denen Antitumor oder antivirale Behandlung erforderlich ist und in Fällen, in denen sich immunosuppressive Eigenschaften zeigen. Dosierung und Dosierungsrate können ähnlich denen sein, die zur Zeit bei klinischen Untersuchungen für IFN-a-Materialien gelten z.B. ca. (1-10) x 10⁶ Einheiten täglich und bei Präparaten, die zu mehr als 1 % rein sind, bis zu beispielsweise 5 x 107 Einheiten täglich.Beispielsweise können für eine zweckmäßige Dosierungsform bei einem im wesentlichen homogenen, bakteriell produzierten erfindungsgemäßen IFN, bei parenteraler Verwendung 3 mg IFN-omega in 25 ml 5%igem tierischem Serum Albumin vorzugsweise Pferde Serum Albumin gelöst werden. Diese Lösung wird dann durch ein bakteriologisches Filter gegeben und die gefilterte Lösung aseptisch auf 100 Fläschchen verteilt, von dem jedes 6 x 10⁶ Einheiten reines IFN zur parenteralen Applikation geeignet, enthält. Die Gläschen werden vor Verwendung vorzugsweise in der Kälte (-20*C) aufbewahrt. Die erfindungsgemäßen Substanzen können in bekannter Weise formuliert werden, um pharmazeutisch verwendbare Mittel zu erhalten, wobei das erfindungsgemäße Polypeptid mit einer pharmazeutischen, akzeptablen Trägersubstanz vermischt wird. Gebräuchliche Trägerstoffe und ihre Formulierung sind bei E.W. Martin in Remingtom's Pharmaceutical Sciences beschrieben, worauf ausdrücklich Bezug genommen wird. Die erfindungsgemäßen Interferone werden mit einer angemessenen Menge des Trägerstoffes vermischt, um geeignete pharmazeutische Mittel zu schaffen, die für eine effektive Anwendung beim Empfänger (Patienten) geeignet sind. Bevorzugt wird eine parenterale Applikation vorgenommen.

Mit Hilfe der vorliegenden Erfindung ist es also erstmals möglich, Pferde Interferone und die sie kodierenden Gensequenzen zu erhalten.

### Gegenstand der Erfindung sind im Einzelnen

### Proteine:

Pferde-alpha-Interferone, im wesentlichen frei von anderen Proteinen tierischer Herkunft
- in im wesentlichen reiner Form.
- frei von nativer Glykosylierung.
- ein Leader-Peptid enthaltend.
- eine Aminosäuresequenz nach Formel I, 11, V oder VI oder biologisch aktive Varianten dieser Sequenzen enthaltend.
- nach dem erfindungsgemäßen Verfahren herstellbar.

Pferde-omega-Interferone, im wesentlichen frei von anderen Proteinen tierischer Herkunft.
- in im wesentlichen reiner Form.
- frei von nativer Glykosylierung.
- ein Leader-Peptid enthaltend.
- eine Aminosäuresequenz nach Formel IV oder VII oder biologisch aktive Varianten dieser Sequenzen enthaltend.
- nach dem erfindungsgemäßen Verfahren herstellbar.

Pferde-beta-Interferone, im wesentlichen frei von anderen Proteinen tierischer Herkunft.
- in im wesentlichen reiner Form.
- frei von nativer Glykosylierung.
- ein Leader-Peptid enthaltend.
- eine Aminosäuresequenz nach Formel 111 oder biologisch aktive Varianten dieser Sequenzen enthaltend.
- nach dem erfindungsgemäßen Verfahren herstellbar.

### DNA-Sequenzen:

- für EqlFN-alpha kodierende Sequenzen.
- für EqlFN-alpha kodierende Sequenzen oder degenerierte Variationen dieser Sequenzen, die in die Hindlll bzw.EcoRl Schnittstelle des Plasmides pUC9 oder pUC8 eingefügt sind.
- das Plasmid pAH50.
- das Plasmid pRH63.
- das Plasmid pRH82.
- das Plasmid pRH83.
- für EqlFN-alpha kodierende Sequenzen oder degenerierte Variationen dieser Sequenzen, die unter stringenten Bedingungen, die eine Homologie größer als 85% vorzugsweise größer als 95% erkennen lassen, mit den Inserts der Plasmide pAH50, pRH63, pRH82 oder pRH83 hybridisieren.
- für EqlFN-alpha kodierende Sequenzen oder degenerierte Variationen dieser Sequenzen, die in einem Expressionsvektor enthalten sind, der in Mikroorganismen, vorzugsweise in Prokaryoten oder Eukaryoten und in Säugetierzellen replizierbar ist.
- die DNA-Sequenz nach Formel I, II, V oder VI oder degenerierte Variationen dieser Sequenzen.
- für EqlFN-omega kodierende Sequenzen.
- für EqlFN-omega kodieronde Sequenzen oder degenerierte Variationen dieser Sequenzen, die in die EcoRI Schnittstelle des Plasmides pUC9 oder pUC8 eingefügt sind.
- das Plasmid pRH61.
- das Plasmid pRH62.
- für EqlFN-omega kodierende Sequenzen oder degenerierte Variationen dieser Sequenzen, die unter stringenten Bedingungen, die eine Homologie größer als 85% vorzugsweise größer als 95% erkennen lassen, mit den Inserts der Plasmide pRH61 oder pRH62 hybridisieren.
- für EqlFN-omega kodierende Sequenzen oder degenerierte Variationen dieser Sequenzen, die in einem Expressionsvektor enthalten sind, der in Mikroorganismen, vorzugsweise in Prokaryoten oder Eukaryoten und in Säugetierzellen replizierbar ist.
- die DNA-Sequenz nach Formel IV oder VII oder degenerierte Variationen dieser Sequenzen.
- für EqlFN-beta kodierende Sequenzen.
- für EqlFN-beta kodierende Sequenzen oder degenerierte Variationen dieser Sequenzen, die in die Hindlll Schnittstelle des Plasmides pAH60 eingefügt ist.
- das Plasmid pAH60.
- für EqlFN-beta kodierende Sequenzen oder degenerierte Variationen dieser Sequenzen, die unter stringenten Bedingungen, die eine Homologie größer als 85% vorzugsweise größer als 95% erkennen lassen, mit den Inserts des Plasmides pAH60 hybridisieren.
- für EqlFN-beta kodierende Sequenzen oder degenerierte Variationen dieser Sequenzen, die in einem Expressionsvektor enthalten sind, der in Mikroorganismen, vorzugsweise in Prokaryoten oder Eukaryoten und in Säugetierzellen replizierbar ist.
- die DNA-Sequenz nach Formel III oder degenerierte Variationen dieser Sequenz.

### transformierte Wirtsorganismen:

- die die für EqIFN-alpha, -omega oder -beta kodierenden genetischen Informationen enthalten, vorzugsweise Prokaryoten, Eukaryoten oder Säugetierzellen, insbesondere E.coli oder E.coli JM101.
- die die genetischen Sequenzen für die erfindungsgemäßen Proteine in einem in den Wirtsorganismen replizierbaren Vektor enthalten.

### Plasmide:

- Plasmid pAH51, dadurch gekennzeichnet, daß ein 4,2 kb langes DNA-Fragment des mit Hindll geschnittenen Plasmides pAH50 mit Sphl-Linkern versehen und nach Schneiden mit Sphl zirkularisiert ist.
- Plasmid pAH51/2, dadurch gekennzeichnet, daß es in der Hindlll/Bglll-Schnittstelle des Plasmides pAH51 anstelle des plasmideigenen, längeren Fragmentes ein Fragment enthält, das aus dem 0,4 kb langen Pvull Fragment des Plasmides pAH50 erhalten wurde, nachdem in Gegenwart des 15-mer Oligonukleotid-Primers 5'TGTGACCTGCCTCAC denaturiert, mit Klenow-Fragment verlängert, der Oligonukleotidkomplex 
5´AGCTTAAAGATG
3´ATTTCTAC
 anligiert und mit Hindlll und Bglll geschnitten worden war.
- Expressionsplasmid parpATER103, dadurch gekennzeichnet,daß in die EcoRl/Hindlll-Schnittstelle des Plasmides pAT153 ein 0,47 kb langes DNA-Fragment des mit EcoRI partiell und mit Hindlll vollständig geschnittenen Plasmides parpER33 eingefügt ist.
- Expressionsplasmid pAH52/2, dadurch gekennzeichnet, daß in die Hindlll/BamHI-Schnittstelle des Plasmides parpATER103 anstelle des plasmideigenen, kürzeren Fragmentes das 1,0 kb lange Hindlll/BamHI-Fragment des Plasmides pAH51/2 eingefügt ist.
- Expressionsplasmid pAH52, dadurch gekennzeichnet, daß in die Hindlll/Sphl-Schnittstelle des Plasmides parpATER103 anstelle des plasmideigenen, kürzeren Fragmentes das Hindlll/Sphl-Fragment des Plasmides pAH51/2 eingefügt ist.
- Expressionsplasmid pAH53, dadurch gekennzeichnet, daß das mit Klenow-Fragment begradigte und dephosphorylierte 2,4 kb lange EcoRl/Pvull-Fragment aus pBR322 mit einem, mit Klenow-Fragment begradigtem, 1,1 kb langem EcoRI/SphI-Fragment des Plasmides pAH52 verknüpft ist.
- Expressionsplasmid pAH55, dadurch gekennzeichnet, daß das Plasmid pAH52/2 anstelle des plasmideigenen, kürzeren Bglll/BamHI-Fragmentes das 1,0 kb lange Bglll/BamHI-Fragment des Plasmides pRH63 aufweist.
- Plasmid pAH61, dadurch gekennzeichnet, daß in die Hindlll/Sphl-Schnittstelle des Plasmides parpATER103 anstelle des plasmideigenen, kürzeren Fragmentes ein 1,85 kb langes DNA-Fragment des mit HgiAI geschnittenen Plasmides pAH60, das mit T4-DNA Polymerase begradigt, mit Sphl-Linkern versehen und anschließend mit Hindlll und Sphl geschnitten wurde, eingefügt ist.
- M13pAH61, dadurch gekennzeichnet, daß das 1,3 kb lange BamHI/Sall-Fragment des Plasmides pAH61 mit BamHl/Sall doppelt verdauter M13mp9-Phagen-DNA verknüpft ist.
- Expressionsplasmid pAH62, dadurch gekennzeichnet, daß ein mit Hilfe des 15-mer
   5'GTGAACTATGACTTG

   doppelsträngig gemachtes, mit S1 Nuklease behandeltes, mit dem Oligonukleotidkomplex 
5´AGCTTAAAGATG
    3´ATTTCTAC
 ligiertes und mit Hindlll und Sphl geschnittenes Fragment des M13pAH61 in die Hindlll/Sphl-Schnittstelle des Plasmides parpATER103 anstelle des plasmideigenen, kürzeren Fragmentes eingefügt ist.
- Expressionsplasmid pRH100, dadurch gekennzeichnet, daß in die Hindlll-Schnittstelle des Plasmides pER103 der Oligonukleotidkomplex 
5´AGCTTAAAGATGAGCTCATCTTTA
3´ATTTCTACTCGAGTAGAAATTCGA
 eingefügt ist.

Außerdem werden Verfahren zur Herstellung dieser Plasmide beschrieben:
- Verfahren zur Herstellung des Plasmides pAH51, dadurch gekennzeichnet, daß das Plasmid pAH50 mittels Hindll geschnitten, das 4,2 kb lange Fragment mit Sphl-Linkern versehen wird, anschließend mit Sphl geschnitten und mit DNA-Ligase zirkularisiert wird und das so erhaltene Plasmid zur Replikation in E.coli HB 101 transformiert und kultiviert wird.
- Verfahren zur Herstellung des Plasmides pAH51/2, dadurch gekennzeichnet, daß das Plasmid pAH50 mit Pvull geschnitten wird, das 0,4 kb lange Fragment in Gegenwart des 15-mer Oligonukleotid-Primers 5'TGTGACCTGCCTCAC denaturiert, der an den Einzelstrang gebundene Primer mit Klenow-Fragment verlängert, ein möglicher 3'-Uberhang entfernt,der Oligonukleotidkomplex 
5´AGCTTAAAGATG
3´ATTTCTAC
 anligiert wird, das erhaltene DNA-Fragment nach Restriktionsendonukleaseverdauung mit Hindlll und Bglll in die Hindlll/Bglll-Schnittstelle des Plasmides pAH51 anstelle des plasmideigenen, längeren Fragmentes eingefügt wird und das so erhaltene Plasmid zur Replikation in E.coli HB 101 transformiert und kultiviert wird.
- Verfahren zur Herstellung des Plasmides parpATER103, dadurch gekennzeichnet, daß in das durch Restriktionsendonukleaseverdauung mit EcoRI und Hindlll linearisierte Plasmid pAT153 ein 0,47 kb langes Fragment des mit Hindlll vollständig, mit EcoRI partiell geschnittenen Plasmides parpER33 mittels Ligasereaktion eingefügt wird und das so erhaltene Plasmid zur Replikation in E.coli HB 101 transformiert und kultiviert wird.
- Verfahren zur Herstellung des Expressionsplasmides pAH52/2, dadurch gekennzeichnet, daß in die Hindlll/BamHI-Schnittstelle des Plasmides parpATER103 anstelle des plasmideigenen, kürzeren Fragmentes das 1,0 kb lange Hindlll/BamHI-Fragment des Plasmides pAH51/2 eingefügt wird und das so erhaltene Plasmid zur Replikation in E.coli HB 101 transformiert und kultiviert wird.
- Verfahren zur Herstellung des Expressionsplasmides pAH52, dadurch gekennzeichnet, daß in die Hindlll/Sphl-Schnittstelle des Plasmides parpATER103 anstelle des plasmideigenen, kürzeren Fragmentes das Hindlll/Sphl-Fragment des Plasmides pAH51/2 eingefügt wird und das so erhaltene Plasmid zur Replikation in E.coli HB 101 transformiert und kultiviert wird.
- Verfahren zur Herstellung des Expressionsplasmides pAH53, dadurch gekennzeichnet, daß das mit Klenow-Fragment begradigte und dephosphorylierte 2,4 kb lange EcoRl/Pvull-Fragment aus pBR322 mit einem, mit Klenow-Fragment begradigtem 1,1 kb langem EcoRl/Sphl-Fragment des Plasmides pAH52 verknüpft wird und das so erhaltene Plasmid zur Replikation in E.coli HB 101 transformiert und kultiviert wird.
- Verfahren zur Herstellung des Expressionsplasmides pAH55, dadurch gekennzeichnet, daß in das Plasmid pAH52/2 anstelle des plasmideigenen, kürzeren Bglll/BamHI-Fragmentes das 1,0 kb lange Bglll/BamHI-Fragment des Plasmides pRH63 eingefügt wird und das so erhaltene Plasmid zur Replikation in E.coli HB 101 transformiert und kultiviert wird.
- Verfahren zur Herstellung des Plasmides pAH61, dadurch gekennzeichnet, daß in die Hindlll/Sphl-Schnittstelle des Plasmides parpATER103 anstelle des plasmideigenen, kürzeren Fragmentes ein 1,85 kb langes DNA-Fragment des mit HgiAI geschnittenen Plasmides pAH60, das mit T4-DNA Polymerase begradigt, mit Sphl-Linkern versehen und anschließend mit Hindlll und Sphl geschnitten wird, eingefügt wird und das so erhaltene Plasmid zur Replikation in E.coli HB 101 transformiert und kultiviert wird.
- Verfahren zur Herstellung des M13pAH61, dadurch gekennzeichnet, daß das 1,3 kb lange BamHl/Sall-Fragment des Plasmides pAH61 mit BamHl/Sall doppelt verdauter M13mp9-Phagen-DNA verknüpft wird und zur Replikation in E.coli JM 101 transformiert und kultiviert wird.
- Verfahren zur Herstellung des Expressionsplasmides pAH62, dadurch gekennzeichnet, daß ein mit Hilfe des 15-mer 5'GTGAACTATGACTTG doppelsträngig gemachtes, mit S1 Nuklease behandeltes, mit dem Oligonukleotidkomplex 
5´AGCTTAAAGATG
    3´ATTTCTAC
 ligiertes und mit Hindlll und Sphl geschnittenes Fragment des M13pAH61 in die Hindlll/Sphl-Schnittstelle des Plasmides parpATER103 anstelle des plasmideigenen, kürzeren Fragmentes eingefügt wird und das so erhaltene Plasmid zur Replikation in E.coli HB 101 transformiert und kultiviert wird.

Gegenstand der Erfindung sind ebenfalls Verfahren zur Herstellung der erfindungsgemäßen Proteine:
- Verfahren zur Herstellung von EqIFN-alpha, -beta, -omega, dadurch gekennzeichnet, daß
   a) ein Wirtsorganismus, vorzugsweise ein Prokaryot, Eukaryot oder eine Säugetierzelle, insbesondere E.coli oder Saccharomyces cerevisiae mit genetischen Informationen, kodierend für EqIFN-alpha, -beta, -omega, vorzugsweise mit den für die erfindungsgemäßen Proteine kodierenden Sequenzen aus den Plasmiden pAH50, pRH63, pRH82, pRH83, pRH61, pAH60, pRH62 oder den mit diesen Plasmiden unter stringenten Bedingungen, die eine Homologie größer als 85% vorzugsweise größer 90% erkennen lassen, hybridisierende Sequenzen, insbesondere Sequenzen gemäß einer der Formeln I bis VII oder degenerierten Variationen dieser Sequenzen transformiert,
   b) die kodierende Sequenz in einem Expressionsvektor, vorzugsweise in einem der Expressionsvektoren pAH52, pAH52/2, pAH53, pAH55 oder pAH62 enthalten ist und diese Information zur Produktion des EqIFN-alpha, -beta oder -omega im Wirtsorganismus exprimiert und
   c) das Interferon EqIFN-alpha, -beta oder -omega, vorzugsweise ein Interferon gemäß einer der Formeln I bis VII isoliert und gereinigt wird.

### Legende zu den Figuren

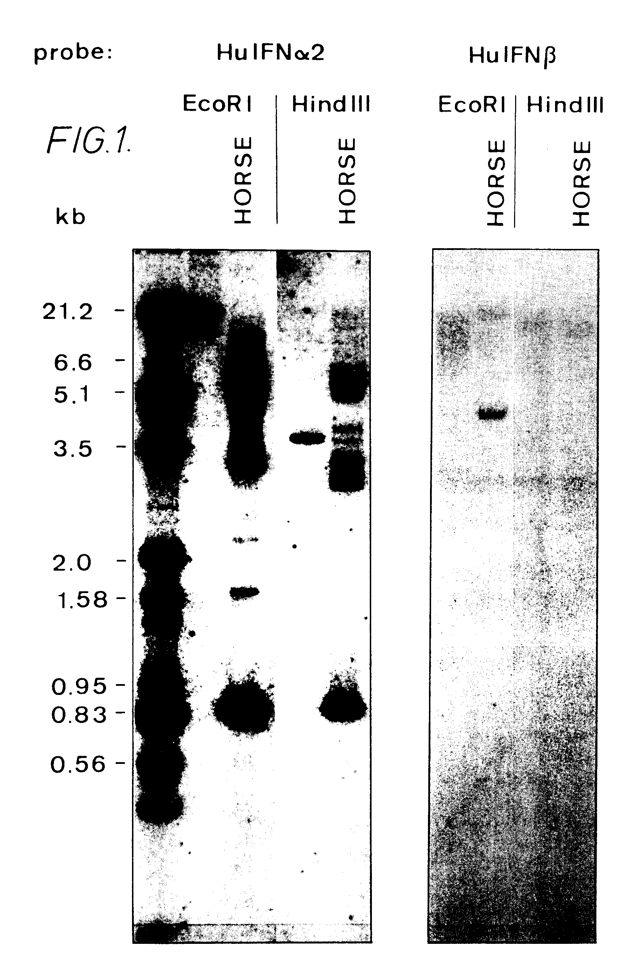

Fig. 1: Autoradiogramm zum Screening der Pferde-Genbibliothek mit human-Interferongenen.
Fig. 2: Restriktionskarte des Klons Eq-alphal sowie des Plasmidinserts von pAH50.
Fig. 3: Restriktionskarte des Klons Eq-beta6 sowie des Plasmidinserts von pAH60.
Fig. 4: Sequenz des Hindlll-Fragmentes von pAH50.
Fig. 5: Homologie zwischen EqIFN-a1 und verschiedenen a-Interferonen.
Fig. 6: Homologie zwischen EqIFN-ß, HuIFN-ß, BoIFN-ß und MuIFN-ß.
Fig. 7: Vergleich der Aminosäuresequenzen von EqIFN-a1 mit a-Interferonen verschiedener Spezies.

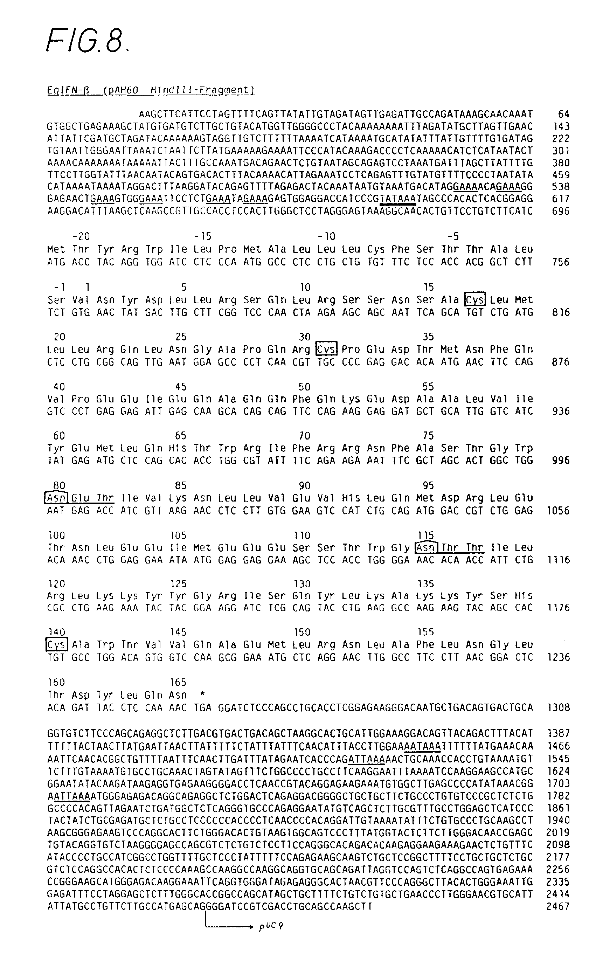

Fig. 8: Sequenz des Hindlll-Fragmentes von pAH60.
Fig. 9: Restriktionskarten der Inserts von pRH61 und pRH63.

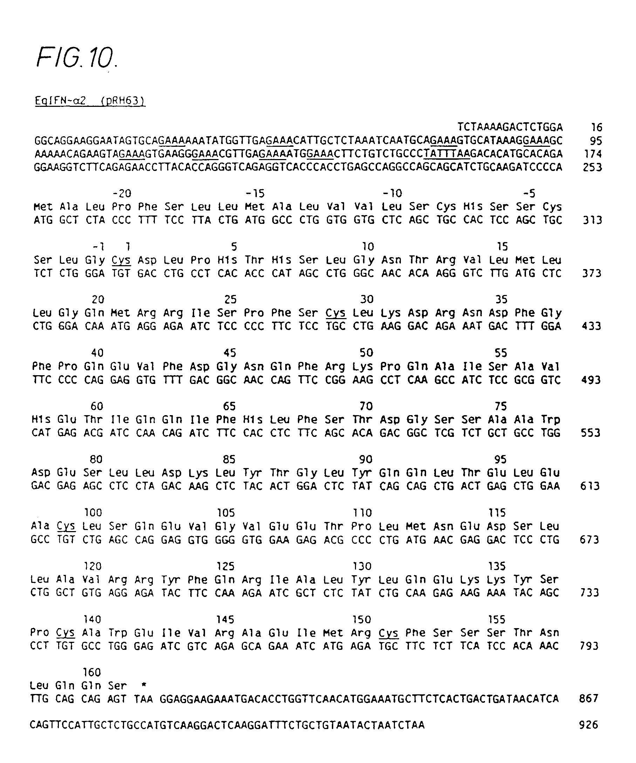

Fig. 10: Teilsequenz des Inserts von pRH63.
Fig. 11: Vergleich der Sequenzen von EqIFN-a1 und EqIFN-a2.

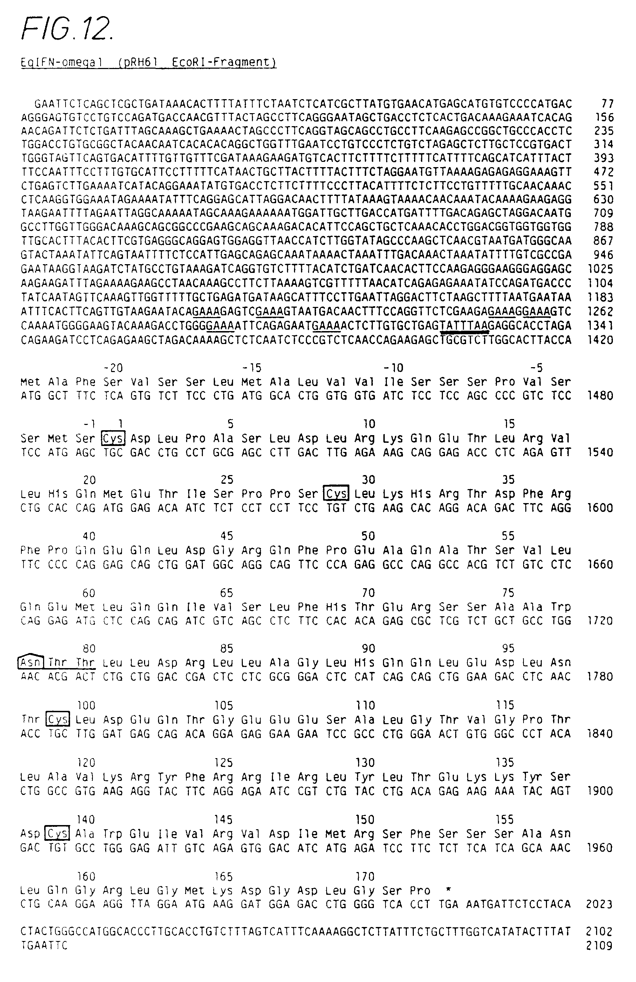

Fig. 12: Sequenz des EcoRI-Fragmentes von pRH61.
Fig. 13: Konstruktion des Plasmides parpATER103.
Fig. 14: Konstruktion der Plasmide pAH51, pAH51/2, pAH52, pAH52/2 und pAH53.
Fig. 15: Konstruktion des Plasmides pAH55.
Fig. 16: Konstruktion der Plasmide pAH61 und pAH62.
Fig. 17: Autoradiogramm der exprimierten Pferde-Interferone in Maxizellen.
Fig. 18: Autoradiogramm zum Nachweis der Gesamtanzahl von Sequenzen im Pferdegenom, die hohe Homologie mit Interferongenen der Klassen IFN-a, IFN-ß bzw. IFN-omega aufweisen.
Fig. 19: Vergleich der Aminosäuresequenzen von EqIFN-a1, EqIFN-a2, EqIFN-ß, EqIFN-_{"},1 und CaIFN-a1 mit Interferonen verschiedener Spezies.
Fig 20: Homologie zwischen EqIFN-a1, EqIFN-a2, EqIFN-ß, EqIFN-ω1 und verschiedenen Interferonen.

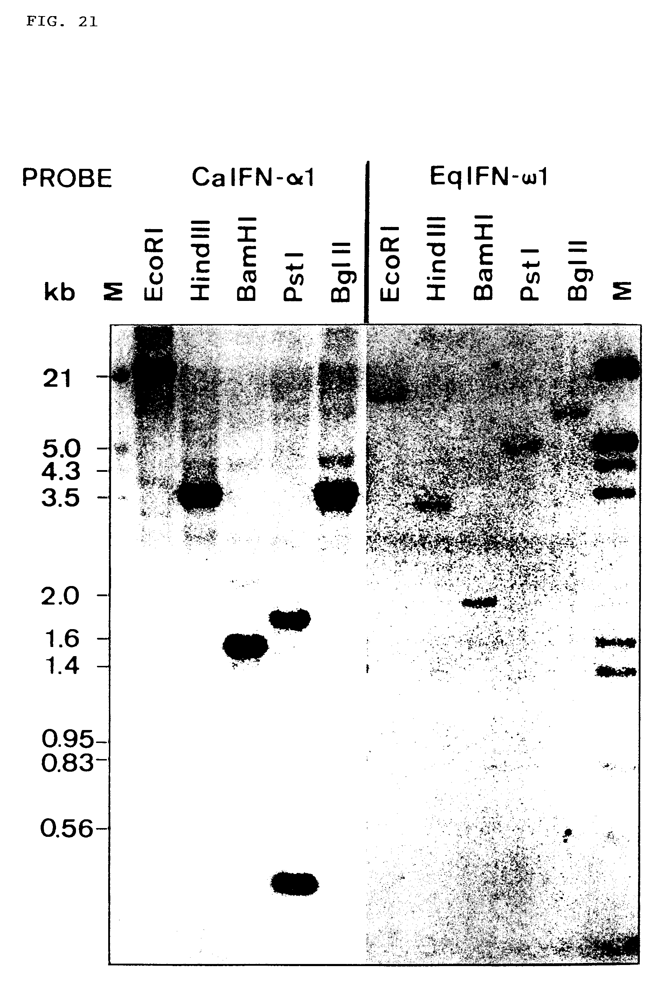

Fig. 21: Autoradiogramm zum Nachweis von mit CalFN-alphal und EqlFN-omega hybridisierenden Sequenzen in genomischer Hunde-DNA.
Fig. 22: Restriktionskarten der Plasmidinserts von pAH50, PAH60, pRH61, pRH62 und pRH63 sowie der Klone Eq-alphal , Eq-alpha16, Eq-alpha20 und Eq-beta6.
Fig. 23: Teilsequenz des Inserts von Plasmid pRH62.
Fig. 24: Vergleich der Sequenzen von EqIFN-ω1 und EqIFN-ω2.
Fig. 25: Homologie zwischen EqIFN-a1 und EqIFN-a2, EqIFN-ß, EqIFN-ω1, EqIFN-ω2 und CaIFN-a1 + mit verschiedenen Interferonen.
Fig. 26: Teilsequenz des Inserts von Plasmid pRH83.
Fig. 27: Teilsequenz des Inserts von Plasmid pRH82.

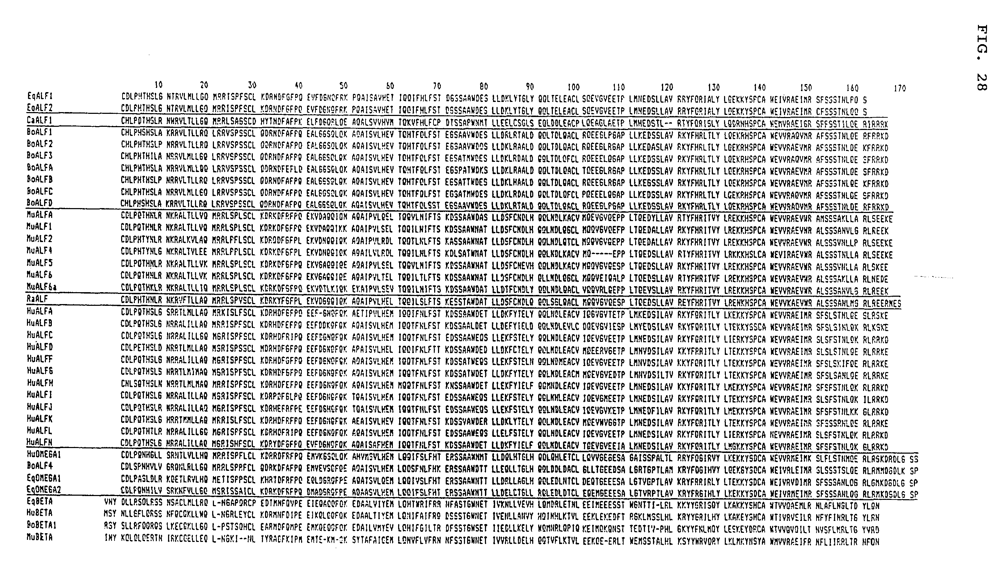

Fig. 28: Vergleich der Aminosäuresequenz von EqIFN-a1, EqIFNa2, EqIFN-ß, EqIFN-ω1, EqIFN-ω2 mit Interferonen verschiedener Spezies.

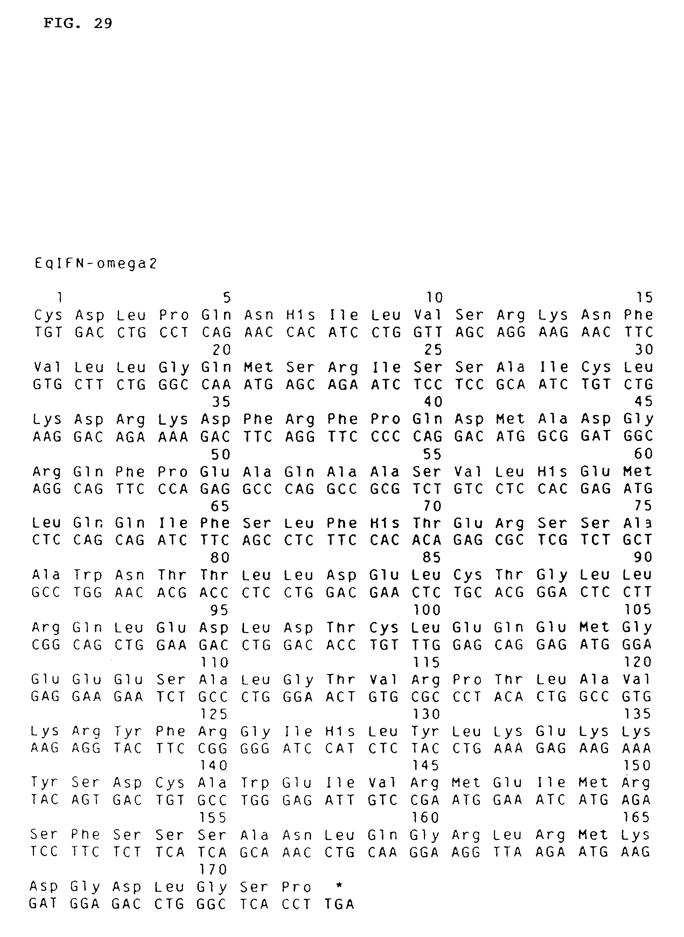

Fig. 29: Aminosäure- und DNA-Sequenz des reifen EqIFN-omega2.

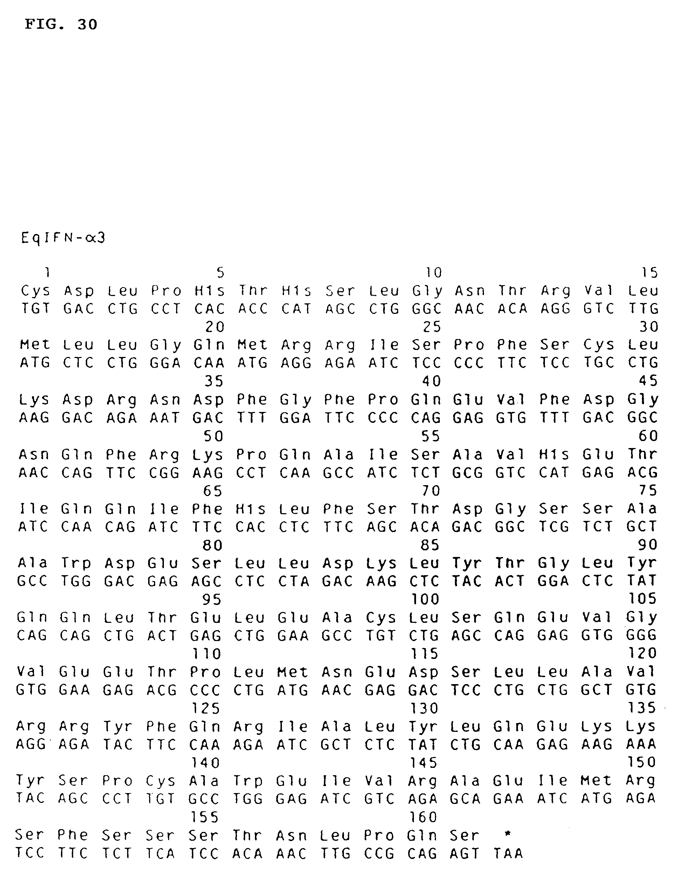

Fig. 30: Aminosäure- und DNA-Sequenz des reifen EqIFN-alpha3.

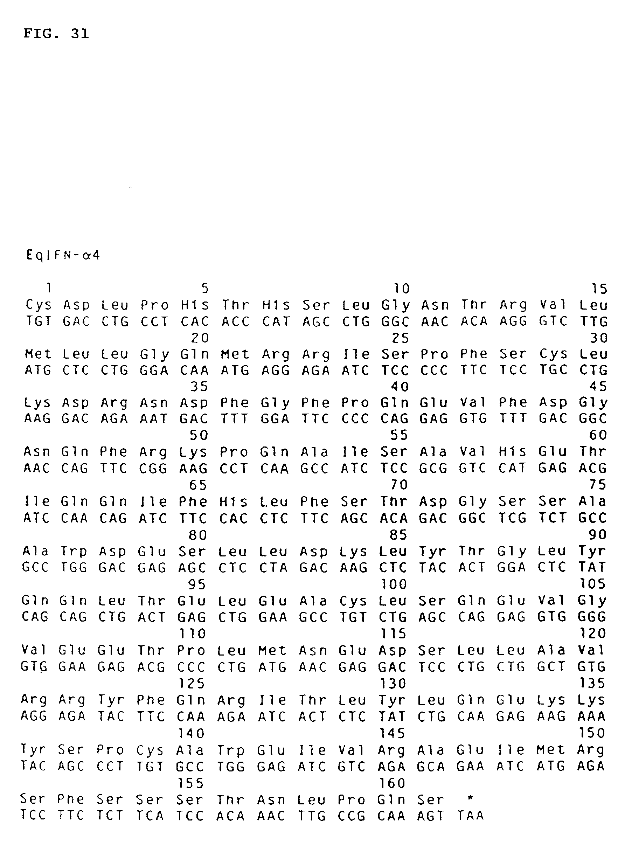

Fig. 31: Aminosäure- und DNA-Sequenz des reifen EqIFN-alpha4.

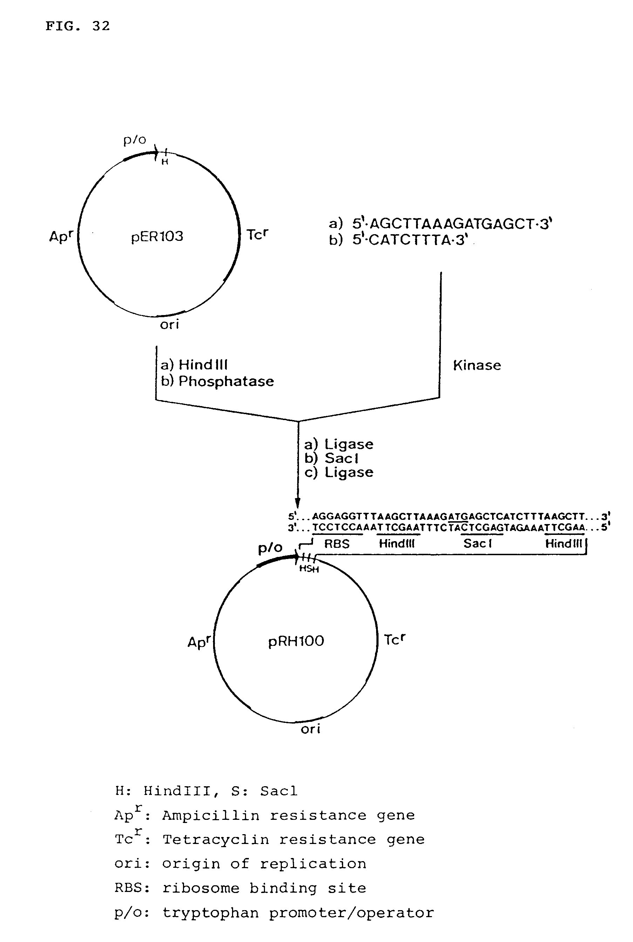

Fig. 32: Konstruktion des Plasmides pRH100.

### A) Isolierung von Pferde-DNA

Gefrorenes Gewebe, z.B. Pferdeleber wurde in flüssigem Stickstoff zu feinem Pulver zermahlen und in 0,5M EDTA, 10 mM Tris-HCI pH 8,0, 0,5 % SDS, 0,1 mg/ml Proteinase K (20 ml/g Gewebe) 3 Stunden bei 55°C inkubiert. Die erhaltene, viskose Lösung wurde durch eine Phenolextraktion und 3-malige Extraktion mit Phenol/Chloroform/lsoamylalkohol (25/24/1 Vol) von Protein befreit, gegen 50mM Tris-HCI pH 8,0, 10mM EDTA, 10 mM NaCI dialysiert und die DNA mit 2 Volumina Äthanol ausgefällt. Nach vollständiger Trocknung im Vakuum wurde die DNA in TE-Puffer (10mM Tris-HCI pH 8,0, 1 mM EDTA) bei 4°C in Lösung gebracht und mit 1,273 g CsCI/ml Lösung 62 Stunden mit 40.000UpM bei 20°C zentrifugiert (Sorvall 50Ti-Rotor). Der CsCI-Gradient wurde ausgetropft, die DNA enthaltenden Fraktionen gegen TE-Puffer dialysiert und die DNA anschließend mit 2 Volumina Äthanol gefällt, mit 70%igem Äthanol gewaschen, getrocknet und wieder in TE-Puffer gelöst (4°C).

Das fertige DNA-Präparat war frei von RNA und länger als 50 kb (durch Elektrophorese an einem 0,45 %igen Agarosegel bestimmt).

### B) Partielle Endonuklease Verdauung und Größenfraktionierung von Pferde-DNA

Zweimal 50µg Pferde-DNA wurden mit 1,6 Einheiten Sau3A in 450µl Reaktionsmedium (10 mM Tris-HCI pH 7,5, 10 mM MgCl₂ 1 mM Dithiothreitol) bei 37°C inkubiert. Nach 15,25 und 40 Minuten wurden 150 µl Aliquots entnommen und mit 15mM EDTA versetzt und durch 10minütiges Erwärmen auf 70°C die Reaktion gestoppt. Nach Zusatz von 0,3M NaAcetat pH6,0 wurde die DNA mit 2,5 Volumina Äthanol gefällt. Nach dem Wiederauflösen in TE-Puffer wurde die DNA auf einem 0,45%igen Agarosegel in TBE-Puffer (10,8g/1 Tris, 5,5 g/I Borsäure,0,93 g/I (Na₂EDTA) bei ca. 1 V/cm über Nacht elektrophoretisch der Größe nach getrennt. Anhand von Größenmarkern (mit EcoRI und Hindlll doppelverdaute und mit Hindlll verdaute Lambda-DNA) wurde das Gelstück mit DNA einer Länge von 10-23kb herausgeschnitten, die DNA aus dem Gel in einen Dialysenschlauch 3 Stunden bei 300V elektroeluiert (Puffer 0,1 x TBE), auf einer Elutip-D-Säule (Schleicher und Schüll) gemäß der Verwendungsvorschrift gereinigt und anschließend mit Äthanol gefällt.

Um die Selbstligation von Pferde-DNA-Fragment zu verhindern, was einerseits zu artifiziellen Hybriden von Pferde-DNA-Sequenzen, andererseits zu zu großen und damit nicht mehr in Lambda-Phagen verpackbaren DNA-Stücken führen kann, wurden die größenfraktionierten Pferde-DNA-Stücke dephosphoryliert.

Dazu wurde die DNA in 140µl Reaktionsmedium (50 mM Tris-HCI pH 9,5, 10mM MgC1₂,0,1 mM ZnAcetat, 1 mM Spermidin) mit 5 Einheiten Bovine Intestinal Phosphatase 30 Minuten 37°C inkubiert, weitere 5 Einheiten Enzym zugegeben und 30 Minuten inkubiert. Nach Zugabe von EDTA auf 25mM Endkonzentration wurde die DNA einmal mit Phenol/Chloroform/lsoamylalkohol (25/24/1 Vol) 2-mal mit Chloroform/Isoamylalkohol (24/1 Vol) und 3-mal mit Diäthyläther extrahiert, mit Äthanol gefällt, getrocknet und in 0,1 x TE-Puffer gelöst.

### C) Aufbau der Pferdegenom-DNA-Bibliothek

Die dephosphorylierten. 10-23kb Pferde-DNA-Fragmente wurden in einem lambda-Vektor, beispielsweise Lambda-EMBL3 oder -3A (3) mit G-A-T-C kohäsiven Enden, durch Entfernung des internen BamHl-Fragmentes der Phagen-DNA gewonnen, geklont.

Der Vektor wurde in einem E.coli Stamm mit dem Suppressorfaktor sup F, beispielsweise E.coli NM526, 538 oder 539 (3), in LB-Brühe (20) mit 5mM MgS0₄ gezüchtet, mit Polyäthylenglykol gefällt und durch 2-malige CsCI-Dichtegradientenzentrifugation (0.71 g CsCI/ml Lösung. 40 Stunden 45.000 UoM, 20°C) gereinigt. Nach Dialyse gegen TE-Puffer wurde die Phagen-DNA durch 2-malige Extraktion mit Phenol/Chloroform/lsoamylalkohol (25/24/1 Vol) und 2-malige Extraktion mit Chloroform/Isoamylalkohol (24/1 Vol) von Protein befreit und durch Äthanolfällung aufkonzentriert.

Zur Gewinnung der Endfragmente von EMBL3A wurden 50µg Phagen-DNA 2 Stunden bei 37°C in 450µl Reaktionsmedium (10 mM Tris-HCI pH 7,5, 10 mM MgCI₂, 1mM Dithiothreitol) vollständig mit BamHl verdaut, mit 15mM EDTA 10 Minuten bei 70°C die Reaktion gestoppt und die DNA mit Äthanol gefällt.

Zur Vermeidung der Religation wurde das Mittelfragment mit EcoRI nachgeschnitten und das wegfallende Oligonukleotid mittels Isopropanolfällung entfernt.

Die BamHI-verdaute Lambda-DNA wurde hierzu 2 Stunden bei 37°C in 450µl 10mM Tris-HCI pH 7,5, 100 mM NaCI, 10 mM MgCl₂ mit EcoRI vollständig verdaut und die Reaktion durch Zusatz von 15mM EDTA und 10 minütiges Erwärmen auf 70°Cgestoppt. Nach Zusatz von NaAcetat zu einer Endkonzentration von 0,3M wurden die 3 großen DNA-Fragmente mit 0,6 Volumina Isopropanol 15 Minuten bei 0°C gefällt, 2- mal mit 0,45M NaAcetat/0,6 Volumina Isopropanol und 1-mal mit 0,3M NaAcetat/2,5 Volumina Äthanol gewaschen und in 15µl 0,1 x TE-Puffer gelöst. Die BamHI/EcoRI-Linker bleiben bei dieser Prozedur in Lösung.

Die EMBL3A Fragmente (8µg) wurden mit ca. 5 µg 10-23kb Pferde-DNA und 10 Einheiten T4-DNA-Ligase (NEN) vereinigt und über Nachtbei 14°C und einen Tag bei 4°C in 50µl Ligationsmedium (66mM Tris-HCI pH 7,2, 0,1 M NaCI, 10mM MgCI₂, 1 mM EDTA, 5mM Dithiothreitol, 0,5mM ATP) inkubiert. Das ligierte DNA-Gemisch wurde mittels eines in vitro-Lambda-Verpackungssystems (27) in reife Lambda-Phagenpartikel gepackt.

Die Komponenten dieses Systems, d.h. Ultraschallextrakt (SE), Gefrier-Auftau-Lysat (FTL), Puffer M1 und A wurden gemäß (27) hergestellt. 10µl Aliquots des ligierten DNA-Gemisches wurden 2 Minuten bei Raumtemperatur mit 25µl SE inkubiert, das ebenso wie FTL 30 Minuten aus Eis aufgetaut worden war, mit 100µl FTL versetzt und 60 Minuten bei Raumtemperatur weiterinkubiert. Das Packungsgemisch wurde mit 150µl Lambda-Diluent (100 mM Tris-HCI pH 7,5, 10mM MgS0₄ 1 mM EDTA) verdünnt und bei 4°Cgelagert.

Ein kleiner Anteil der gepackten Lambda-Phagen wurde am E.coli Stamm NM 528 SupF titriert. Insgesamt ergab das Verfahren etwa 1x10⁶ unabhängige Pferde-DNA-Rekombinanten. Der Rest des verpackten Materials wurde durch Plattieren auf NM528 in einer Dichte von 30.000 plaquebildenden Einheiten (pfu) pro 13,5 cm LB/MgS0₄-Agar-Platte vervielfacht.

### D) Screening der Pferde-Genbibliothek für Interferongene

Zur Identifizierung der rekombinanten Phagen, die Hundeinterferongene beinhalten, wurde die durch Southern-Blots (17) nachgewiesene Nukleotidhomologie mit radioaktiv markierten Human-IFN-alpha-Genen ausgenützt.

Hierzu wurden je 10µg hochmolekulare Pferde-DNA vollständig mit EcoRI bzw. Hindlll verdaut, auf 0,8%igem Agarosegel elektrophoretisch aufgetrennt und auf Nitrocellulosefilter übertragen. Aus einem aus dem Expressionsplasmid pER33 (14) stammenden 845 bp Hindlll-Fragment, das den gesamten proteincodierenden Bereich für reifes Humaninterferon-alpha2ARG enthält, wurde nach üblichen Verfahren (25) ein P-32-markiertes DNA-Stück hergestellt.

Für das Screening nach equinen beta-Interferongenen wurde wie oben aus einem 363 bp Pstl-Bglll Fragment eines für humanes beta-Interferon codierenden cDNA-Klons P1 F12 (15) eine radioaktiv markierte DNA-Probe hergestellt. Diese Probe codiert für die Aminosäuren 48-166 des reifen ß-Interferons.

Die Nitrocellulosefilter wurden 7 Stunden bei 65°C in 5xSSPE(0,9M NaCI, 50mM NaH₂P0₄, 5mM EDTA, pH 7,4), 5 x Denhardt-Lösung (0,1 % Ficoll, 0,1% Polyvinylpyrrolidon, 0,1 % Rinderserumalbumin), 0,1 % SDS, 20 mg/ml Lachssperma-DNA prähybridisiert und anschließend mit 13 x 10⁶ cpm der markierten Probe in der gleichen Lösung, jedoch ohne Lachssperma-DNA, hybridisiert. Nach Inkubation bei 65°C über Nacht wurden die Filter 4-mal 1 bis 1,5 Stunden in 3 x SSC (o,45 M NaCl,45mM NaCitrat), 0,1 % SDS bei 65°C gewaschen und 7 Tage auf Kodak X-omat S-Röntgenfilm mit Kodak Regular Verstärkerfolien exponiert (Fig.1). Das Auftreten von mehreren Banden beweist eine Familie von alpha-Interferongenen bei Pferden wie sie bei Rindern, Schweinen und Menschen früher nachgewiesen wurde.

Für das Screening der Interferongene in der Pferde-DNA-Bibliothek wurden daher die gleichen Hybridisierbedingungen angewandt.

600.000 rekombinante Lambda-Phagen wurden auf E.coli NM528 in einer Dichte von 30.000pfu/13,5 cm Platteplattiert. Vierfache Nitrocellulosereplikas wurden von jeder Platte nach dem Verfahren von Benton und Davis (19) hergestellt.

Nach 2 Stunden Backen bei 80°C wurden die Filter 1,5 Stunden bei 65°C in in 1 M NaCI, 10 mM Tris-HCI pH 8,0, 0,1 % SDS gewaschen, über Nacht wie oben beschrieben, prähybridisiert und je 2 Filterreplikas von jeder Platte 24 Stunden mit 1,5 x 10⁶cpm radioaktiver alpha-Interferon-Probe bzw. 1 x 10⁶ cpm β-Interferon -Probe pro Filter hybridisiert. Nach 3-mal wiederholtem Screening wurden 8 Pferde-alpha-Interferon-Klone und 6 Pferde-beta-Interferon-Kloneerhalten, die positive Hybridisierungssignale ergaben.

### E) Charakterisierung der rekombinanten Phagen

Von 7 mit humanem alpha-IFN und 3 mit humanem β-IFN hybridisierenden Rekombinanten wurde Phagen-DNA hergestellt.Die DNAs wurden mit EcoRl, BamHl, Hindlll, Pstl, Bglll, Sall, Smal verdaut und in einem 0,8 %igen Agarosegel elektrophoretisch aufgetrennt. Die Größe der hybridiserenden Fragmente wurde nach dem Southern-Verfahren bestimmt. Die Position der Restriktionsstellen innerhalb des Lambda-Inserts wurde mittels einer Methode von Rackwitz et al. (4) nach partiellem Restriktionsverdau der Lambda-DNA, Markierung der rechten bzw. linken kohäsiven Enden der Lambda-Arme mit synthetischen, P-32- markierten Oligonukleotiden und Elektrophorese in 0,45 %igen Agarosegelen bestimmt. Die daraus erhaltenen Restriktionskarten der Klone Eq-alpha1 , Eq-alpha16, Eq-alpha20 und Eq-beta6 sind in Fig. 2, 3 und 22 dargestellt.

### F) Subklonierung des Pferdeinterferon-alpha-Gens

Ein Restriktionsfragment des Klons Eq-alphal , das mit dem humanen alpha-Interferon-Marker hybridisiert hatte, wurde in die Mehrfachrestriktionsenzym-Klonierungsstelle des pBR322-Derivates pUC9 subkloniert. Die Insertion eines fremden DNA-Fragments führt zu einer Unterbrechung des lac z-Genes der β-Galaktosidase und verändert damit den Phänotyp des mit dem Plasmid transformierten E.coli Stammes JM101 von lac + zu lac-. Aufgrund der nicht funktionierenden ß-Glaktosidase kann mit Isopropylthiogalaktosid (IPTG) induzierter JM101 das farblose Substratanalog 5-Brom-4-chlor-3-indolyl-ß-D-galactosid (BCIG) nicht zum blauen Farbstoff spalten. Bakterienkolonien mit lac-Phänotyp können daher an der weißen Farbe erkannt werden.

Ein 3,2 kb Hindlll-Fragment aus dem Klon Eq- alphal wurde aus einem Agarosegel eluiert, auf einer Elutip-D-Säule gereinigt und in etwa 10-fach molarem Überschuß mit 40ng mit Smal geschnittenem und dephosphoryliertem pUC9-Vektor ligiert, in E.coli JM101 transformiert und mit LB Top-Agar mit 0,2mg/ml BCIG, 0,17 mg/ml IPTG und 0,1 mg/ml Ampicillin ausgegossen. Weiße Kolonien wurden in 5ml LB-Brühe mit 0,1 mg/ml Ampicillin über Nacht bei 37°C hochgezüchtet und nach dem eingesetzten Fragment mit einem Plasmid-Minipräparationsverfahren (25) gescreent. Ein so erhaltenes Plasmid wurde pAH50 benannt.

### G) DNA-Sequenz von Pferde-alpha-Interferon-Genen aus Klon Eq-alphal

Das 3,2 kb Hindlll-Insert von pAH50 (3,2 kb Hindlll-Fragment Subklon von Eq-alphal , Fig.3) wurde nach der Dideoxy-Methode von Sanger (23) nach dem Shotgun-Verfahren sequenziert. 60µg pAH50 Plasmid-DNA wurde vollständig mit Hind III verdaut, das 3,2kb Fragment aus einem 1 %igen Agarosegel isoliert und wie oben beschrieben gereinigt.

15µg dieses Fragments wurden in 100µl Ligationsmedium mit 14 Einheiten T₄-DNA-Ligase über Nacht bei 14°C und weitere 4 Tage bei 4°C mit sich selbst ligiert. Diese ligierte DNA wurde in einem Eisbad mit Ultraschall in 20 Sekunden Pulsen, insgesamt 100-140 Sekunden, in kleine Stücke zerteilt. Die DNA-Enden wurden 2 Stunden bei 14°Cin 250µl Reaktionsmedium (50mM Tris-HCI pH 7,5, 10 mM MgCI₂, 1mM Dithiothreitol, 0,5 mg/ml Rinderserumalbumin je 0,1 mM dATP, dGTP, dGTP, dTTP) mit 15 Einheiten des großen Fragmentes der E.coli Polymerase I (Klenow Fragment) repariert. Nach Konzentrierung durch Äthanolfällung wurde die so vorbehandelte DNA auf einem 1%igen Agarosegel aufgetrennt und DNA-Fragmente in einem Größenbereich von 0,35-1,Okb isoliert und gereinigt. Die Fragmente wurden in etwa 10- fach molarem Überschuß mit der mit Smal geschnittenen und dephosphorylierten, replikativen Form von Bakteriophage M13mp8 (22) ligiert und E.coli JM101 transformiert.Die Einzelstrang-DNA der so erhaltenen, rekombinanten Phagen wurde isoliert und nach Binden eines synthetischen Oligonukleotides die Zweitstrangsynthese in 4 Einzelreaktionen mit dem Klenow Fragment der E.coli DNA-Polymerase I durchgeführt.

Die Sequenzen der Inserts der verschiedenen rekombinanten Phagen wurden mit Hilfe eines von C. Pieler modifizierten Computerprogrammes von Staden (24) zu einer Totalsequenz vereint, die in Fig.4 dargestellt ist.

### H) Subklonierung des Pferde-ß-Interferon-Gens

Für die Subklonierung des im Lambda-Klon Eq-beta6 identifizierten Pferde-ß-Interferongens wurde analog wie bei F) vorgegangen. Ein 4.5 kb Pvull-Fragment, das mit der humanen β-Interferonprobe hybridisierte, wurde isoliert, gereinigt und in die Smal Restriktionsstelle des Plasmides pUC9 mit glatten Enden ligiert und E.coli JM101 transformiert. Ein Transformant mit gewünschtem Insert (pAH60) wurde großgezüchtet und das Plasmid mittels Southern-Analyse genauer charakterisiert. Die erhaltene Restriktionskarte ist in Fig.3 dargestellt. Das 2.5 kb Hindlll-Fragment wurde analog G) nach der Dideoxy-Methode von Sanger sequenziert. Die in Fig.8 dargestellte Gesamtsequenz des 2.5 kb Fragmentes wurde aus 52 Einzelsequenzen zusammengesetzt.

### I) Subklonierung und Sequenzierung des Pferde-Interferon-Genes aus Klon Eq-a 16

Ein 3,3 kb langes EcoRI Restriktionsfragment aus dem Lambda-Klon Eq-a16, das mit einem humanen a IFN-Marker hybridisiert hatte (siehe Beispiel D,E), wurde in die EcoRl-Stelle des Plasmides pUC8 subkloniert. Ein erhaltenes Plasmid wurde pRH63 benannt. Anhand einer erstellten Restriktionskarte (Fig. 9) wurden definierte Restriktionsfragmente gerichtet in M13-Phagen subkloniert und nach der Sanger-Methode die DNA-Sequenz bestimmt (Fig. 10).

### J) Subklonierung und Sequenzierung des Pferde-Interferon-Genes aus Klon Eq-a20

Ein 2,2 kb langes EcoRI-Fragment des Lambda-Klons Eq-a20, das schwach mit der humanen a-IFN Probe hybridisiert hatte, wurde in die EcoRl-Stelle des Plasmids pUC9 subkloniert. Ein erhaltener Klon wurde pRH61 benannt (Fig. 9). Das gesamte 2,2 kb EcoRl-Insert wurde isoliert und nach dem Shotgun-Verfahren mit der Sanger-Methode (Beispiel G) die DNA-Sequenz bestimmt (Fig. 12).

### K) Herstellung des Expressionsplasmides parpATER103

Ausgehend von dem Expressionsplasmid parpER33 wurde die für die erhöhte Plasmidstabilität in E.coli verantwortliche "par"-Sequenz und die Tryptophan Promoter-Operator-Sequenz samt der künstlichen ribosomalen Bindungsstelle in den Plasmidvektor pAT153 (Amersham) eingesetzt. pAT153 ist ein verkürztes Derivat des Plasmides pBR322, dem ein Abschnitt fehlt, der für die Mobilisierung von DNA notwendig ist (36).

Die Vorgangsweise der Herstellung von Plasmid parpATER103 ist in Fig. 13 dargestellt.Das Plasmid parpER33 wurde mit Hindlll vollständig und mit EcoRI partiell geschnitten, das entstandene 0,47 kb lange DNA-Fragment von einem Agarosegel isoliert und gereinigt und mit EcoRI und Hindlll zweifach geschnittenem pAT153 ligiert. Ein nach Transformation von E.coli HB101 erhaltenes Plasmid der gewünschten Struktur, die man durch Verdauung mit verschiedenen Restriktionsenzymen bestimmte, wurde parpATER103 benannt.

### L) Direkte Expression von reifem EqIFN-a1 in E.coli

Die Herstellung der Expressionsplasmide pAH52, pAH52/2 und pAH53 sowie deren Vorstufen ist in Fig. 14 dargestellt. 20 µg des Plasmides pAH50 (Beispiel F) wurden mit 30 Einheiten Hindll (Boehringer Mannheim) verdaut und das 4,2 kb lange DNA-Fragment, welches das gesamte EqIFN-a1 Gen enthält von einem Agarosegel mit DE81-Papier (Whatman) isoliert und gereinigt. Dazu wird nach Auftrennung der DNA-Fragmente im Agarosegel vor und hinter die zu isolierende DNA-Bande ein Schlitz geschnitten, in den ein Streifen DE81-Papier gesteckt wird. Die Elektrophorese wird weitergeführt, bis das gewünschte DNA-Fragment vollständig an dem vorderen DE81-Streifen gebunden ist. Der hintere DE81-Streifen verhindert eine Verunreinigung durch größere DNA-Fragmente. Das DE81-Papier mit da gebundenen DNA-Fragment wird zweimal 5 Minuten in 400 µl Niedrigsalz-Puffer (0,2 M NaCI, 25 mM Tris-HCI pH 8,0, 1 mM EDTA) gewaschen und anschließend die DNA zweimal 10 Minuten mit 200 µl Hochsalz-Puffer (1 M NaCI, 25 mM Tris-HCI pH 8,0, 1 mM EDTA) vom DE81-Papier eluiert und mit 1 ml Äthanol ausgefällt. Die Enden des Hindll-Fragmentes wurden mit Sphl-Linkern versehen. Dazu wurden 0,2 µg Sphl-Linker (Worthington) mit 2 Einheiten Polynukleotid-Kinase in 10 µl Reaktionsmedium 45 Minuten bei 37°C inkubiert (70 mM Tris-HCI pH 7,6, 10 mM MgCI₂, 1 mM ATP, 5 mM Dithiothreit). Die kinasierten Sphl-Linker und das Hindll-Fragment wurden mit 8 Einheiten T4-DNA-Ligase 20 Stunden bei 4°C ligiert. Anschließend wurde das Enzym bei 70 ° C inaktiviert und die DNA mit 30 Einheiten Sphl in einem Gesamtvolumen von 100 µl verdaut, mit Phenol und Chloroform extrahiert und mit Äthanol ausgefällt. Die DNA wurde mit Ligase zirkularisiert und E.coli HB101 transformiert. Ein Plasmid der gewünschten Struktur erhielt die Bezeichnung pAH51. Es enthält das EqIFN-a1-Gen mit einer verkürzten 3'-nichttranslatierten Region und einer zusätzlichen Sphl-Schnittstelle.

Um in der Endkonstruktion die DNA-Sequenz für das reife Pferde-a-Interferon in der richtigen Distanz mit der Promotersequenz zu verbinden, wurde von eina 0,4 kb langen DNA-Fragment ausgegangen, das aus 20 µg mit Pvull geschnittenem Plasmid pAH50 isoliert wurde. 1 nmol synthetisches 15mer Oligonukleotid mit der Sequenz 5'-TGTGACCTGCCTCAC wurde mit Polynukleotid-Kinase kinasiert. Es enthält die Sequenz, welche für die ersten 5 Aminosäuren des reifen EqIFN-a1 aus Klon pAH50 codiert. Das 15mer wurde mit etwa 7 pmol des 0,4 kb Pvull-Fragmentes gemischt und in einem Gesamtvolumen von 34 µl fünf Minuten gekocht, um den DNA-Doppelstrang zu denaturieren. Nach Abkühlen wurde der an den Einzelstrang gebundene Oligonukleotid-Primer in 70 µl Reaktionsmedium (50 mM Tris-HCI pH 7,2, 10 mM MgS0₄, 0,1 mM Dithiothreit, 50 µg/ml Rinderserumalbumin,je 1 mM dATP, dGPT, dCTP, dTTP) mit 30 Einheiten Klenow-Fragment 3 Stunden bei 37°C verlängert. Um einen eventuell verbliebenen 3'-Überhang sicher zu entfernen wurde die DNA anschließend mit 16 Einheiten T4 DNA-Polymerase 20 Minuten bei 37°C in 120 µl Reaktionsmedium inkubiert (33 mM Tris-Acetat pH 7,9, 66 mM KAc, 10 mM Mg(Ac)₂, 0,5 mM Dithiothreit, 0,1 mg/ml Rinderserumalbumin, je 1 mM dATP, dGTP, dCTP, dTTP). Die entstandene DNA mit geraden Enden wurde mit Phenol und Chloroform extrahiert und mit 0,45 M NaAcetat und 0,6 Volumsteilen 2-Propanol 15 Minuten bei 0 ° C ausgefällt. An dieses DNA-Stück wurde ein Gemisch zweier kinasierter, zueinander komplementärer Oligonukleotide: 12mer 5'-AGCTTAAAGATG, 8mer 5'-CATCTTTA ligiert, das eine Hindlll-Schnittstelle und das Translationsstartcodon ATG erzeugt. Je 1 nmol dieser zwei Oligonukleotide wurde in 20 µl mit 14 Einheiten Ligase 40 Stunden bei 4 ° C an das DNA-Fragment ligiert. Nach Inaktivierung des Enzyms bei 70 ° C wurde die erhaltene DNA in 100 µl mit 80 Einheiten Hindlll und 20 Einheiten Bglll geschnitten und DNA-Fragmente von etwa 190 bp Länge aus einem 2%igen Agarosegel mit DE81-Papier isoliert und gereinigt. Das erhaltene DNA-Fragment wurde mit etwa 50 ng mit Hindlll und Bglll doppelt geschnittenem pAH51-Vektor ligiert und E.coli HB101 transformiert.

Von 65 erhaltenen Kolonien wurde aus 4 Plasmiden, die das gewünschte Restriktionsmuster aufwiesen, ein Hindlll/BamHl DNA-Fragment isoliert und mit der Sanger-Methode sequenziert, wobei zwei Klone genau die gewünschte Sequenz enthielten. Ein solches Plasmid wurde pAH51/2 benannt. Dieses enthält die Sequenz für reifes EqIFN-a1 mit vorangestelltem Translationsstartcodon ATG und Hindlll-Schnittstelle.

### Herstellung der Expressionsplasmide pAH52 und pAH52/2

20 µg Plasmid pAH51/2 wurden mit Sphl und Hindlll zweifach geschnitten, das entstehende 1,0 kb lange DNA-Fragment von einem Agarosegel isoliert und mit 40 ng Hindlll und Sphl doppelt geschnittenem Plasmid parpATER103 (Beispiel K) ligiert. Ein nach Transformation von E.coli HB101 erhaltenes Plasmid der gewünschten Struktur wurde pAH52 benannt. Es enthält alle für eine induzierbare Expression von reifem EqIFN-a1 notwendigen Informationen. In analoger Weise wurde aus mit Hindlll und BamHl doppelt geschnittenem pAH51/2 und mit Hindlll/BamHl geschnittenem parpATER103 das Plasmid pAH52/2 hergestellt. Dieses Expressionsplasmid ist etwa 0,2 kb größer als pAH52 und weist zusätzlich eine singuläre BamHl-Schnittstelle auf.

### Herstellung des Plasmides pAH53

Ein wesentlich verkleinertes Expressionsplasmid zur Herstellung von reifem EqIFN-a1 in E.coli, in dem Tryptophan-Promoter, Interferongen, Ampicillin-Resistenzgen und Replikationsursprung in einer Richtung orientiert sind, wurde aus den Plasmiden pAH52 und pBR322 hergestellt. 10 µg pAH52 wurden mit Sphl und EcoRI geschnitten, die Enzyme bei 70 ° C inaktiviert und die DNA-Enden nach Zusatz von je 0,15 mM dATP, dGTP, dCTP, dTTP mit Klenow-Fragment 1 Stunde bei 22 ° C stumpf gemacht.

Die DNA-Fragmente wurden auf einem Agarosegel nach der Größe fraktioniert und ein 1,1 kb langes Stück isoliert, das Promoter und Interferongen enthält.10 µg pBR322 Plasmid wurde mit EcoRI und Pvull doppelt verdaut, die Enden wie oben beschrieben mit Klenow-Fragment begradigt und anschließend mit Kalbsdarmphosphatase dephosphoryliert. Ein DNA-Fragment von 2,4 kb Länge wurde von einem Agarosegel isoliert. Die beiden so erhaltenen DNA-Stücke wurden mit T4 DNA-Ligase verknüpft und E.coli HB101 transformiert. Ein so erhaltenes Plasmid, in da zwei EcoRl-Erkennungsstellen geschaffen wurden, erhielt die Bezeichnung pAH53.

### M) Herstellung eines Expressionsplasmides für EqIFN-a2 (pAH55)

Aufgrund der hohen Homologie der Gene für EqIFN-a1 (pAH50) und EqIIFN-α2 (pRH63, Fig. 11) ist es möglich, aus da Expressionsplasmid pAH52/2 (Beispiel L) und dem Lambda-Subklon pRH63 ein Expressionsplasmid für EqIFN-a2 herzustellen (Fig. 15). 20 u.g pRH63 Plasmid wurden mit Bglll und BamHl zweifach geschnitten und das entstehende DNA-Fragment von 1,0 kb Länge, das die codierende Sequenz für EqIFN-a2 ab der 64.Aminosäure enthält von einem Agarosegel isoliert. 10 µg des Plasmides pAH52/2 wurde ebenfalls mit Bglll und BamHl geschnitten, die Enden mit Kalbsdarmphosphatase dephosphoryliert und das größere der zwei entstehenden DNA-Fragmente von einem Agarosegel gewonnen. Dieses DNA-Stück enthält den Plasmidvektoranteil, den Promoter und die codierende Sequenz für die ersten 63 Aminosäuren des reifen Interferons. Die beiden beschriebenen DNA-Fragmente wurden mit Ligase verknüpft und E.coli HB101 transformiert. Ein so erhaltenes Plasmid, welches das Insert in der korrekten Orientierung enthält (mit BamHl und Bglll schneidbar!) wurde pAH55 benannt. Dieses Plasmid ermöglicht die Expression von reifem EqIFN-a2 in E.coli.

### N) Herstellung eines Expressionsplasmides für reifes EqIFN-ß (pAH62)

Die Vorgangsweise ist in Fig. 16 schematisch dargestellt. 30 µg pAH60 Plasmid wurden mit 30 Einheiten HgiAI in 150 µl Volumen geschnitten. Nach Inaktivierung des Enzyms bei 70°C wurden die 3'überhängenden DNA-Enden mit 7 Einheiten T4 DNA-Polymerase (Zugabe von je 1 mM dATP, dGTP, dCTP, dTTP) 30 Minuten bei 37°C begradigt. An die stumpfen Enden wurden Sphl-Linker ligiert (siehe Beispiel L) und die erhaltene DNA mit Sphl und Hindlll geschnitten. Ein entstandenes DNA-Fragment von 1,85 kb Länge wurde von einem Agarosegel isoliert und mit 50 ng mit Hindlll und Sphl doppelt geschnittenem Plasmid parpATER103 (Beispiel K) ligiert. Ein nach Transformation von E.coli HB 101 erhaltener Klon mit da gewünschten Plasmid wurde pAH61 benannt. Dieses Plasmid stellt eine Zwischenstufe für die weitere Konstruktion des Expressionsplasmides dar. 20 µg Plasmid pAH61 wurden mit BamHl und Sall zweifach geschnitten und ein entstandenes 1,3 kb langes DNA-Fragment von einem Agarosegel isoliert, gereinigt und mit BamHl/Sall doppelt verdauter M13mp9 Phagen-DNA ligiert. Nach Transformation von E.coli JM101 konnte von einem rekombinanten M13-Phagen (M13pAH61) einzelsträngige Phagen-DNA gewonnen werden. 3 pmol dieser Einzelstrang-DNA wurden mit 38pmol kinasiertem 15mer Oligonukleotid 5'GTGAACTATGACTTG in 50 µl 20 mM Tris HCI pH 8,0, 10 mM MgCl₂ gemischt, auf 95°C erhitzt und langsam auf Raumtemperatur abgekühlt. Das Oligonukleotid bindet genau ab der ersten Base der Sequenz des reifen ß-Interferons. Die Zweitstrangsynthese an der Einzelstrangvorlage, ausgehend vom 15mer-Primer wurde in 100 µl Volumen nach Zugabe von je 3 mM dATP, dGTP, dCTP, dTTP und 15 Einheiten Klenow-Fragment 1 Stunde bei 22 °C durchgeführt. Nach Zusatz von 20 mM EDTA wurde die DNA mit Phenol und Chloroform extrahiert und mit Äthanol ausgefällt.

Verbliebene, einzelsträngige DNA-Abschnitte wurden mit 150 Einheiten S1-Nuklease (Sigma) in 400 µl Reaktionsmischung 2 Stunden bei 14°C verdaut (4 mM Zn(Ac)₂, 30 mM NaAC, 250 mM NaCI, 5% Glycerin, pH 4,6). Die Reaktion wurde durch Zugabe von EDTA und Extraktion mit Phenol und Chloroform gestoppt und die DNA mit Äthanol ausgefällt. An die durch diese Behandlung stumpfendig gemachte DNA wurde wie in Beispiel H das Gemisch der 12mer und 8mer Oligonukleotide 5'-AGGTTAAAGATG und 5'-CATCTTTA anligiert und die entstandene DNA mit Hindlll und Sphl geschnitten. Ein DNA-Fragment der gewünschten Länge von 1,1 kb wurde von einem Agarosegel isoliert und mit Hindlll/Sphl doppelt geschnittenem Plasmid parpATER103 ligiert. Nach Transformation von E.coli HB101 wurden 54 Kolonien erhalten. Von 9 daraus isolierten Plasmid-DNAs wurde ein 1,3 kb langes EcoRl/Sall Fragment isoliert und mit der Sanger-Methode sequenziert. Ein daraus ermitteltes Plasmid mit der gewünschten Sequenz wurde pAH62 benannt. Dieses Plasmid ermöglicht die effiziente Expression von reifem EqIFN-ß Protein in E.coli. Ein Plasmid, das eine Deletion der ersten Base (G) des reifen β-IFN Genes trägt, wurde pAH62deltaGl benannt. Dieses Plasmid erlaubt die Expression eines am Amino-Terminus verkürzten β-IFN durch Translationsstart am nächstfolgenden ATG (entspricht Aminosäure 19 des reifen ß-IFN), das überraschenderweise antivirale Aktivität, wenn auch deutlich weniger, verglichen mit dem unverkürzten Protein, aufweist (siehe Beispiel 0).

### O) Expression der Interferonaktivität durch E.coli HB 101 enthaltend das Plasmid pAH52, pAH52/2, pAH53, pAH55 oder pAH62

100 ml Bakterienkultur werden bei 37°C unter kräftigem Schütteln bis zur unten angegebenen optischen Dichte bei 600 nm in folgendem tryptophanfreiem Medium inkubiert (Angaben pro Liter Medium): 10 g (NH₄)₂P0₄, 3,5 g KH₂P0₄ pH 7,3 mit NaOH, 0,5 g NaCI, 21 g Casaminosäuren (sauer hydrolysiert), 11 g Glucose, 1 MgS0₄, 0,1 mM CaC1₂, 1 mg Thiamin-HCI, 20mg L-Cystein, 20mg 3-ß-Indolacrylsäure (IAA, Induktor für das Tryptophan-Operon), optionsweise 50-100mg Ampicillin. Anschließend werden die Bakterien durch Zentrifugieren 5 Minuten bei 4000 UpM pelletiert, mit 1/10 des Kulturvolumens eiskaltem 50 mM Tris-HCI pH 8,0, 30 mM NaCI suspendiert und unter Eiskühlung zweimal 30 Sekunden mittels Ultraschallsonde (20 kHz, 100 Watt) aufgebrochen. Die Zelltrümmer werden 10 Minuten bei 10.000 UpM (4°C) entfernt und der Überstand nach Sterilfiltration auf Interferonaktivität in einem Assay, der den cytopathischen Effekt (CPE) von Vesicular Stomatitis Virus (VSV) oder Encephalomyocarditis Virus (EMCV) mißt, getestet.
Testsystem: NBL-6 Zellen (ATCC CCL 57, Pferdehaut-Epidermis-Zellen)/VSV A549 (ATCC CCL 185, humane Lungencarcinom-Zellinie)/EMCV

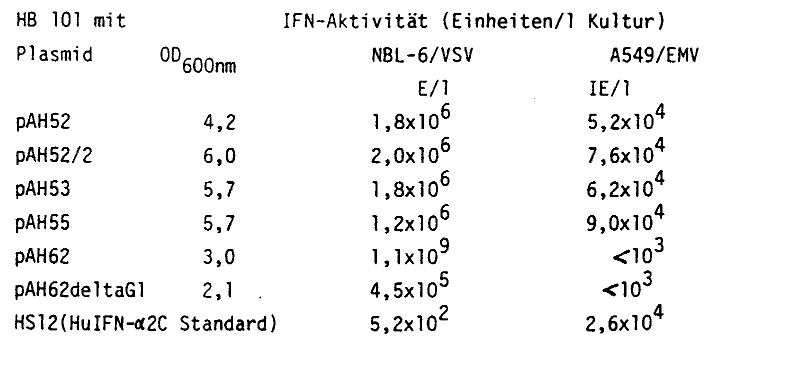

Der Titer auf A 549-Zellen wurde mit human-Interferonstandard auf internationale Einheiten normiert.

### P) Nachweis der exprimierten Pferde-Interferone durch Markierung der Proteine in Maxizellen

Plasmidcodierte Proteine können durch Verwendung der Maxizelltechnik (37) selektiv in vivo markiert werden. E.coli CSR603 wurde nach üblichen Verfahren mit den Expressionsplasmiden transformiert und auf Ampicillin-haltigen Agarplatten auf transformierte Bakterien selektioniert. Die Präparation der Maxizellen und das Markieren der Proteine erfolgte nach einer Vorschrift von A. Sancar (37). Die Zellen wurden in 15 ml Medium (siehe Beispiel 0) ohne Indolacrylsäure bei 37 °C bis zu einer ODₛₒₒₙₘ = 0,5 gezüchtet und 10 ml dieser Kultur in einer Petrischale 5 Sekunden aus 50 cm Entfernung mit einer UV-Germicid-Lampe (15 Watt) unter Schwenken bestrahlt und eine Stunde bei 37°C weiterinkubiert. Die Kulturen wurden mit 100 µg/ml D-Cycloserin versetzt und 14 Stunden bei 37°C inkubiert und die Bakterien anschließend durch Zentrifugation geerntet. Die Zellen wurden zweimal mit 5 ml Hershey-Salzlösung gewaschen, in 5 ml Hershey Medium mit 20 µg/ml Indocrylsäure suspendiert und 2 Stunden bei 37 °C inkubiert. Zu jeder Kultur wurden 5 µCi/ml ³⁵ S-Methionin (1000 Ci/mMol) zugesetzt und eine Stunde bei 37 °C geschüttelt. Die Zellen wurden geerntet in SDS- und 2-Mercaptoethanol-haltigem Elektrophorese-Probenpuffer lysiert und die Proteine auf einem 15 %-igen Polyacrylamidgel aufgetrennt.

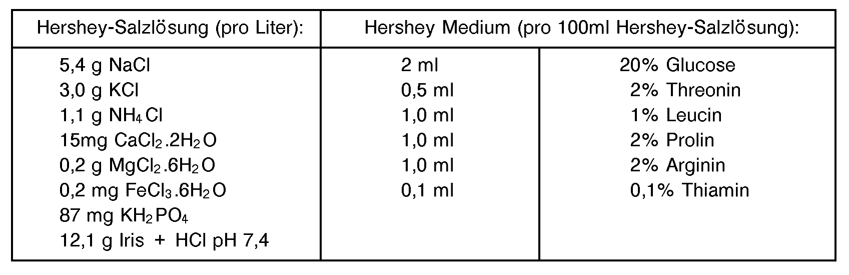

In Fig. 17 ist das Autoradiogramm des getrockneten Geles nach 2 Tagen Exponierung auf DuPont Cronex X-ray Film unter Verwendung einer Kodak Lanex-Regular Verstärkerfolie bei -80 ° C dargestellt. Als Molekulargewichtsstandard wurde ein ¹⁴C-methyliertes Proteingemisch (Amersham) verwendet. Als Kontrollen wurden das Plasmid pER103, das nur den Promoter ohne Interferongen enthält und das Plasmid pER21/1 eingesetzt, welches zwei Kopien des humanen IFN-a 2arg Genes enthält. Die Proteinbanden bei etwa 18 kd sind die von den Plasmiden exprimierten Interferone.

### Q) Nachweis von mit EqIFN-a, EqIFN-ß und EqIFN-omega hybridisierenden Sequenzen in genomischer Pferde-DNA

Zum Nachweis der Gesamtanzahl von Sequenzen im Pferdegenom, welche hohe Homologie mit Interferongenen der Klassen IFN-a, JFN-ß bzw. IFN-omega aufweisen, wurde wie folgt vorgegangen. 30 µg hochmolekulare Pferde-DNA (Beispiel A) wurden mit 100 Einheiten des entsprechenden Restriktionsenzyms in 300 µl Reaktionsvolumen vollständig verdaut und jeweils 10 µg dieser geschnittenen DNA pro Spur auf einem 0,8%igen Agarosegel nach der Größe aufgetrennt.

Nach Southern-Transfer auf Nitrozellulosefilter, denaturieren und fixieren der DNA wurde jedes Filter mit etwa 6x10⁶ cpm nicktranslatierter Probe hybridisiert (17 Stunden bei 65 °C, 5x SSPE, 5x Denhardt-Lösung, 0,1% SDS, 20 µg/ml denaturierte Lachssperma-DNA). Als Probe für EqIFN-a wurde ein 1,0 kb langes Hindlll/Sphl-Fragment aus Plasmid pAH52, für EqIFN-ß ein 1,1 kb langes Hindlll/Sphl-Fragment aus Plasmid pAH62 verwendet, welches jeweils die codierende Sequenz für das gesamte reife Interferon enthält. Als Probe für EqlFN-omega wurde das 2,1 kb EcoRl-Insert aus Plasmid pRH61 eingesetzt. Die Filter wurden anschließend unter stringenten Bedingungen gewaschen, daß keine Kreuzhybridisierung zwischen diesen 3 Interferonsequenzen eintritt: 4mal 45 Minuten bei 65 ° C mit 0,3x SSC (45 mM NaCI, 4,5 mM Na₃Ci-trat),0,1% SDS. Die Autoradiographie erfolgte auf DuPont Cronex X-ray Film unter Verwendung von Kodak Lanex-Regular Verstärkerfolie 7 Tage bei -80 ° C.

Legende zu Figur 18:
Spaltenbezeichnung: M = Größenmarker (Lambda x EcoRl/Hindlll) E = EcoRI, H = HindIII, Ba = BamHI, P = Pstl, B = Bglll

### R) Konstruktion von Expressionsplasmid pRH 100

Alle Enzymreaktionen wurden unter den von den Herstellern angegebenen Bedingungen durchgeführt.

7 µg Plasmid pER 103 (Eva Dworkin-Rastl et al., Gene 21 (1983) 237-248, EP-A-0.115-613) wurden in 50 µl Reaktionsmedium mit der Restriktionsendonuklease Hindlll linearisiert. Nach einer einstündigen Inkubation bei 37°C wurden 50 µl 2x CIP-Puffer zugesetzt (2x CIP-Puffer = 20 mM Tris, pH = 9,2, 0,2 mM EDTA). Durch Zugabe von 2 Einheiten alkalischer Phosphatase aus Kälberdarm (CIP) wurden die 5'terminalen Phosphatreste entfernt; inkubiert wurde bei 45 °C 30 Minuten lang. Die Reaktion wurde durch Zugabe von 4 µl 0,5 EDTA-Lösung sowie Zugabe von 10 µl 1M Tris, pH = 8,0 Lösung gestoppt. Die Proteine wurden durch zweimalige Phenol- und einmalige Phenol-/Chloroformextraktion entfernt. Die DNA wurde aus der wäßrigen Phase nach Zugabe von 0,1 vol 3M Natriumacetatlösung pH = 5,5 und 250 µl Äthanol ausgefällt, das DNA-Präzipitat nach Zentrifugieren einmal mit 70%-iger Äthanollösung gewaschen.Die DNA wurde getrocknet, und das Pellet anschließend in 20 µl TE-Puffer (10 mM Tris pH = 8,0, 1 mM EDTA) gelöst.

Jeweils 1 µg der synthetisch hergestellten Oligodesoxynukleotide d(AGCTTAAAGATGAGCT) und d-(CATCTTTA) wurden in je 10 µl Reaktionslösung unter Zugabe von 10 Einheiten T4-PNK (Polynukleotidkinase) sowie 1 mM rATP phosphoryliert. Die Reaktion fand bei 37°C statt und dauerte 45 Minuten. Die Reaktion wurde durch 10-minütiges Erhitzen auf 70 °C abgebrochen.

Jeweils 5 µl der Plasmidlösung und der phosphorylierten Oligonukleotide wurden gemischt und 5 Minuten auf 70 °C erwärmt. Danach wurde die Lösung auf 0°C abgekühlt, 2 µl 10 x Ligasepuffer (500 mM Tris, pH=7,5), 100 mM MgCl₂ 200 mM DTT (Dithiothreit), 1 mM rATP, 500 µG/ml BSA (Rinderserumalbumin), sowie 2 µl Wasser und 10 Einheiten T4-DNA Ligase zugesetzt. Die Reaktion dauerte 40 Stunden und wurde bei 4°C durchgeführt. Sie wurde durch 10-minütiges Erhitzen auf 70°C abgebrochen.

2 µl dieser Ligasereaktion wurden in insgesamt 30 µl Lösung mit 10 Einheiten der Restriktionsendonuklease Sacl (New England Biolabs) 3 Stunden bei 37°C verdaut. Die Reaktion wurde durch 10-minütiges Erhitzen auf 70 °C abgebrochen. 5 µl dieser Reaktionsmischung wurden in insgesamt 30 µl durch Zugabe von 10 Einheiten T4-PNK bei 14°C 16 Stunden lang ligiert.

200 µl kompetenter E.coli Hb101 wurden mit 10 µl dieser Ligasereaktion versetzt. Die Bakterien wurden 45 Minuten auf Eis gehalten und anschließend 2 Minuten auf 42°C erwärmt, um die DNA-Aufnahme zu ermöglichen. Anschließend wurde die Bakteriensuspension wieder bei 0°C 10 Minuten inkubiert. Abschließend wurden die transformierten Bakterien auf einem LB-Agar, der 50 ug/ml Ampicillin enthielt, ausgestrichen.

Von den entstandenen Bakterienkolonien wurden 12 willkürlich ausgesucht und daraus im Mikromaßstab die Plasmide isoliert (Birnboim und Doly, Nucl. Acids Res. 7 (1979) 1513-1523). Die resultierende DNA wurde mit der Restriktionsendonuklease Sacl geschnitten und die DNA auf einem Agarosegel (1 %, 1x TBE-Puffer) aufgetrennt. Die Wanderung der DNA als lineares Molekül der Größe von etwa 4.400 bp bestätigte die Einführung einer Sacl-Erkennungsstelle in das Plasmid. Eines dieser Plasmide wurde willkürlich ausgesucht. Mit der DNA aus der dazugehörigen Minipräparation wurde nochmals E.coli HB101 transformiert. Von den resultierenden transformierten Bakterien wurde eine Kolonie ausgewählt und in größerem Maßstab angezüchtet. Das daraus isolierte Plasmid wurde mit den Restriktionsendonukleasen EcoRI und BamHl geschnitten, die DNA auf einem 1%-igen Agarosegel aufgetrennt, und das kleinere Fragment aus dem Gel durch Elektroelution isoliert. Dieses etwa 460 bp lange EcoRI-BamHI DNA-Fragment wurde nach Sanger sequenziert. (F. Sanger et al., Proc.Natl.Acad.Sci. (1977) 5463-5467). Das dermaßen analysierte Plasmid wurde pRH 100 genannt.

### S) Subklonierung und Sequenzierung eines zweiten Pferde-Interferon-Genes (EgIPN-omega2) aus Lambda-Klon Eq-a16

Ein 5,5 kb langes EcoRI Restriktionsfragment aus dem Lambda-Klon Eq-a16 (siehe Fig.22), das mit einem humanem a-IFN-Marker schwach hybridisiert hatte (siehe Beispiel D ,E), wurde in die EcoRl-Stelle des Plasmides pUC8 subkloniert und E.coli JM101 mit dem Ligationsgemisch transformiert. Ein erhaltenes Plasmid mit dem gewünschten DNA-Insert wurde pRH62 benannt. Das EcoRl-Insert des Plasmides pRH62 wurde aus einem Agarosegel isoliert, und wie in Beispiel G beschrieben nach dem Shotgun-Verfahren in M13mp8 subkloniert. Die nach Transformation von E.coli JM101 erhaltenen M13 Phagen-Plaques wurden nach dem Verfahren von Benton und Davis (19) auf Nitrocellulose-Filter Übertragen (Beispiel D) und hybridisiert. Als Hybridisierungsprobe wurde das 1,0 kb lange Hindlll Fragment des Plasmides pRH61 eingesetzt, das die gesamte codierende Sequenz für EqlFN-omegal enthält. Von rekomoinanten M13-Phagen, die ein positives Hybridisierungssignal ergaben, wurde Einzelstrang-DNA isoliert und nach der Sanger-Methode sequenziert.

### T) Subklonierung und Sequenzierung von zwei weiteren Pferde-alpha-Interferon-Genen (EqIFN-a3, EqIFN- a4)

Ein 3,2 kb langes Hindlll Restriktionsfragment aus dem Lambda-Klon Eq-a24 das mit einem humanen a-IFN-Marker hybridisiert hatte (siehe Beispiel D ,E), wurde in die Hindlll-Stelle des Plasmides pUC8 subkloniert und E.coli JM101 transformiert. Ein erhaltenes Plasmid mit dem gewünschten Insert wurde pRH83 benannt. Auf die gleiche Weise wurde ein 2,8 kb langes Hindlll Restriktionsfragment aus dem Lambda-Klon Eq-a9 in pUC8 geklont und das erhaltene Plasmid pRH82 benannt. Die Hindlll Inserts der Plasmide pRH82 und pRH83 wurden nach dem Shotgun-Verfahren (Beispiel G) in M13mp8 subkloniert und E.coli JM101 transformiert. Die erhaltenen Phagen-Plaques wurden wie zuvor beschrieben nach der Benton und Davis Methode hybridisiert. Von Phagen, die mit dem 1,0 kb langen Hindlll-BamHl Fragment aus Plasmid pAH52/2 (Beispiel L), welches die codierende Sequenz für reifes EqIFN-a1 enthält, hybridisier- ten,.wurde Einzelstrang-DNA isoliert und nach der Sanger-Methode sequenziert.

### LITERATURVERZEICHNIS:

( 1) D.W.Leung, D.J.Capon, D.V.Goeddel The structure and bacterial expression of three distinct bovine interferon-beta genes. Gene cloning transmission and expression in Escherichia coli Bio/Technology (1984) 2,5,458-464
( 2) D.J.Capon, D.V.Goeddel, GENENTECH Tierische Interferone Offenlegungsschrift DE 33 08 030 A1, 7.3.83
( 3) A.M.Frischauf, H.Lehrach, P.Poustka, N.Murray Lambda replacement vectors carrying polylinker sequences J. Mol. Biol., (1983), 170,827-842
(4) H.R.Rackwitz, G.Zehetner. A.M. Frischauf, H. Lehrach A protocoll for rapid restriction mapping of sequences cloned into lambda vectors Gene (1984). 30. 195-200
( 5) D.Skup et al. Molecular cloning of partial cDNA copies of two distinct mouse IFN beta-mRNAs Nucl. Acids Res. (1982),10,10,3069-3084
( 6) Y.Higashi et al. Structure and expression of a cloned cDNA for Mouse Interferon-beta J. Biol. Chem. (1983),258,9522-9529
( 7) V.Wilson. A.J.Jeffreys; P.A.Barree, P.G.Boseley, P.M.Slocombe, A.Easton, D.C.Burke A comparison of vertebrate interferon gene families detected by hybridization with human interferon DNA J. Mol. Biol. (1983) 166, 457-475
( 8) S.C.Tsai, M.J.Appel Hyporesponsiveness to dog interferon induction in vitro J. gen, Virol. (1983),64,2007-2012
( 9) R.Dijkema, P.Pouwels, A.de Reus, H.Schellekens Structure and expression in Escherichia coli of a rat interferon-alpha gene Nucl. Acids Res., (1984),12,2,1227-1242
(10) G.D.Shaw, W.Boll, H.Taira, N.Mantei, P.Lengyel, C.Weissmann Structure and expression of cloned murine IFN-alpha genes Nucl. Acids Res., (1983),11,3,555-573
(11) H.Bielefeldt Ohmann, L.A.Babiuk Effect of bovine recombinant alpha-1 interferon on inflammatory responses of bovine phagocytes J. Interferon Res., (1984),4,2,249-263
(12) H.Bielefeldt Ohmann, J.E.Gilchrist, L.A.Babiuk Effect of recombinant DNA-produced bovine interferon alpha (BoIFN-_1) on the interaction between bovine alveolar macrophages and bovine Herpesvirus type 1 J. gen. Virol., (1984),65,1487-1495
(13) Y.Higashi; Y.Sokawa,Y.Watanabe, Y.Kawade, S.Ohno, C.Takaoka, T.Taniguchi Structure and expression of a cloned cDNA for mouse interferon-beta J. biol. Chem., (1983),258,15,9522-9529
(14) E.Dworkin-Rast1, P.Swetly, M.B.Dworkin Construction of expression plasmids producing high levels of human leukocyte-type interferon in Escherichia coli Gene, (1983),237-248
(15) E.Dworkin-Rastl, M.B.Dworkin, P.Swetly Molecular cloning of human alpha and beta interferon genes from Namalwa cells J. Interferon Res., (1982),2,4,575-585
(16) D.V.Goeddel, D.W.Leung, T.J.Dull, M.Gross, R.M.Lawn, R.McCandliss: P.H.Seeburg, A.Ullrich, E.Yelverton, P.W.Gray The structure of eight distinct cloned human leukocyte interferor cDNAs Nature, (1981),290,20-26
(17) E.M.Southern Detection of specific sequences among DNA fragments separated by gel electrophoreses J. Mol. Biol., (1975),98,503-
(18) Blin, N. and Stafford, D.W. A general method for isolation of high molecular weight DNA from eukaryotes Nucl.Acids Res. (1976), 3, 2303-2308
(19) W.D.Benton, R.W.Davies Screening lambda gt recombinant clones by hybridization to single plaques in situ Science, (1977),196,180-182
(20) Miller (1972) Experiments in Molecular Genetics, Cold Spring Harbor Laboratory, Cold Spring Harbor, New York
(21) W.E.Stewart 11 (1979) The Interferon System, Springer-Verlag, New York
(22) J.Messing et al. Gene, (1982),19,269-276
(23) F.Sanger, S.Nicklen, A.R.Coulson DNA sequencing with chain-terminating inhibitors Proc. Natl. Acad. Sci. USA, (1977),74,5463-5467
(24) R.Staden Automation of the computer handling of gel reading data produced by the shotgun method of DNA sequencing Nucl. Acids Res., (1982),10,4731-4751
(25) T.Maniatis, E.F.Fritsch, J.Sambrook Molecular Cloning, Cold Spring Harbor, New York
(26) Birnboim and Doly Nucl. Acids Res., (1979),7,1513
(27) Scalenghe, F., Turco, E., Edström, J.E., Pirotta, V. and Melli, M. Microdissection and cloning of DNA from a specific region of Orosophila melanogaster polytene chromosomes Chromosoma (1981), 82, 205-216
(28) K.Todokoro, D.Kioussis, C.Weissmann Two non-allelic human interferon alpha genes with identica codin regions EMBO J., (1984),3,8,1809-1812
(29) B.Hohn, K.Murray Packaging recombinant DNA molekules into bacteriophage particles in vitro Proc. Natl. Acad. Sci. USA: (1977),74,3259-3263
(30) Hohn, B. In vitro packaging of lambda and cosmid DNA Meth.Enzymology (1979), 68, 299-309
(31) J.M.Messing, R.Crea, P.H.Seeburg A system for shotgun sequencing Nucl. Acids Res., (1981),9,309-321
(32) Tovey, M.G., Bandu, M.T., Begon-Lours, J., Brouty-Boye, D. and Gresser, I. Antiviral activity of bovine interferons on primate cells J.Gen.Virol. (1977), 36, 341-344
(33) Hauptmann, R. and Swetly, P. A novel class of human type I interferons Nucl.Acids Res. (1985), 13, 4739-4749
(34) Capon, D.J., Shepard, H.M. and Goeddel, D.V. Two distinct families of human and bovine interferon-alpha genes are coordinately expressed and encode functional polypeptides Mol.Cell.Biol. (1985), 5, 768-779
(35) Feinstein, S.I., Mory, Y., Chernejovsky, Y., Maroteaux, L., Nir, U., Lavie, V. and Revel, M. Family of human alpha-interferon-like sequences Mol.Cell.Biol. (1985), 5, 510-517
(36) Twigg, A.J. and Sherratt, D.J. Trans-complementable copy-number mutants of plasmid ColE1 Nature (1980), 283, 216-218
(37) Sancar, A., Hack, A.M. and Rupp, W.D. Simple method for identification of plasmid-coded proteins J.Bacteriol. (1979), 137, 692-693
(38) Velan, B., Cohen, S., Grosfeld, H., Leitner, M. and Shafferman, A. Bovine interferon alpha genes. Structure and expression J.Biol.Chem. (1985), 260, 5498-5504
(39) Zwarthoff, E.C., Mooren, T.A. and Tropman, J. Organization, structure and expression of murine interferon alpha genes Nucl.Acids Res. (1985), 13, 791-803

## Patentansprüche

1. Rekombinantes DNA-Molekül, das eine für EqlFN-alpha,-beta oder -omega kodierende Sequenz enthält, dadurch gekennzeichnet, daß die kodierende Sequenz ausgewählt ist aus
a) den Inserts der Plasmide pAH50 (Fig.4), pRH63 (Fig.10), pRH82 (Fig.27), pRH83 (Fig.26), pRH61 (Fig.12), pRH62 (Fig.23) oder pAH60 (Fig.8) und
b) DNA-Sequenzen, die unter stringenten Bedingungen die eine Homologie größer als 85%, vorzugsweise größer als 90% erkennen lassen, mit den Inserts der Plasmide pAH50, pRH63, pRH83 pRH61, pRH62 oder pAH60 hybridisieren, und für ein Protein mit dem Aktivitätsspektrum des EqIFN-alpha, -beta oder -omega kodieren.

2. Rekombinantes DNA-Molekül nach Anspruch 1, dadurch gekennzeichnet, daß die DNA-Sequenz aus folgenden Sequenzen ausgewählt ist: 
TGT GAC CTG CCT CAC ACC CAT AGC CTG GGC AAC ACA AGG GTC TTG
ATG CTC CTG GGG CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCT GCG GTC CAT GAG ACG
ATC CAA CAG ATC TTC CAC CTC TTC AGC ACA GAC GGC TCG TCT GCC
GCC TGG GAC GAG AGC CTC CTA GAC AAA CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
 
AGG AGA TAC TTC CAA AGA ATC GCT CTC TAT CTG CAA GAG AAG AAA

TAC AGC CCT TGT GCC TGG GAG ATC GTC AGA GCA GAA ATC ATG AGA

TCC TTC TCT TCA TCC ACA AAC TTG CCG CAG AGT TAA,

TGT GAC CTG CCT CAC ACC CAT AGC CTG GGC AAC ACA AGG GTC TTG

ATG CTC CTG GGA CAA ATG AGG AGA ATC TCC CCC TTC TCC TGC CTG

AAG GAC AGA AAT GAC TTT GGA TTC CCC CAG GAG GTG TTT GAC GGC

AAC CAG TTC CGG AAG CCT CAA GCC ATC TCC GCG GTC CAT GAG ACG

ATC CAA CAG ATC TTC CAC CTC TTC AGC ACA GAC GGC TCG TCT GCT

GCC TGG GAC GAG AGC CTC CTA GAC AAG CTC TAC ACT GGA CTC TAT

CAG CAG CTG ACT GAG CTG GAA GCC TGT CTG AGC CAG GAG GTG GGG

GTG GAA GAG ACG CCC CTG ATG AAC GAG GAC TCC CTG CTG GCT GTG

AGG AGA TAC TTC CAA AGA ATC GCT CTC TAT CTG CAA GAG AAG AAA

TAC AGC CCT TGT GCC TGG GAG ATC GTC AGA GCA GAA ATC ATG AGA

TGC TTC TCT TCA TCC ACA AAC TTG CAG CAG AGT TAA,

TGT GAC CTG CCT CAC ACC CAT AGC CTG GGC AAC ACA AGG GTC TTG

ATG CTC CTG GGA CAA ATG AGG AGA ATC TCC CCC TTC TCC TGC CTG

AAG GAC AGA AAT GAC TTT GGA TTC CCC CAG GAG GTG TTT GAC GGC

AAC CAG TTC CGG AAG CCT CAA GCC ATC TCT GCG GTC CAT GAG ACG

ATC CAA CAG ATC TTC CAC CTC TTC AGC ACA GAC GGC TCG TCT GCT
 
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCC GCG GTC CAT GAG ACG
ATC CAA CAG ATC TTC CAC CTC TTC AGC ACA GAC GGC TCG TCT GCC
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC ACT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAA AGT TAA,
TGC GAC CTG CCT GCG AGC CTT GAC TTG AGA AAG CAG GAG ACC CTC
AGA GTT CTG CAC CAG ATG GAG ACA ATC TCT CCT CCT TCC TGT CTG
 
AAG CAC AGG ACA GAC TTC AGG TTC CCC CAG GAG CAG CTG GAT GGC

AGG CAG TTC CCA GAG GCC CAG GCC ACG TCT GTC CTC CAG GAG ATG

CTC CAG CAG ATC GTC AGC CTC TTC CAC ACA GAG CGC TCG TCT GCT

GCC TGG AAC ACG ACT CTG CTG GAC CGA CTC CTC GCG GGA CTC CAT

CAG CAG CTG GAA GAC CTC AAC ACC TGC TTG GAT GAG CAG ACA GGA

GAG GAA GAA TCC GCC CTG GGA ACT GTG GGC CCT ACA CTG GCC GTG

AAG AGG TAC TTC AGG AGA ATC CGT CTG TAC CTG ACA GAG AAG AAA

TAC AGT GAC TGT GCC TGG GAG ATT GTC AGA GTG GAC ATC ATG AGA

TCC TTC TCT TCA TCA GCA AAC CTG CAA GGA AGG TTA GGA ATG AAG

GAT GGA GAC CTG GGG TCA CCT TGA,

TGT GAC CTG CCT CAG AAC CAC ATC CTG GTT AGC AGG AAG AAC TTC

GTG CTT CTG GGC CAA ATG AGC AGA ATC TCC TCC GCA ATC TGT CTG

AAG GAC AGA AAA GAC TTC AGG TTC CCC CAG GAC ATG GCG GAT GGC

AGG CAG TTC CCA GAG GCC CAG GCC GCG TCT GTC CTC CAC GAG ATG

CTC CAG CAG ATC TTC AGC CTC TTC CAC ACA GAG CGC TCG TCT GCT

GCC TGG AAC ACG ACC CTC CTG GAC GAA CTC TGC ACG GGA CTC CTT

CGG CAG CTG GAA GAC CTG GAC ACC TGT TTG GAG CAG GAG ATG GGA

GAG GAA GAA TCT GCC CTG GGA ACT GTG CGC CCT ACA CTG GCC GTG

AAG AGG TAC TTC CGG GGG ATC CAT CTC TAC CTG AAA GAG AAG AAA
 
TAC AGT GAC TGT GCC TGG GAG ATT GTC CGA ATG GAA ATC ATG AGA
TCC TTC TCT TCA TCA GCA AAC CTG CAA GGA AGG TTA AGA ATG AAG
GAT GGA GAC CTG GGC TCA CCT TGA oder
GTG AAC TAT GAC TTG CTT CGG TCC CAA CTA AGA AGC AGC AAT TCA
GCA TGT CTG ATG CTC CTG CGG CAG TTG AAT GGA GCC CCT CAA CGT
TGC CCC GAG GAC ACA ATG AAC TTC CAG GTC CCT GAG GAG ATT GAG
CAA GCA CAG CAG TTC CAG AAG GAG GAT GCT GCA TTG GTC ATC TAT
GAG ATG CTC CAG CAC ACC TGG CGT ATT TTC AGA AGA AAT TTC GCT
AGC ACT GGC TGG AAT GAG ACC ATC GTT AAG AAC CTC CTT GTG GAA
GTC CAT CTG CAG ATG GAC CGT CTG GAG ACA AAC CTG GAG GAA ATA
ATG GAG GAG GAA AGC TCC ACC TGG GGA AAC ACA ACC ATT CTG CGC
CTG AAG AAA TAC TAC GGA AGG ATC TCG CAG TAC CTG AAG GCC AAG
AAG TAC AGC CAC TGT GCC TGG ACA GTG GTC CAA GCG GAA ATG CTC
AGG AAC TTG GCC TTC CTT AAC GGA CTC ACA GAT TAC CTC CAA AAC
TGA,
 sowie deren degenerierte Varianten

3. Rekombinantes DNA-Molekül zur Expression einer DNA-Sequenz in Mikroorganismen, dadurch gekennzeichnet, daß man in einen Expressionsvektor, der in Prokaryoten, Eukaryoten oder in Säugetierzellen replizierbar ist, eine DNA-Sequenz nach einem der Ansprüche 1 oder 2 einbringt.

4. Rekombinantes DNA-Molekül nach Anspruch 3, dadurch gekennzeichnet, daß es das Expressionsplasmid pAH52, pAH52/2, pAH53, pAH55 oder pAH62 ist.

5. Transformierter Wirtsorganismus, der die für EqIFN-alpha, -beta oder -omega codierende Sequenz nach einem der vorhergehenden Ansprüche enthält.

6. Transformierter Wirtsorganismus nach Anspruch 5, dadurch gekennzeichnet, daß er ein Prokaryot oder ein Eukaryot, vorzugsweise E.coli oder Saccaromyces cerevisiae oder eine Säugetierzelle ist.

7. Transformierter Wirtsorganismus nach Anspruch 5 oder 6, dadurch gekennzeichnet, daß die genetische Sequenz in einem in dem Wirtsorganismus replizierbaren, das Protein exprimierenden Vektor, vorzugsweise in einem der Plasmide gemäß Anspruch 3 oder 4 enthalten ist.

8. Interferon, dadurch gekennzeichnet, daß es ein EqIFN-alpha, -beta oder -omega, codiert durch eine DNA-Sequenz gemäß einem der Ansprüche 1 oder 2 ist, im wesentlichen frei von anderen Proteinen tierischer Herkunft, vorzugsweise in im wesentlichen reiner Form vorliegt.

9. Interferon nach Anspruch 8, dadurch gekennzeichnet, daß es frei von nativer Glykosylierung vorliegt.

10. Interferon nach Anspruch 8 oder 9, dadurch gekennzeichnet, daß es der Aminosäuresequenz gemäß einer der Formeln 
Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly
 
Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Ala Leu Tyr Leu Gln Glu Lys Lys

Tyr Ser Pro Cys Ala Trp Glu Ile Val Arg Ala Glu Ile Met Arg

Ser Phe Ser Ser Ser Thr Asn Leu Pro Gln Ser,

Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly

Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Ala Leu Tyr Leu Gln Glu Lys Lys

Tyr Ser Pro Cys Ala Trp Glu Ile Val Arg Ala Glu Ile Met Arg

Cys Phe Ser Ser Ser Thr Asn Leu Gln Gln Ser,
 
Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly

Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Thr Leu Tyr Leu Gln Glu Lys Lys

Tyr Ser Pro Cys Ala Trp Glu Ile Val Arg Ala Glu Ile Met Arg

Ser Phe Ser Ser Ser Thr Asn Leu Pro Gln Ser,

Cys Asp Leu Pro Ala Ser Leu Asp Leu Arg Lys Gln Glu Thr Leu

Arg Val Leu His Gln Met Glu Thr Ile Ser Pro Pro Ser Cys Leu

Lys His Arg Thr Asp Phe Arg Phe Pro Gln Glu Gln Leu Asp Gly

Arg Gln Phe Pro Glu Ala Gln Ala Thr Ser Val Leu Gln Glu Met

Leu Gln Gln Ile Val Ser Leu Phe His Thr Glu Arg Ser Ser Ala

Ala Trp Asn Thr Thr Leu Leu Asp Arg Leu Leu Ala Gly Leu His

Gln Gln Leu Glu Asp Leu Asn Thr Cys Leu Asp Glu Gln Thr Gly

Glu Glu Glu Ser Ala Leu Gly Thr Val Gly Pro Thr Leu Ala Val
 
Lys Arg Tyr Phe Arg Arg Ile Arg Leu Tyr Leu Thr Glu Lys Lys

Tyr Ser Asp Cys Ala Trp Glu Ile Val Arg Val Asp Ile Met Arg

Ser Phe Ser Ser Ser Ala Asn Leu Gln Gly Arg Leu Gly Met Lys

Asp Gly Asp Leu Gly Ser Pro oder

Cys Asp Leu Pro Gln Asn His Ile Leu Val Ser Arg Lys Asn Phe

Val Leu Leu Gly Gln Met Ser Arg Ile Ser Ser Ala Ile Cys Leu

Lys Asp Arg Lys Asp Phe Arg Phe Pro Gln Asp Met Ala Asp Gly

Arg Gln Phe Pro Glu Ala Gln Ala Ala Ser Val Leu His Glu Met

Leu Gln Gln Ile Phe Ser Leu Phe His Thr Glu Arg Ser Ser Ala

Ala Trp Asn Thr Thr Leu Leu Asp Glu Leu Cys Thr Gly Leu Leu

Arg Gln Leu Glu Asp Leu Asp Thr Cys Leu Glu Gln Glu Met Gly

Glu Glu Glu Ser Ala Leu Gly Thr Val Arg Pro Thr Leu Ala Val

Lys Arg Tyr Phe Arg Gly Ile His Leu Tyr Leu Lys Glu Lys Lys

Tyr Ser Asp Cys Ala Trp Glu Ile Val Arg Met Glu Ile Met Arg

Ser Phe Ser Ser Ser Ala Asn Leu Gln Gly Arg Leu Arg Met Lys

Asp Gly Asp Leu Gly Ser Pro
 oder biologisch aktiven Varianten dieser Sequenzen, mit dem Aktivitätssprektrum des EqIFN-alpha, -beta oder -omega entspricht.

11. Verfahren zur Herstellung von EqIFN-alpha, -beta oder -omega, dadurch gekennzeichnet, daß
a) ein Wirtsorganismus, vorzugsweise ein Wirtsorganismus nach einem der Ansprüche 5 bis 7 mit genetischen Informationen gemäß einem der Ansprüche 1 bis 3 transformiert.
b) die codierende Sequenz in einem Expressionsvektor, vorzugsweise in einem der Expressionsvektoren gemäß Anspruch 3 oder 4 enthalten ist und diese Information im Wirtsorganismus esprimiert und
c) das Interferon EqIFN-alpha, -beta oder -omega gemäß einem der Ansprüche 8 bis 10 vorzugsweise ein Interferon gemäß Anspruch 11 isoliert und gereinigt wird.

12. Verwendung der Interferone gemäß einem der Ansprüche 8 bis 10 zur Herstellung von Arzneimitteln.

13. Mittel für die therapeutische Behandlung, dadurch gekennzeichnet, daß es neben pharmazeutisch inerten Trägerstoffen eine wirksame Menge an einem Interferon gemäß einem der Ansprüche 8 bis 10 enthält.

14. EqIFN-alpha, -beta oder -omega gemäß einem der Ansprüche 8 bis 10 zur Verwendung als Arzneimittel.

## Claims

1. Recombinant DNA-molecule which contains a sequence coding for EqIFN-alpha, -beta or -omega, characterised in that the coding sequence is selected from
a) the inserts of the plasmids pAH50 (Fig.4), pRH63 (Fig.10), pRH82 (Fig.27), pRH83 (Fig.26), pRH61 (Fig.12), pRH62 (Fig.23) or pAH60 (Fig.8) and
b) DNA-sequences which hybridise with the inserts of the plasmids pAH50, pRH63, pRH83, pRH61, pRH62 or pAH60 under stringent conditions which show a homology of more than 85% and preferably more than 90%, and which code for a protein with the activity spectrum of EqIFN-alpha, -beta or -omega.

2. Recombinant DNA-molecule according to claim 1, characterised in that the DNA-sequence is selected from the following sequences: 
TGT GAC CTG CCT CAC ACC CAT AGC CTG GGC AAC ACA AGG GTC TTG

ATG CTC CTG GGG CAA ATG AGG AGA ATC TCC CCC TTC TCC TGC CTG

AAG GAC AGA AAT GAC TTT GGA TTC CCC CAG GAG GTG TTT GAC GGC

AAC CAG TTC CGG AAG CCT CAA GCC ATC TCT GCG GTC CAT GAG ACG

ATC CAA CAG ATC TTC CAC CTC TTC AGC ACA GAC GGC TCG TCT GCC

GCC TGG GAC GAG AGC CTC CTA GAC AAA CTC TAC ACT GGA CTC TAT

CAG CAG CTG ACT GAG CTG GAA GCC TGT CTG AGC CAG GAG GTG GGG

GTG GAA GAG ACG CCC CTG ATG AAC GAG GAC TCC CTG CTG GCT GTG
 
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCC GCG GTC CAT GAG ACG
ATC CAA CAG ATC TTC CAC CTC TTC AGC ACA GAC GGC TCG TCT GCT
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TGC TTC TCT TCA TCC ACA AAC TTG CAG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCT GCG GTC CAT GAG ACG
ATC CAA CAG ATC TTC CAC CTC TTC AGC ACA GAC GGC TCG TCT GCT
 
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCC GCG GTC CAT GAG ACG
ATC CAA CAG ATC TTC CAC CTC TTC AGC ACA GAC GGC TCG TCT GCC
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC ACT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAA AGT TAA;
TGC GAC CTG CCT GCG AGC CTT GAC TTG AGA AAG CAG GAG ACC CTC
AGA GTT CTG CAC CAG ATG GAG ACA ATC TCT CCT CCT TCC TGT CTG
 
AAG CAC AGG ACA GAC TTC AGG TTC CCC CAG GAG CAG CTG GAT GGC

AGG CAG TTC CCA GAG GCC CAG GCC ACG TCT GTC CTC CAG GAG ATG

CTC CAG CAG ATC GTC AGC CTC TTC CAC ACA GAG CGC TCG TCT GCT

GCC TGG AAC ACG ACT CTG CTG GAC CGA CTC CTC GCG GGA CTC CAT

CAG CAG CTG GAA GAC CTC AAC ACC TGC TTG GAT GAG CAG ACA GGA

GAG GAA GAA TCC GCC CTG GGA ACT GTG GGC CCT ACA CTG GCC GTG

AAG AGG TAC TTC AGG AGA ATC CGT CTG TAC CTG ACA GAG AAG AAA

TAC AGT GAC TGT GCC TGG GAG ATT GTC AGA GTG GAC ATC ATG AGA

TCC TTC TCT TCA TCA GCA AAC CTG CAA GGA AGG TTA GGA ATG AAG

GAT GGA GAC CTG GGG TCA CCT TGA,

TGT GAC CTG CCT CAG AAC CAC ATC CTG GTT AGC AGG AAG AAC TTC

GTG CTT CTG GGC CAA ATG AGC AGA ATC TCC TCC GCA ATC TGT CTG

AAG GAC AGA AAA GAC TTC AGG TTC CCC CAG GAC ATG GCG GAT GGC

AGG CAG TTC CCA GAG GCC CAG GCC GCG TCT GTC CTC CAC GAG ATG

CTC CAG CAG ATC TTC AGC CTC TTC CAC ACA GAG CGC TCG TCT GCT

GCC TGG AAC ACG ACC CTC CTG GAC GAA CTC TGC ACG GGA CTC CTT

CGG CAG CTG GAA GAC CTG GAC ACC TGT TTG GAG CAG GAG ATG GGA

GAG GAA GAA TCT GCC CTG GGA ACT GTG CGC CCT ACA CTG GCC GTG

AAG AGG TAC TTC CGG GGG ATC CAT CTC TAC CTG AAA GAG AAG AAA
 
TAC AGT GAC TGT GCC TGG GAG ATT GTC CGA ATG GAA ATC ATG AGA

TCC TTC TCT TCA TCA GCA AAC CTG CAA GGA AGG TTA AGA ATG AAG

GAT GGA GAC CTG GGC TCA CCT TGA or

GTG AAC TAT GAC TTG CTT CGG TCC CAA CTA AGA AGC AGC AAT TCA

GCA TGT CTG ATG CTC CTG CGG CAG TTG AAT GGA GCC CCT CAA CGT

TGC CCC GAG GAC ACA ATG AAC TTC CAG GTC CCT GAG GAG ATT GAG

CAA GCA CAG CAG TTC CAG AAG GAG GAT GCT GCA TTG GTC ATC TAT

GAG ATG CTC CAG CAC ACC TGG CGT ATT TTC AGA AGA AAT TTC GCT

AGC ACT GGC TGG AAT GAG ACC ATC GTT AAG AAC CTC CTT GTG GAA

GTC CAT CTG CAG ATG GAC CGT CTG GAG ACA AAC CTG GAG GAA ATA

ATG GAG GAG GAA AGC TCC ACC TGG GGA AAC ACA ACC ATT CTG CGC

CTG AAG AAA TAC TAC GGA AGG ATC TCG CAG TAC CTG AAG GCC AAG

AAG TAC AGC CAC TGT GCC TGG ACA GTG GTC CAA GCG GAA ATG CTC

AGG AAC TTG GCC TTC CTT AAC GGA CTC ACA GAT TAC CTC CAA AAC

TGA,
 and the degenerate variants thereof.

3. Recombinant DNA-molecule for expressing a DNA sequence in microorganisms, characterised in that a DNA-sequence according to one of claims 1 and 2 is introduced into an expression vector which is replicable in prokaryotes, eukaroytes or in mammalian cells.

4. Recombinant DNA-molecule according to claim 3, characterised in that it is the expression plasmid pAH52, pAH52/2, pAH53, pAH55 or pAH62.

5. Transformed host organism which contains the sequence coding for EqIFN-alpha, -beta or -omega according to one of the preceding claims.

6. Transformed host organism according to claim 5, characterised in that it is a prokaryote or a eukaryote, preferably E. coli or Saccharomyces cerevisiae or a mammalian cell.

7. Transformed host organism according to claim 5 or 6, characterised in that the genetic sequence is contained in a vector which is replicable in the host organism and expresses the protein, preferably in one of the plasmids according to claim 3 or 4.

8. Interferon, characterised in that it is an EqIFN-alpha, -beta or -omega coded by a DNA-sequence according to one of claims 1 or 2, substantially free from other proteins of animal origin, and preferably present in substantially pure form.

9. Interferon according to claim 8, characterised in that it is free from native glycosylation.

10. Interferon according to claim 8 or 9, characterised in that it corresponds to the amino acid sequence according to one of the formulae 
Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly
 
Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Ala Leu Tyr Leu Gln Glu Lys Lys

Tyr Ser Pro Cys Ala Trp Glu Ile Val Arg Ala Glu Ile Met Arg

Ser Phe Ser Ser Ser Thr Asn Leu Pro Gln Ser,

Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly

Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Ala Leu Tyr Leu Gln Glu Lys Lys

Tyr Ser Pro Cys Ala Trp Glu Ile Val Arg Ala Glu Ile Met Arg

Cys Phe Ser Ser Ser Thr Asn Leu Gln Gln Ser,
 
Cys Asp Leu Pro His Thr His Ser Leu Gly Asn Thr Arg Val Leu

Met Leu Leu Gly Gln Met Arg Arg Ile Ser Pro Phe Ser Cys Leu

Lys Asp Arg Asn Asp Phe Gly Phe Pro Gln Glu Val Phe Asp Gly

Asn Gln Phe Arg Lys Pro Gln Ala Ile Ser Ala Val His Glu Thr

Ile Gln Gln Ile Phe His Leu Phe Ser Thr Asp Gly Ser Ser Ala

Ala Trp Asp Glu Ser Leu Leu Asp Lys Leu Tyr Thr Gly Leu Tyr

Gln Gln Leu Thr Glu Leu Glu Ala Cys Leu Ser Gln Glu Val Gly

Val Glu Glu Thr Pro Leu Met Asn Glu Asp Ser Leu Leu Ala Val

Arg Arg Tyr Phe Gln Arg Ile Thr Leu Tyr Leu Gln Glu Lys Lys

Tyr Ser Pro Cys Ala Trp Glu Ile Val Arg Ala Glu Ile Met Arg

Ser Phe Ser Ser Ser Thr Asn Leu Pro Gln Ser,

Cys Asp Leu Pro Ala Ser Leu Asp Leu Arg Lys Gln Glu Thr Leu

Arg Val Leu His Gln Met Glu Thr Ile Ser Pro Pro Ser Cys Leu

Lys His Arg Thr Asp Phe Arg Phe Pro Gln Glu Gln Leu Asp Gly

Arg Gln Phe Pro Glu Ala Gln Ala Thr Ser Val Leu Gln Glu Met

Leu Gln Gln Ile Val Ser Leu Phe His Thr Glu Arg Ser Ser Ala

Ala Trp Asn Thr Thr Leu Leu Asp Arg Leu Leu Ala Gly Leu His

Gln Gln Leu Glu Asp Leu Asn Thr Cys Leu Asp Glu Gln Thr Gly

Glu Glu Glu Ser Ala Leu Gly Thr Val Gly Pro Thr Leu Ala Val
 
Lys Arg Tyr Phe Arg Arg Ile Arg Leu Tyr Leu Thr Glu Lys Lys

Tyr Ser Asp Cys Ala Trp Glu Ile Val Arg Val Asp Ile Met Arg

Ser Phe Ser Ser Ser Ala Asn Leu Gln Gly Arg Leu Gly Met Lys

Asp Gly Asp Leu Gly Ser Pro or

Cys Asp Leu Pro Gln Asn His Ile Leu Val Ser Arg Lys Asn Phe

Val Leu Leu Gly Gln Met Ser Arg Ile Ser Ser Ala Ile Cys Leu

Lys Asp Arg Lys Asp Phe Arg Phe Pro Gln Asp Met Ala Asp Gly

Arg Gln Phe Pro Glu Ala Gln Ala Ala Ser Val Leu His Glu Met

Leu Gln Gln Ile Phe Ser Leu Phe His Thr Glu Arg Ser Ser Ala

Ala Trp Asn Thr Thr Leu Leu Asp Glu Leu Cys Thr Gly Leu Leu

Arg Gln Leu Glu Asp Leu Asp Thr Cys Leu Glu Gln Glu Met Gly

Glu Glu Glu Ser Ala Leu Gly Thr Val Arg Pro Thr Leu Ala Val

Lys Arg Tyr Phe Arg Gly Ile His Leu Tyr Leu Lys Glu Lys Lys

Tyr Ser Asp Cys Ala Trp Glu Ile Val Arg Met Glu Ile Met Arg

Ser Phe Ser Ser Ser Ala Asn Leu Gln Gly Arg Leu Arg Met Lys

Asp Gly Asp Leu Gly Ser Pro
 or biologically active variants of these sequences, with the activity spectrum of EqIFN-alpha, -beta or -omega.

11. Process for preparing EqIFN-alpha, -beta or -omega, characterised in that
a) a host organism, preferably a host organism according to one of claims 5 to 7, is transformed with genetic information according to one of claims 1 to 3,
b) the coding sequence is contained in an expression vector, preferably in one of the expression vectors according to claim 3 or 4 and this information is expressed in the host organism and
c) the interferon EqIFN-alpha, -beta or -omega according to one of claims 8 to 10, preferably an interferon according to claim 11, is isolated and purified.

12. Use of the interferons according to one of claims 8 to 10 for preparing pharmaceutical compositions.

13. Agent for therapeutic treatment, characterised in that it contains, in addition to pharmaceutically inert carriers, an effective amount of an interferon according to one of claims 8 to 10.

14. EqIFN-alpha, -beta or -omega according to one of claims 8 to 10 for use as a pharmaceutical composition.

## Revendications

1. Molécule d'ADN recombinée qui contient une séquence codant pour EqlFN alpha, bêta ou oméga, caractérisée en ce que la séquence codante est choisie parmi
a) les inserts des plasmides pAH50 (fig.4), pRH63 (fig.10), pRH82 (fig.27), pRH83 (fig.26), pRH61 (fig.12), pRH62 (fig.23) ou pAH60 (fig.8) et
b) des séquences d'ADN qui, dans des conditions strictes qui permettent d'identifier une homologie supérieure à 85%, de préférence supérieure à 90%, s'hybrident avec les inserts des plasmides pAH50, pRH63, pRH83, pRH61, pRH62 ou pAH60, et qui codent pour une protéine présentant le spectre d'activité de EqIFN alpha, bêta ou oméga.

2. Molécule d'ADN recombinée selon la revendication 1, caractérisée en ce que la séquence d'ADN est choisie parmi les séquences suivantes: 
TGT GAC CTG CCT CAC ACC CAT AGC CTG GGC AAC ACA AGG GTC TTG
ATG CTC CTG GGG CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCT GCG GCC CAT GAG ACG
ATC CAA CAG ATC TTC CAC CTC TTC AGC ACA GAC GGC TCG TCT GCC
GCC TGG GAC GAG AGC CTC CTA GAC AAA CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCC GCG GTC CAT GAG ACG
ATC CAA CAG ATC TTC CAC CTC TTC AGC ACA GAC GGC TCG TCT GCT
GCC TGG GAC GAG AGC CTC CTA GAC AAG CTC TAC ACT GGA CTC TAT
 
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TGC TTC TCT TCA TCC ACA AAC TTG CAG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCT GCG GTC CAT GAG ACG
ATC CAA CAG ATC TTC CAC CTC TTC AGC ACA GAC GGC TCG TCT GCT
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC GCT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAG AGT TAA,
TGT GAC CTG CCT CAC ACC CAT AGC CTG GGC AAC ACA AGG GTC TTG
ATG CTC CTG GGA CAA ATG AGG AGA ATC TCC CCC TTC TCC TGC CTG
AAG GAC AGA AAT GAC TTT GGA TTC CCC CAG GAG GTG TTT GAC GGC
AAC CAG TTC CGG AAG CCT CAA GCC ATC TCC GCG GTC CAT GAG ACG
ATC CAA CAG ATC TTC CAC CTC TTC AGC ACA GAC GGC TCG TCT GCC
GCC TGG GAC GAG AGC CTC CTA GAC AAG CTC TAC ACT GGA CTC TAT
CAG CAG CTG ACT GAG CTG GAA GCC TGT CTG AGC CAG GAG GTG GGG
GTG GAA GAG ACG CCC CTG ATG AAC GAG GAC TCC CTG CTG GCT GTG
AGG AGA TAC TTC CAA AGA ATC ACT CTC TAT CTG CAA GAG AAG AAA
TAC AGC CCT TGT GCC TGG GAG ATC GTC AGA GCA GAA ATC ATG AGA
TCC TTC TCT TCA TCC ACA AAC TTG CCG CAA AGT TAA,
TGC GAC CTG CCT GCG AGC CTT GAC TTG AGA AAG CAG GAG ACC CTC
AGA GTT CTG CAC CAG ATG GAG ACA ATC TCT CCT CCT TCC TGT CTG
AAG CAC AGG ACA GAC TTC AGG TTC CCC CAG GAG CAG CTG GAT GGC
AGG CAG TTC CCA GAG GCC CAG GCC ACG TCT GTC CTC CAG GAG ATG
CTC CAG CAG ATC GTC AGC CTC TTC CAC ACA GAG CGC TCG TCT GCT
GCC TGG AAC ACG ACT CTG CTG GAC CGA CTC CTC GCG GGA CTC CAT
CAG CAG CTG GAA GAC CTC AAC ACC TGC TTG GAT GAG CAG ACA GGA
 
GAG GAA GAA TCC GCC CTG GGA ACT GTG GGC CCT ACA CTG GCC GTG
AAG AGG TAC TTC AGG AGA ATC CGT CTG TAC CTG ACA GAG AAG AAA
TAC AGT GAC TGT GCC TGG GAG ATT GTC AGA GTG GAC ATC ATG AGA
TCC TTC TCT TCA TCA GCA AAC CTG CAA GGA AGG TTA GGA ATG AAG
GAT GGA GAC CTG GGG TCA CCT TGA,
TGT GAC CTG CCT CAG AAC CAC ATC CTG GTT AGC AGG AAG AAC TTC
GTG CTT CTG GGC CAA ATG AGC AGA ATC TCC TCC GCA ATC TGT CTG
AAG GAC AGA AAA GAC TTC AGG TTC CCC CAG GAC ATG GCG GAT GGC
AGG CAG TTC CCA GAG GCC CAG GCC GCG TCT GTC CTC CAC GAG ATG
CTC CAG CAG ATC TTC AGC CTC TTC CAC ACA GAG CGC TCG TCT GCT
GCC TGG AAC ACG ACC CTC CTG GAC GAA CTC TGC ACG GGA CTC CTT
CGG CAG CTG GAA GAC CTG GAC ACC TGT TTG GAG CAG GAG ATG GGA
GAG GAA GAA TCT GCC CTG GGA ACT GTG CGC CCT ACA CTG GCC GTG
AAG AGG TAC TTC CGG GGG ATC CAT CTC TAC CTG AAA GAG AAG AAA
TAC AGT GAC TGT GCC TGG GAG ATT GTC CGA ATG GAA ATC ATG AGA
TCC TTC TCT TCA TCA GCA AAC CTG CAA GGA AGG TTA AGA ATG AAG
GAT GGA GAC CTG GGC TCA CCT TGA ou
GTG AAC TAT GAC TTG CTT CGG TCC CAA CTA AGA AGC AGC AAT TCA
GCA TGT CTG ATG CTC CTG CGG CAG TTG AAT GGA GCC CCT CAA CGT
TGC CCC GAG GAC ACA ATG AAC TTC CAG GTC CCT GAG GAG ATT GAG
CAA GCA CAG CAG TTC CAG AAG GAG GAT GCT GCA TTG GTC ATC TAT
GAG ATG CTC CAG CAC ACC TGG CGT ATT TTC AGA AGA AAT TTC GCT
AGC ACT GGC TGG AAT GAG ACC ATC GTT AAG AAC CTC CTT GTG GAA
GTC CAT CTG CAG ATG GAC CGT CTG GAG ACA AAC CTG GAG GAA ATA
ATG GAG GAG GAA AGC TCC ACC TGG GGA AAC ACA ACC ATT CTG CGC
CTG AAG AAA TAC TAC GGA AGG ATC TCG CAG TAC CTG AAG GCC AAG
AAG TAC AGC CAC TGT GCC TGG ACA GTG GTC CAA GCG GAA ATG CTC
AGG AAC TTG GCC TTC CTT AAC GGA CTC ACA GAT TAC CTC CAA AAC
TGA,
 ainsi que leurs variantes dégénérées.

3. Molécule d'ADN recombinée pour l'expression d'une séquence d'ADN dans des micro-organismes, caractérisée en ce que l'on introduit dans un vecteur d'expression, qui est réplicable dans les procaryotes, les eucaryotes ou les cellules de mammifères, une séquence d'ADN selon l'une des revendications 1 ou 2.

4. Molécule d'ADN recombinée selon la revendication 3, caractérisée en ce que le plasmide d'expression est pAH52, pAH52/2, pAH53, pAH55 ou pAH62.

5. Organisme hôte transformé, qui contient la séquence codant pour EqlFN alpha, bêta ou oméga selon l'une des revendications précédentes.

6. Organisme hôte transformé selon la revendication 5, caractérisé en ce qu'il s'agit d'un procaryote ou d'un eucaryote, de préférence de E. coli ou de Saccaromyces cerevisiae ou d'une cellule de mammifère.

7. Organisme hôte transformé selon la revendication 5 ou 6, caractérisé en ce que la séquence génétique est contenue dans un vecteur réplicable dans l'organisme hôte, qui exprime la protéine, de préférence dans l'un des plasmides selon la revendication 3 ou 4.

8. Interféron, caractérisé en ce qu'il s'agit d'un EqlFN alpha, bêta ou oméga, codé par une séquence d'ADN selon l'une des revendications 1 ou 2, sensiblement exempt d'autres protéines d'origine animale, de préférence sous forme sensiblement pure.

9. Interféron selon la revendication 8, caractérisé en ce qu'il est exempt de glycosylation native.

10. Interféron selon la revendication 8 ou 9, caractérisé en ce qu'il correspond à la séquence d'acides aminés selon l'une des formules 
Cys Asp Leu Pro His Thr His Ser Leu Gly Asn Thr Arg Val Leu
Met Leu Leu Gly Gln Met Arg Arg Ile Ser Pro Phe Ser Cys Leu
Lys Asp Arg Asn Asp Phe Gly Phe Pro Gln Glu Val Phe Asp Gly
Asn Gln Phe Arg Lys Pro Gln Ala Ile Ser Ala Val His Glu Thr
Ile Gln Gln Ile Phe His Leu Phe Ser Thr Asp Gly Ser Ser Ala
Ala Trp Asp Glu Ser Leu Leu Asp Lys Leu Tyr Thr Gly Leu Tyr
Gln Gln Leu Thr Glu Leu Glu Ala Cys Leu Ser Gln Glu Val Gly
 
Val Glu Glu Thr Pro Leu Met Asn Glu Asp Ser Leu Leu Ala Val
Arg Arg Tyr Phe Gln Arg Ile Ala Leu Tyr Leu Gln Glu Lys Lys
Tyr Ser Pro Cys Ala Trp Glu Ile Val Arg Ala Glu Ile Met Arg
Ser Phe Ser Ser Ser Thr Asn Leu Pro Gln Ser,
Cys Asp Leu Pro His Thr His Ser Leu Gly Asn Thr Arg Val Leu
Met Leu Leu Gly Gln Met Arg Arg Ile Ser Pro Phe Ser Cys Leu
Lys Asp Arg Asn Asp Phe Gly Phe Pro Gln Glu Val Phe Asp Gly
Asn Gln Phe Arg Lys Pro Gln Ala Ile Ser Ala Val His Glu Thr
Ile Gln Gln Ile Phe His Leu Phe Ser Thr Asp Gly Ser Ser Ala
Ala Trp Asp Glu Ser Leu Leu Asp Lys Leu Tyr Thr Gly Leu Tyr
Gln Gln Leu Thr Glu Leu Glu Ala Cys Leu Ser Gln Glu Val Gly
Val Glu Glu Thr Pro Leu Met Asn Glu Asp Ser Leu Leu Ala Val
Arg Arg Tyr Phe Gln Arg Ile Ala Leu Tyr Leu Gln Glu Lys Lys
Tyr Ser Pro Cys Ala Trp Glu Ile Val Arg Ala Glu Ile Met Arg
Cys Phe Ser Ser Ser Thr Asn Leu Gln Gln Ser,
Cys Asp Leu Pro His Thr His Ser Leu Gly Asn Thr Arg Val Leu
Met Leu Leu Gly Gln Met Arg Arg Ile Ser Pro Phe Ser Cys Leu
Lys Asp Arg Asn Asp Phe Gly Phe Pro Gln Glu Val Phe Asp Gly
Asn Gln Phe Arg Lys Pro Gln Ala Ile Ser Ala Val His Glu Thr
Ile Gln Gln Ile Phe His Leu Phe Ser Thr Asp Gly Ser Ser Ala
Ala Trp Asp Glu Ser Leu Leu Asp Lys Leu Tyr Thr Gly Leu Tyr
Gln Gln Leu Thr Glu Leu Glu Ala Cys Leu Ser Gln Glu Val Gly
Val Glu Glu Thr Pro Leu Met Asn Glu Asp Ser Leu Leu Ala Val
Arg Arg Tyr Phe Gln Arg Ile Thr Leu Tyr Leu Gln Glu Lys Lys
Tyr Ser Pro Cys Ala Trp Glu Ile Val Arg Ala Glu Ile Met Arg
Ser Phe Ser Ser Ser Thr Asn Leu Pro Gln Ser,
Cys Asp Leu Pro Ala Ser Leu Asp Leu Arg Lys Gln Glu Thr Leu
Arg Val Leu His Gln Met Glu Thr Ile Ser Pro Pro Ser Cys Leu
Lys His Arg Thr Asp Phe Arg Phe Pro Gln Glu Gln Leu Asp Gly
Arg Gln Phe Pro Glu Ala Gln Ala Thr Ser Val Leu Gln Glu Met
Leu Gln Gln Ile Val Ser Leu Phe His Thr Glu Arg Ser Ser Ala
Ala Trp Asn Thr Thr Leu Leu Asp Arg Leu Leu Ala Gly Leu His
Gln Gln Leu Glu Asp Leu Asn Thr Cys Leu Asp Glu Gln Thr Gly
Glu Glu Glu Ser Ala Leu Gly Thr Val Gly Pro Thr Leu Ala Val
 
Lys Arg Tyr Phe Arg Arg Ile Arg Leu Tyr Leu Thr Glu Lys Lys
Tyr Ser Asp Cys Ala Trp Glu Ile Val Arg Val Asp Ile Met Arg
Ser Phe Ser Ser Ser Ala Asn Leu Gln Gly Arg Leu Gly Met Lys
Asp Gly Asp Leu Gly Ser Pro ou
Cys Asp Leu Pro Gln Asn His Ile Leu Val Ser Arg Lys Asn Phe
Val Leu Leu Gly Gln Met Ser Arg Ile Ser Ser Ala Ile Cys Leu
Lys Asp Arg Lys Asp Phe Arg Phe Pro Gln Asp Met Ala Asp Gly
Arg Gln Phe Pro Glu Ala Gln Ala Ala Ser Val Leu His Glu Met
Leu Gln Gln Ile Phe Ser Leu Phe His Thr Glu Arg Ser Ser Ala
Ala Trp Asn Thr Thr Leu Leu Asp Glu Leu Cys Thr Gly Leu Leu
Arg Gln Leu Glu Asp Leu Asp Thr Cys Leu Glu Gln Glu Met Gly
Glu Glu Glu Ser Ala Leu Gly Thr Val Arg Pro Thr Leu Ala Val
Lys Arg Tyr Phe Arg Gly Ile His Leu Tyr Leu Lys Glu Lys Lys
Tyr Ser Asp Cys Ala Trp Glu Ile Val Arg Met Glu Ile Met Arg
Ser Phe Ser Ser Ser Ala Asn Leu Gln Gly Arg Leu Arg Met Lys
Asp Gly Asp Leu Gly Ser Pro
 ou à des variantes biologiquement actives de ces séquences, avec le spectre d'activité de EqlFN alpha, bêta ou oméga.

11. Procédé de préparation de EqlFN alpha, bêta ou oméga, caractérisé en ce que
a) un organisme hôte, de préférence un organisme hôte selon l'une des revendications 5 à 7, est transformé avec des informations génétiques selon l'une des revendications 1 à 3
b) la séquence codante est contenue dans un vecteur d'expression, de préférence dans l'un des vecteurs d'expression selon la revendication 3 ou 4, et cette information est exprimée dans l'organisme hôte et
c) l'interféron EqIFN alpha, bêta ou oméga selon l'une des revendications 8 à 10, de préférence un interféron selon la revendication 10, est isolé et purifié.

12. Utilisation des interférons selon l'une des revendications 8 à 10 pour la préparation de médicaments.

13. Agent de traitement thérapeutique, caractérisé en ce qu'il contient, outre des véhicules inertes du point de vue pharmaceutique, une quantité efficace d'un interféron selon l'une des revendications 8 à 10.

14. EqIFN alpha, bêta ou oméga selon l'une des revendications 8 à 10 destiné à être utilisé comme médicament.
